# EUROPEAN PATENT SPECIFICATION

(11) **EP 3 617 198 B1**
(45) Date of publication and mention of the grant of the patent: **25.10.2023**
(21) Application number: 18790575.7
(22) Date of filing: 27.04.2018
(51) Int. Cl.: C07D 271/08, A61K 31/4245, A61P 35/00, A61P 31/00, A61P 25/28, A61P 27/12, A61P 25/24, A61P 25/22, A61P 31/04, A61P 31/10, A61P 31/14

(54) **GUANIDINE DERIVATIVE**
GUANIDINDERIVAT
DÉRIVÉ DE GUANIDINE

(30) Priority: 27.04.2017 CN 201710288663
(43) Date of publication of application: 04.03.2020
(73) Proprietor: Lunan Pharmaceutical Group Corporation, Linyi, Shandong 276006 (CN)
(72) Inventor: ZHANG, Guimin, Linyi Shandong 276006 (CN); YAO, Jingchun, Linyi Shandong 276006 (CN); REN, Yushan, Linyi Shandong 276006 (CN)
(74) Representative: Lavoix
(86) International application number: PCT/CN2018/084923
(87) International publication number: WO 2018/196861

(56) References cited:
- WO-A1-2015/119944
- WO-A1-2016/155545
- WO-A1-2018/072697
- WO-A1-2018/072697
- WO-A2-2010/005958

## Description

### Technical Field

The invention relates to the field of medicines, in particular to an imino-urea derivative and a preparation method and the compounds for use in medicine.

### Background Art

Indoleamine 2,3-dioxygenase, a monomeric enzyme containing heme, is capable of catalyzing the oxicracking of indole ring of L-tryptophan to form kynurenine. The high expression of indoleamine 2,3-dioxygenase results in depletion of local tryptophan in cells, which induces T cell arrest in G1 phase, thereby inhibiting T cell proliferation. On the other hand, the degradation of indoleamine 2,3-dioxygenase-dependent tryptophan leads to an increase in kynurenine level and also induces oxygen free radical-mediated T cell apoptosis. Thirdly, the up-regulation of dendritic cell indoleamine 2,3-dioxygenase expression enhances local regulatory T cell (Treg)-mediated immunosuppression by degrading local tryptophan, thereby promoting peripheral immune tolerance of body to tumor specific antigen. Indoleamine 2,3-dioxygenase has become the most important small molecule regulatory target for anti-tumor immunotherapy.

It has been found in studies that indoleamine 2,3-dioxygenase is associated with many physiological processes in human body. In 1998, Munn et al. revealed that the fetus was able to survive in the mother body with different genotype during pregnancy without being rejected because the placental plasmoditrophoblast cells synthesized indoleamine 2,3-dioxygenase, which inhibited the rejection reaction of maternal T cells against the fetus through blood flow. After further subcutaneous implantation of the sustained-release capsule containing indoleamine 2,3-dioxygenase inhibitor 1-methyltryptophan in pregnant mice, they found that the embryo was rejected and aborted (Munn DH, Zhou M, Attwood JT, et al. Prevention of allogeneic fetal rejection by tryptophan catabolism. Scienice, 1998, 281 (5380): 1191-3). In addition, some diseases caused by abnormal immune responses, such as transplant rejection and autoimmune diseases, are also closely related to indoleamine 2,3-dioxygenase.

Although great progress has been made in treatment of tumors in recent years, the clinical efficacy is still unsatisfactory. Immune escape is one of the main biological mechanisms of tumorigenesis and tumor metastasis, and has become an important factor affecting the therapeutic effect of tumors. Indoleamine 2,3-dioxygenase, as an immune regulatory enzyme, can effectively inhibit T cell function, enhance Treg cell function, and induce NK cell dysfunction, while tumor cells can use these inherent immune regulation mechanisms in body to escape from the identification and killing of immune system (Jia Yunlong, Wang Yu, Chinese Journal of Cancer Biotherapy, 2004, 21 (6): 693-7). In order to enable tumor patients to get optimum benefit from treatment, it is imperative to rationally adjust the treatment strategy for tumor immune escape. The indoleamine 2,3-dioxygenase inhibitor of the present invention can effectively regulate the immune system of patient, block the immune escape of tumor cells, and has a good therapeutic effect on most spontaneous tumors. Based on the regulation of immune system, in addition to treating tumors, the indoleamine 2,3-dioxygenase inhibitor of the present invention can also treat other diseases related to immunity, such as chronic infection and AIDS.

Indoleamine 2,3-dioxygenase is also closely related to neurological diseases. It can lower the level of 5-hydroxytryptamine and cause mental illnesses such as depression and anxiety. It can also cause accumulation of neurotoxic metabolites such as quinolinic acid in brain, which is closely related to the occurrence of neurodegenerative diseases such as Alzheimer's disease. Indoleamine 2,3-dioxygenase can influence brain function by at least two mechanisms: 1) during an inflammatory response, the catabolism of tryptophan can result in a decrease in circulating tryptophan concentrations, thereby resulting in a decrease in 5-hydroxytryptamine level, which leads to depression; 2) indoleamine 2,3-dioxygenase catabolizes tryptophan into products that enter the kynurenine pathway, resulting in the accumulation of kynurenine and neurotoxic quinolinic acid (Kong Linglei, Kuang Chunxiang, Yang Qing, Chinese Journal of Medicinal Chemistry, 2009, 19(2): 147-154).

WO 2010/005958 relates to 1,2,5-oxadiazole derivatives comprising a sulfamide moiety, which are inhibitors of indoleamine 1,3-dioxygenase and are useful in the treatment of cancer.

### Contents of the Invention:

The present invention provides a compound, or a pharmaceutically acceptable salt thereof, a composition containing the compound or a pharmaceutically acceptable salt thereof, and its use in a method for treating of a cancer, an infectious disease, a neurodegenerative disease, a depression, an anxiety or an age-related cataract.

The compound or a pharmaceutically acceptable salt thereof has excellent activity for inhibiting indoleamine 2,3-dioxygenase, and the activity is significantly superior to other IDO inhibitors. In addition, by measuring the body weight of mice before and after the administration of the compound or a pharmaceutically acceptable salt thereof, it is found that as compared to other IDO inhibitors, the compound of the present invention or a pharmaceutically acceptable salt thereof can significantly improve the life quality of mice during tumor treatment and significantly reduce side effects. In clinical practice, the compound of the present or a pharmaceutically acceptable salt thereof will improve not only patient's life quality, but also significantly improve patient compliance with medications and medicament effectiveness. The compound of the present invention or a pharmaceutically acceptable salt thereof can significantly improve the learning and memory impairment in animals and enhance learning acquisition ability and spatial memory ability, has positive therapeutic significance for neurodegenerative diseases such as Alzheimer's syndrome, and is superior to other IDO inhibitors. The compound of the present invention or a pharmaceutically acceptable salt thereof can promote the function of DC in stimulation of T cell proliferation, so that it can be used for treating tumor diseases, autoimmune diseases, transplant rejection, and infectious diseases, and it is superior to other IDO inhibitors.

### Detailed Description of the Invention:

All subject matter not present in the claims, be it explicitly or implicitly disclaimed, does not form part of the invention and is solely present for reference purposes.

The present invention provides a compound represented by Formula I: 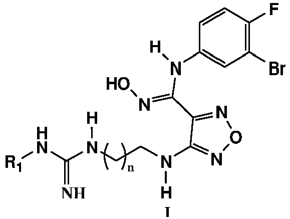 wherein, R₁ is selected from the group consisting of:H, NH₂, CN, methyl, ethyl, propyl, isopropyl, butyl, cyclopropyl, cyclobutyl, cyclopentyl, cyclohexyl, methoxycarbonyl, tert-butoxycarbonyl, fluorenylmethoxycarbonyl, allyloxycarbonyl, benzyl, and 5-azaindene methylcarbonyl; n is an integer selected from 0 to 6, such as 0, 1, 2, 3, 4, 5 or 6; preferably, n is 1 or 2.

In some embodiments, the present invention provides a Compound represented by Formula I or a pharmaceutically acceptable salt thereof, wherein when R₁ in Formula I is H, the compound is represented by Formula II, 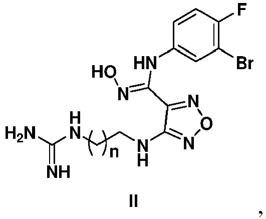 preferably wherein n is an integer selected from 0, 1, 2, or 3.

In some embodiments, the invention provides a compound as follows, or a pharmaceutically acceptable salt thereof:

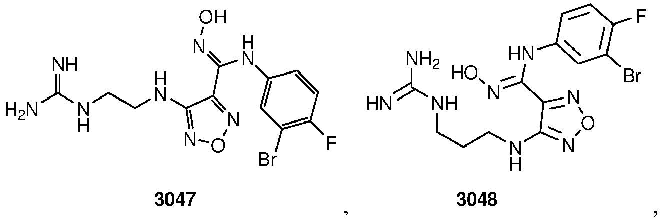

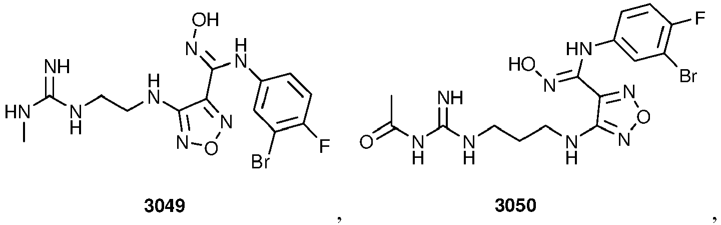

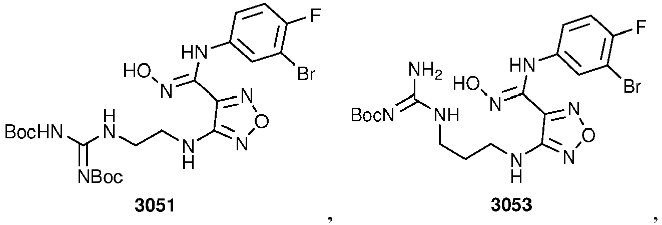

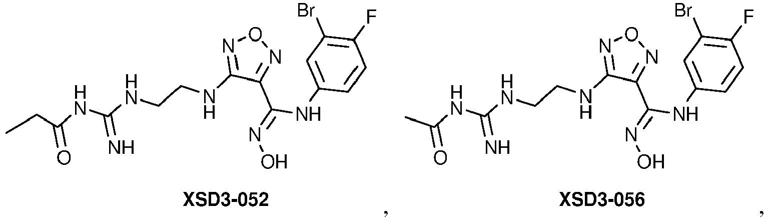

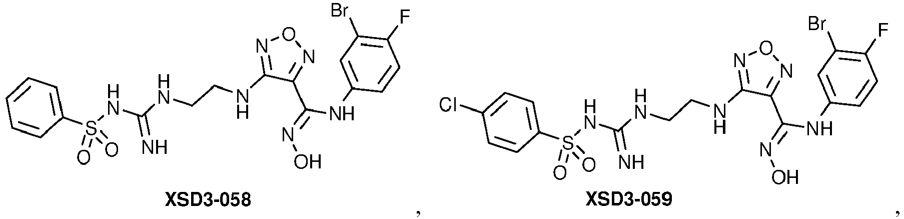

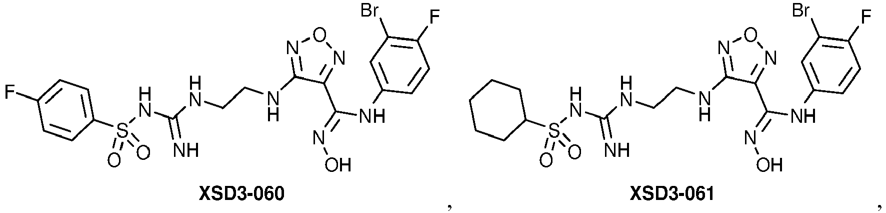

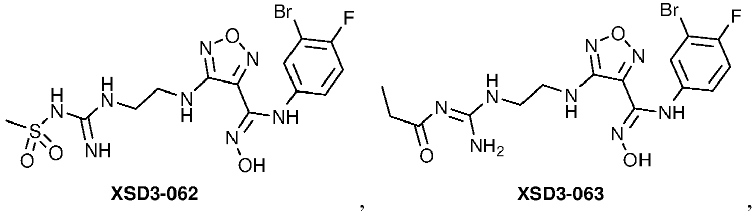

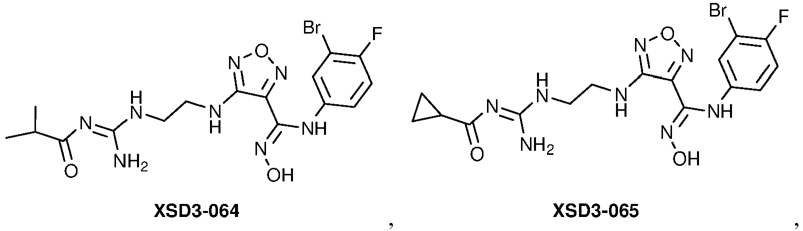

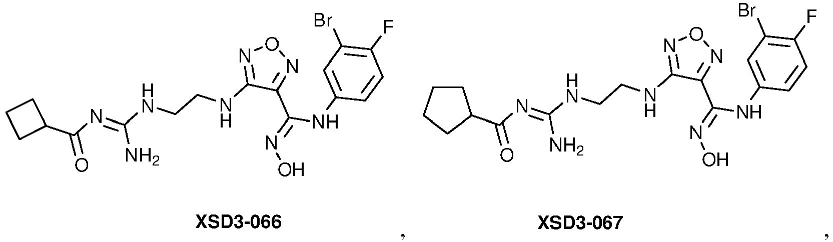

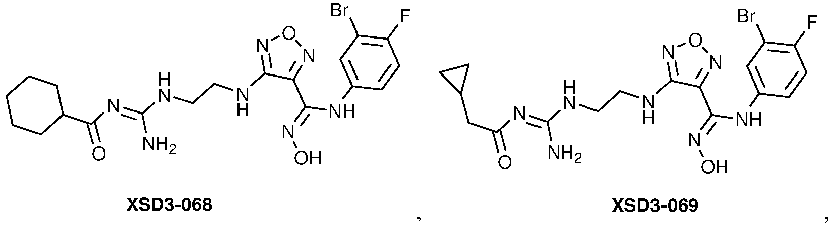

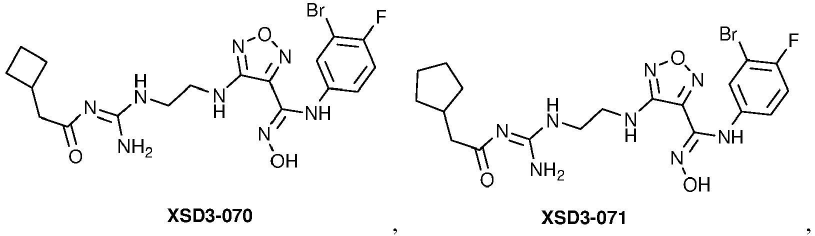

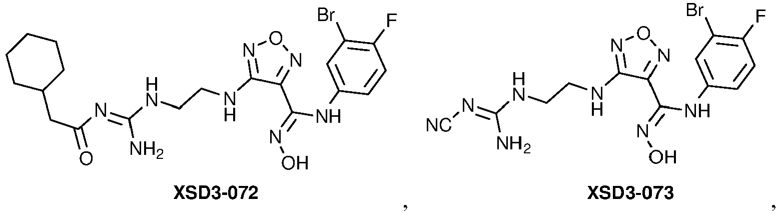

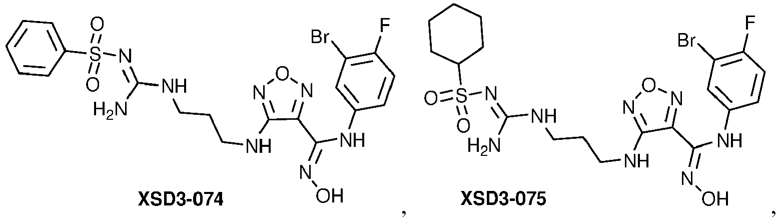

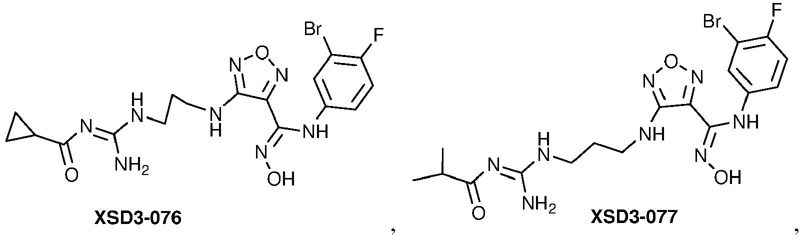

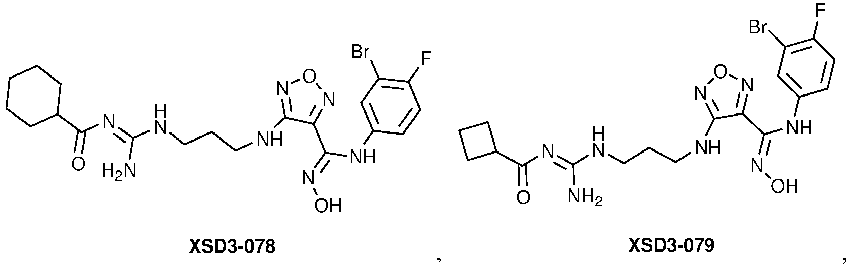

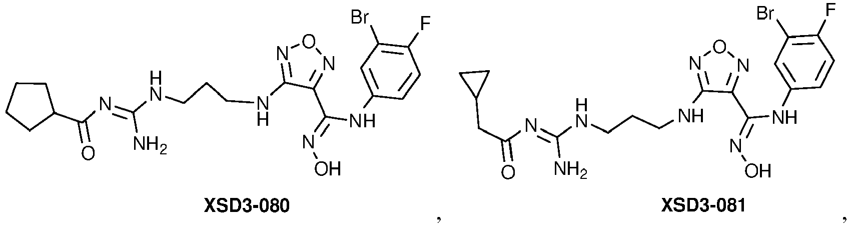

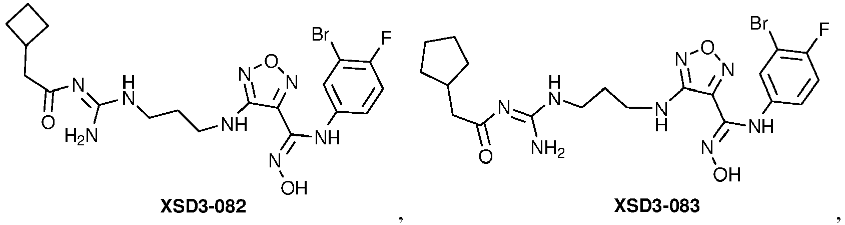

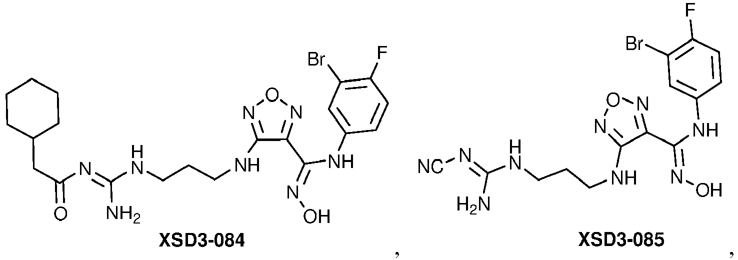

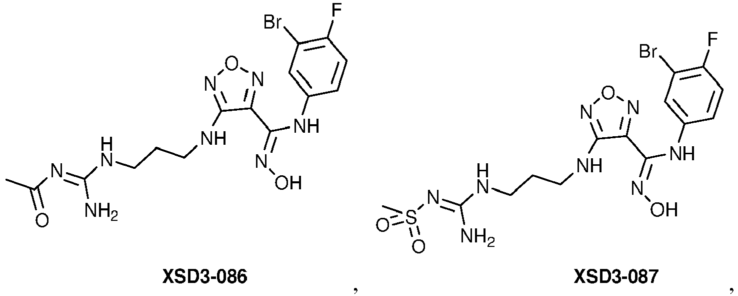

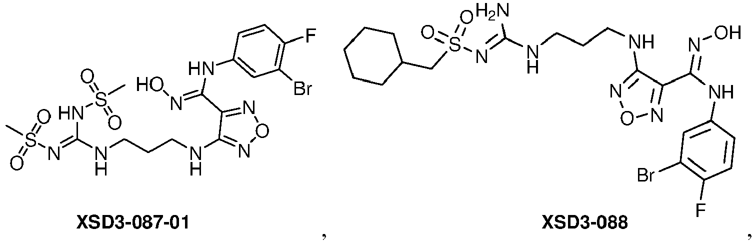

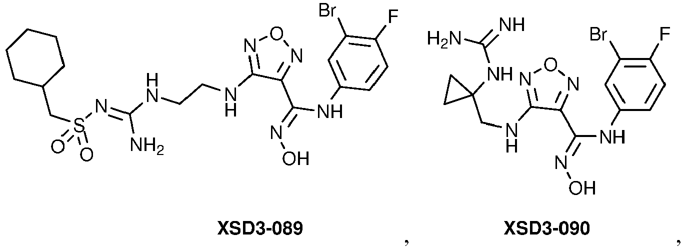

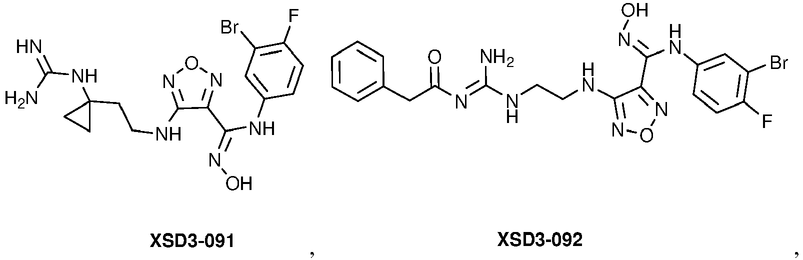

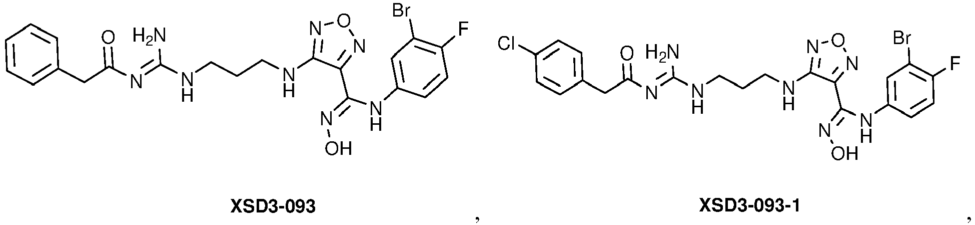

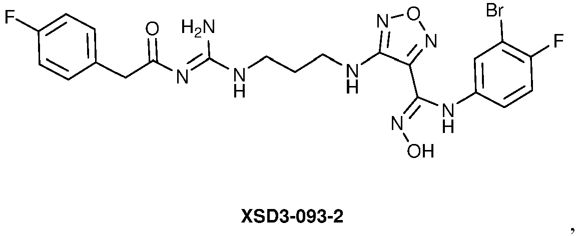

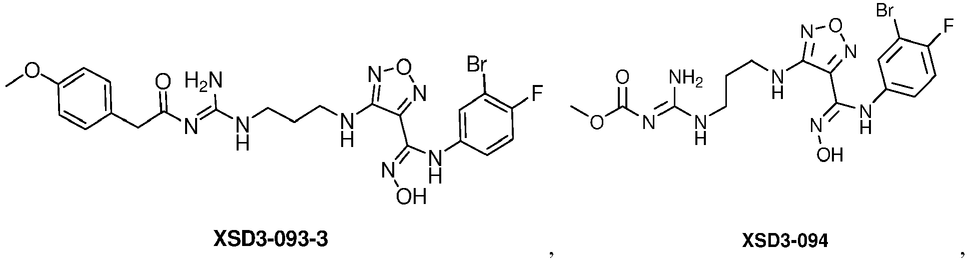

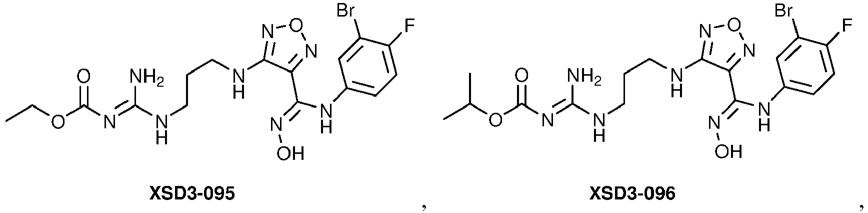

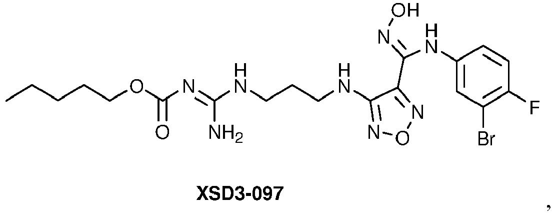

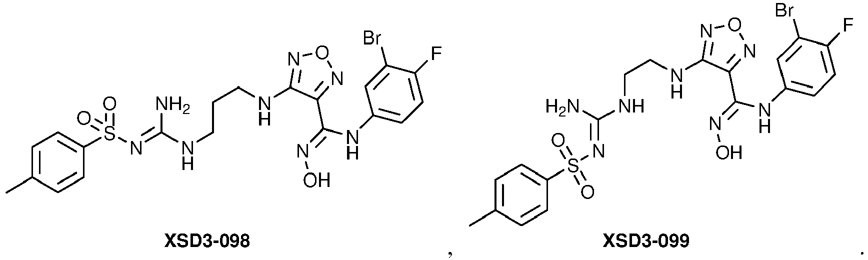

From the general formula or the synthesis method of general formula of the present invention, compounds not limited to these specific compounds can be derived, and under the guidance of the general formula or the synthesis method of general formula of the present invention, specific compounds that can be obtained by those skilled in the art without the need of creative labor are all within the scope of the invention.

The present invention describes a method for synthesizing the above-described compound represented by Formula I₀ (which is not part of the present invention), 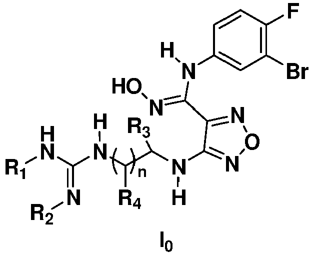 i.e., General Synthesis Method I, steps of which are as follows:
1) Oxidation of compound 1a to obtain compound 2a: 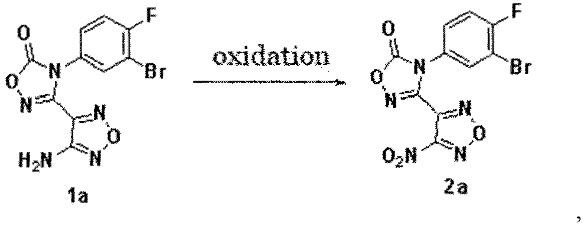 an oxidizing agent used above includes, but is not limited to, at least one of hydrogen peroxide, ozone or peracetic acid;
2) Reaction of a compound represented by formula 1with a compound represented by formula 2a under alkaline condition to obtain a compound represented by formula 2: 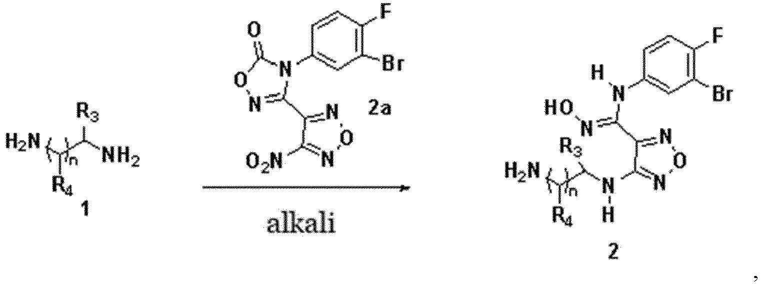 an alkali used above includes, but is not limited to, alkali metal hydroxides, preferably sodium hydroxide, potassium hydroxide, barium hydroxide;
3) Reaction of the compound represented by formula 2 with a compound represented by formula 3a to obtain the compound represented by formula I₀: 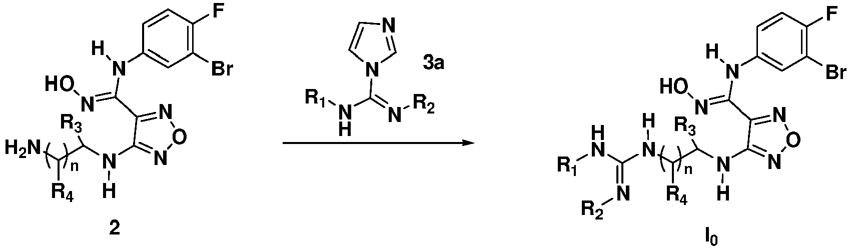
   wherein, R₁, R₂ are each independently selected from the group consisting of: H, substituted or unsubstituted C₁₋₁₀ alkyl, aldehyde group, substituted or unsubstituted carbonyl, cyano, CF₃, substituted or unsubstituted C₁₋₁₀ alkoxy, substituted or unsubstituted sulfonyl, substituted or unsubstituted C₃₋₁₀ cycloalkyl, substituted or unsubstituted C₂₋₁₀ alkenyl, substituted or unsubstituted C₆₋₂₀ aryl, substituted and unsubstituted C₃₋₁₄ heteroaryl;
   R₃, R₄ are each independently a mono-substituent selected from the group consisting of: H, substituted or unsubstituted C₁₋₁₀ alkyl, substituted or unsubstituted C₃₋₁₀ cycloalkyl, cyano, substituted or unsubstituted C₁₋₁₀ alkoxy, substituted or unsubstituted sulfonyl, substituted or unsubstituted C₆₋₂₀ aryl, and substituted or unsubstituted C₃₋₁₄ heteroaryl;
   or R₃, R₄ are each independently selected from di-substituents, thereby forming the following groups together with the C atom at a- or b-position: 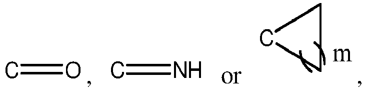 wherein C represents the C atom at a- or b-position, m is an integer selected from 0 to 6;
      n is an integer selected from 0 to 6.

The present invention describes a method for synthesizing the above-described compound represented by Formula I (which is not part of the present invention),

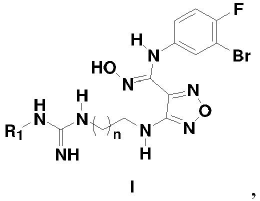

i.e., General Synthesis Method IA, steps of which are as follows:
1) Oxidation of compound **1a** to obtain compound **2a;** 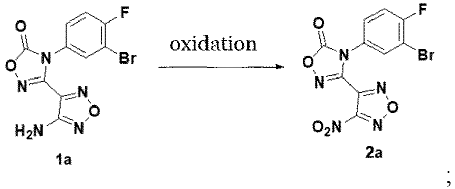
2) Reaction of a compound represented by formula **1** with compound **2a** under alkaline condition to obtain a compound represented by formula **2;** 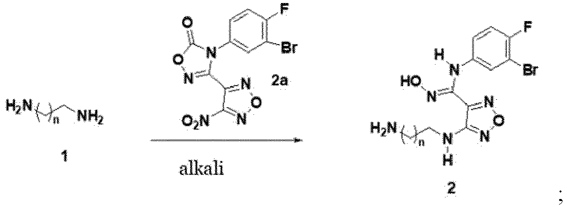
3) Reaction of the compound represented by formula **2** with a compound represented by formula **3a** to obtain the compound represented by formula **I,** 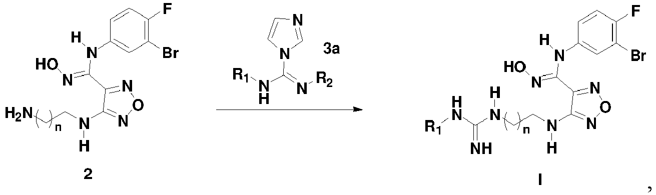
   wherein R₁ is selected from the group consisting of: H, NH₂, CN, methyl, ethyl, propyl, isopropyl, butyl, cyclopropyl, cyclobutyl, cyclopentyl, cyclohexyl, methoxycarbonyl, tert-butoxycarbonyl, fluorenylmethoxycarbonyl, allyloxycarbonyl, benzyl, and 5-azaindene methylcarbonyl; n is an integer selected from 0 to 6;
   an oxidizing agent used above is preferably, but not limited to, at least one of hydrogen peroxide, ozone or peroxyacetic acid;
   the alkali is preferably selected from, but not limited to, alkali metal hydroxides, preferably sodium hydroxide, potassium hydroxide or barium hydroxide.

In some embodiments, the present invention provides a method for synthesizing the above compound represented by Formula II,

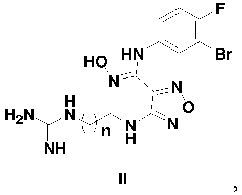

i.e., General Synthesis Method II, steps of which are as follows:
1) Reaction of a compound represented by formula **2** with compound **3a'** to obtain a compound represented by formula **IIa:** 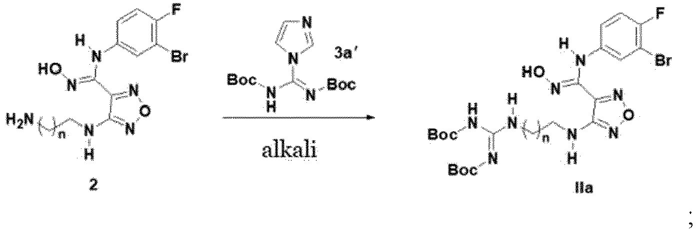
2) Deprotection of the compound represented by formula **IIa** under acidic condition, followed by reaction under alkaline condition to obtain the compound represented by formula **II,** 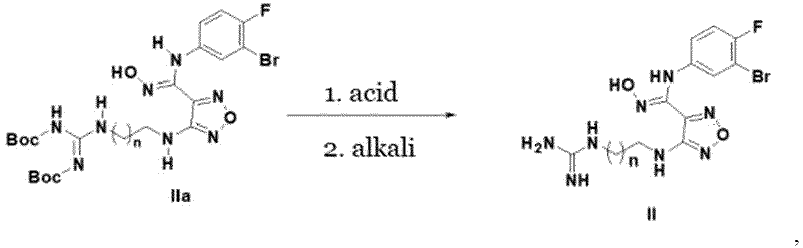
   wherein n is 0, 1, 2, or 3,
   the alkali is selected from, but not limited to, hydroxides of alkali metal or alkaline earth metal, preferably sodium hydroxide, potassium hydroxide or barium hydroxide.

The synthesis method provided by the present invention is only one way to realize each synthesized target compound and its intermediate, wherein each step and number, such as **1a, 2a, 3a, 4a, 1, 2, 3,** etc., are independent, and the preparation thereof is not limited to the method of the present invention.

Unless otherwise specified, the solvent used in each step of the above or below reactions of the present invention is a conventional solvent in the art, and the selection principle is that the solvent is able to dissolve the reactants but not participate in the reaction, extract the product or allow the corresponding product to crystallize therein so as to be separated from the impurity. Examples of the solvent include water, halogenated alkanes, alkylamines, aliphatic hydrocarbons, esters, alcohols, aromatic hydrocarbons, ethers, heterocyclic solvents. Specifically, the solvent is selected from, but not limited to, the following solvents: methanol, ethanol, propanol, isopropyl, diethyl ether, ethyl acetate, acetic acid, cyclohexane, dichloromethane, chloroform, tetrahydrofuran, pyridine, diethyl amine, triethylamine, dimethylformamide, toluene, and mixtures of at least two of them.

Unless otherwise specified, in each of the above or below reactions of the present invention, when a reactant is in an excessive amount, the termination of the reaction may be carried out by adding a substance which can react with the excessive reactant.

Unless otherwise specified, in each of the above or below reactions of the present invention, the purification method of the product in each step of the reactions is selected from the group consisting of extraction, crystallization, solvent removal, column chromatography, the operations thereof are all conventional techniques in the art, and a skilled in the art can handle them according to specific conditions.

The numbers used in the general formula of the present invention are used for conveniently describing the general formula, and they may be modified into other numbers in specific embodiments, such as 1, 2, 3, etc., for convenience of description, and are the expressions of general formula and general reaction equations that do not affect the essence of the structural formula and its reaction equations.

For the compounds encompassed by the Formulas I to II and specific representative substances thereof, their chiral or cis-trans isomers and mixtures of these isomers in any ratio also fall within the scope of the compounds encompassed by the Formulas I to II and specific representative substances thereof.

In some embodiments, the present invention provides a pharmaceutical composition comprising the above compounds, i.e., the compounds encompassed by the Formulas I to II and the above specific compounds, or a pharmaceutically acceptable salt thereof, and one or more pharmaceutically acceptable pharmaceutic adjuvants.

The term "pharmaceutically acceptable salt" as used in the present invention refers to an addition salt formed with a pharmaceutically acceptable acid or alkali, or a solvate thereof. Such pharmaceutically acceptable salts include salts of acids, wherein the acids include hydrochloric acid, sulfuric acid, nitric acid, phosphoric acid, hydrobromic acid, hydroiodic acid, formic acid, acetic acid, p-toluenesulfonic acid, sulfinic acid, methanesulfonic acid, benzoic acid, fumaric acid, citric acid, tartaric acid, maleic acid, fatty acid. Addition salts of non-toxic pharmaceutically acceptable alkali include salts of alkali, and these alkali include sodium, potassium, calcium, magnesium, aluminum, ammonium.

In some embodiments, the present invention provides the above-described compound or a pharmaceutically acceptable salt thereof, which is capable of inhibiting the activity of indoleamine 2,3-dioxygenase, and can be used for treating a disease pathologically characterized by indoleamine 2,3-dioxygenase-mediated tryptophan metabolic pathway; the medicament is used for treating a cancer, an infectious disease, a neurodegenerative disease, a depression, an anxiety or an age-related cataract;
wherein the cancer is selected from the group consisting of lung cancer, liver cancer, colon cancer, pancreatic cancer, breast cancer, prostate cancer, brain cancer, ovarian cancer, cervical cancer, testicular cancer, renal cancer, head and neck cancer, lymphoma, melanoma or leukemia;
the neurodegenerative disease refers to Alzheimer's disease;
the infectious disease refers to an infection caused by a bacterium, a fungus, a virus or a parasite.

In some embodiments, the present invention provides the present compounds for use in a method for inhibiting the activity of indoleamine 2,3-dioxygenase by using the above-described compound or a pharmaceutically acceptable salt thereof, or for treating a disease pathologically characterized by indoleamine 2,3-dioxygenase-mediated tryptophan metabolic pathway, the method comprising administering a therapeutically effective amount of the compound or a pharmaceutically acceptable salt thereof. The disease is selected from the group consisting of cancer, infectious disease, neurodegenerative disease, depression, anxiety, or age-related cataract; wherein the cancer is selected from the group consisting of lung cancer, liver cancer, colon cancer, pancreatic cancer, breast cancer, prostate cancer, brain cancer, ovarian cancer, cervical cancer, testicular cancer, kidney cancer, head and neck cancer, lymphoma, melanoma or leukemia; the neurodegenerative disease refers to Alzheimer's disease; the infectious disease refers to an infection caused by a bacterium, a fungus, a virus or a parasite.

The activity test results according to the examples of the present invention show that the compounds obtained by the present invention have excellent activity for inhibiting indoleamine 2,3-dioxygenase, and the activity is significantly superior to the compound INCB024360. *In vivo* test results show that the compounds of the present invention have a high inhibition rate on tumors, and the therapeutic effect on tumors is significantly better than that of the compound INCB024360 and other IDO inhibitors. Moreover, by measuring the body weights of the mice before and after administration, it is found that as compared to other IDO inhibitors, the indoleamine 2,3-dioxygenase inhibitors of the present invention can significantly reduce the side effects during the period of tumor treatment, significantly improve the life quality of the mice, and in clinical practice, improve not only patient's life quality, but also significantly improve patient compliance with medications and medicament effectiveness.

The compound of the invention can significantly improve the learning and memory impairment, enhance the learning acquisition ability and the spatial memory ability in animals, and has positive therapeutic significance for neurodegenerative diseases such as Alzheimer's syndrome, and the effect thereof is superior to other IDO inhibitors.

Through the T cell proliferation reaction experiment, it is found that the compound of the present invention can promote the function of DC in stimulation of T cell proliferation, so that it can be used for treating tumor diseases, autoimmune diseases, transplant rejection, and infectious diseases, and it is obviously superior to other IDO inhibitors.

When the indoleamine 2,3-dioxygenase inhibitor of the present invention is used for treating a disease pathologically characterized by indoleamine 2,3-dioxygenase-mediated tryptophan metabolic pathway, it shows the following technical advantages:
(1) The antitumor effect is remarkable. The compound of the present invention has activity for significantly inhibiting indoleamine 2,3-dioxygenase, and the *in vivo* test shows that the tumor inhibition rate of the compound of the present invention is significantly higher than those of the positive control drug cyclophosphamide and the compound INCB024360.
(2) The side effect is lowered. The compound of the present invention is an indoleamine 2,3-dioxygenase inhibitor, which reverses the inhibiting of T cells proliferation and regulates the immune function of body by inhibiting the activity of indoleamine 2,3-dioxygenase, thereby completing the monitoring and killing effects of the human immune system on tumor cells. Based on this special mechanism of action, this compound does not adversely affect the growth of normal cells of the human body while inhibiting the growth of tumor cells, thus significantly reducing the side effects. Moreover, it has a significant therapeutic effect on autoimmune diseases, transplant rejection, and infectious diseases associated with T cell proliferation.
(3) The treatment of Alzheimer's and other neurodegenerative diseases is significantly effective, the learning and memory impairment of animals can significantly be improved, and the learning acquisition ability and the spatial memory ability can significantly be enhanced.

### Specific Models for Carrying Out the Invention:

The invention is further described below in conjunction with specific embodiments, but the invention is not limited thereto. In addition, under the guidance of the general formula or the synthesis method of general formula (General Synthesis Method I, General Synthesis Method IA, General Synthesis Method II) and specific embodiments of the present invention, the specific compounds obtained by those skilled in the art without the need of creative labor are all within the scope of the invention.

### Example 1

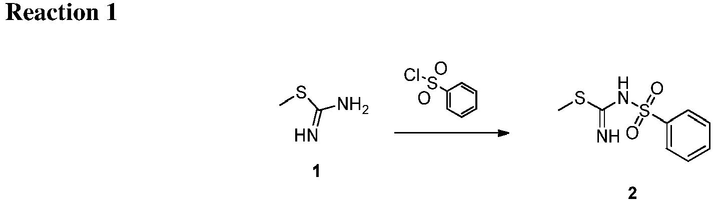

Compound 1 (903 mg, 10 mmol) was dissolved in acetone (10 mL), then potassium carbonate (2.76 g, 20 mmol) was added at room temperature, stirred at room temperature for 0.5 h, added dropwise with benzenesulfonyl chloride, stirred at room temperature overnight, quenched with water, and the resultant was extracted with ethyl acetate, dried over anhydrous sodium sulfate, and concentrated to obtain 900 mg of a white powder.

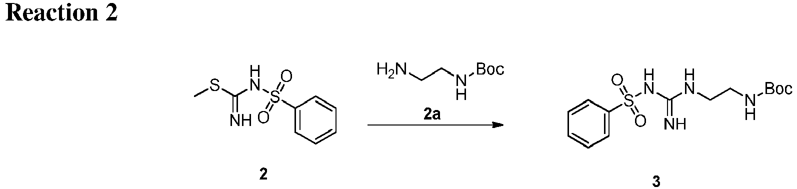

Compound **2** (230 mg, 1 mmol) was dissolved in acetonitrile (5 mL), stirred at room temperature for 0.5 h, then added with compound **2a** (320 mg, 2 mmol), heated at 60°C for 24 h, and the resultant was directly evaporated to dryness under reduced pressure, and subjected to purification by pre-HPLC to obtain 210 mg of the desired product.

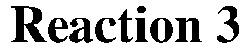

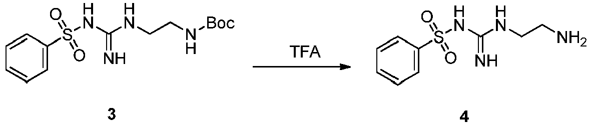

Compound **3** (171 mg, 0.5 mmol) was dissolved in dichloromethane (10 mL), stirred at room temperature for 5 min, added with trifluoroacetic acid (5 mL), reacted at room temperature for 2 h, and then the resultant was directly evaporated to dryness under reduced pressure to give the desired product **4** which was directly used as a crude product in the next step.

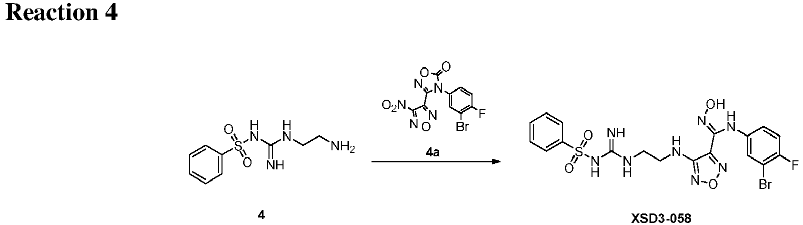

Compound **4** (120 mg, 0.5 mmol) was dissolved in tetrahydrofuran, the compound **4a** (120 mg, 0.5 mmol) was added, the mixture was stirred at room temperature for 10 min, 1N sodium hydroxide (1 mL) was added dropwise, and the mixture was stirred at room temperature for 10 min. The reaction solution was concentrated and subjected to purification by pre-HPLC to give 5 mg of the desired product.

¹H-NMR (400MHz, DMSO-d6): δ(ppm) : 11.45 (s, 1H), 8.89 (s, 1H), 7.76-7.77(m, 2H), 7.48-7.52 (m, 3H), 7.10-7.20(m, 3H), 6.75-6.77 (m, 2H), 6.27 (s, 1H), 3.73-3.77 (m, 2H), 2.52-2.69(m, 2H).

HPLC purity: @214nm 99.5 %, @254nm 100%.

LC-MS: *m*/*z* 541 [M+1].

Referring to the General Synthesis Method I and Example 1, the following compounds were synthesized.

| Example No. | Substituent | | | Substance No. | MS |
|---|---|---|---|---|---|
| Example 2 | R₁ is | 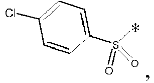 | n is 1, R₂, R₃, R₄ are H. | XSD3-059 | 574.0 |
| Example 3 | R₁ is | 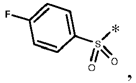 | n is 1, R₂, R₃, R₄ are H. | XSD3-060 | 558.0 |
| Example 4 | R₁ is | 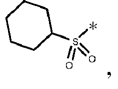 | n is 1, R₂, R₃, R₄ are H. | XSD3-061 | 546.1 |
| Example 5 | R₁ is | 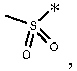 | n is 1, R₂, R₃, R₄ are H. | XSD3-062 | 478.0 |
| Example 6 | R₁ is | 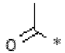 | (methylcarbonyl), n is 2, R₂, R₃, R₄ are H. | 3050 | 456.1 |
| Example 7 | R₁ is | 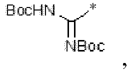 | n is 1, R₂, R₃, R₄ are H. | 3051 | 600.2 |
| Example 8 | R₁ is ethylcarbonyl, n is 1, R₂, R₃, R₄ is H. | | | XSD3-052 | 456.1 |
| Example 9 | R₁ is | 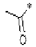 | (methylcarbonyl), n is 1, R₂, R₃, R₄ are H. | XSD3-056 | 442.1 |

### Example 10

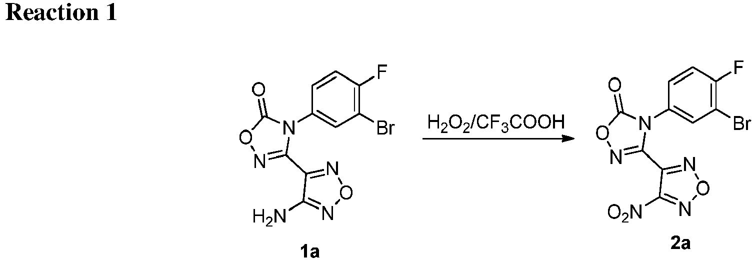

Compound **1a** (682 mg, 2 mmol) was dissolved in trifluoroacetic acid (13 mL), then 30% H₂O₂ was added dropwise at room temperature, the mixture was reacted overnight at 50°C, and the reaction solution changed from turbid to clear yellow. After the reaction was completed, the reaction was quenched with a saturated sodium sulfite solution. After KI starch test paper showed clolorless, the resultant was extracted with ethyl acetate (50 mL * 2), and the organic phase was dried over anhydrous sodium sulfate, concentrated, and then subjected to purification by column chromatography (petroleum ether : ethyl acetate=1:1) to obtain a pale yellow solid **2a** (500 mg, yield 67%).

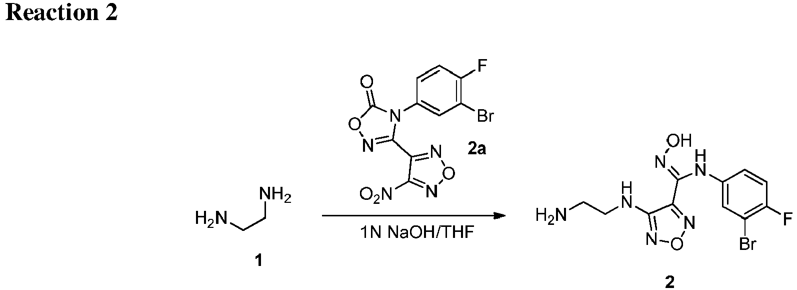

Ethylenediamine **1** (30 mg, 0.5 mmol) was added to a solution of Compound **2a** (170 mg, 0.5 mmol) in tetrahydrofuran (10 mL), then 1N NaOH (0.4 mL) was added, the reaction solution was stirred at room temperature for 0.5 h, and the reaction solution was directly used to prepare and obtain Compound **2** (120 mg).

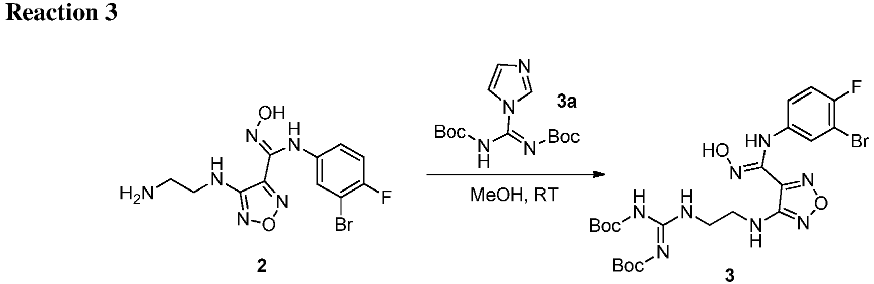

A mixture solution of Compound **2** (120 mg, 0.33 mmol) and Compound **3a** (100 mg, 0.33 mmol) in methanol (10 mL) was stirred at room temperature overnight, and the reaction solution was concentrated and subjected to purification by column chromatography (petroleum ether: ethyl acetate=1:1) to obtain Compound **3** (42 mg, 23%).

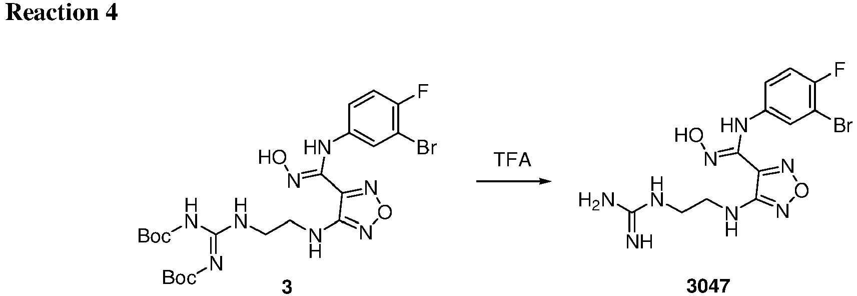

Trifluoroacetic acid (0.6 mL) was added to a solution of Compound 3 (31 mg, 0.05 mmol) in dichloromethane (3 mL), and stirred at room temperature overnight. The reaction solution was concentrated and subjected to purification by pre-HPLC to obtain compound XSD3-047 (9 mg, yield 68%). For the desired compound: ¹H-NMR (400MHz, DMSO-d6): δ(ppm) : 11.4 (s, 1H), 8.88 (s, 1H), 7.22-7.11 (m, 2H), 6.71-6.5 (m, 3H), 6.38-6.21 (m, 2H), 3.37 (m, 3H), 3.01 (m, 2H). MS: *m*/*z* 401.2 [M+1].

### Example 11

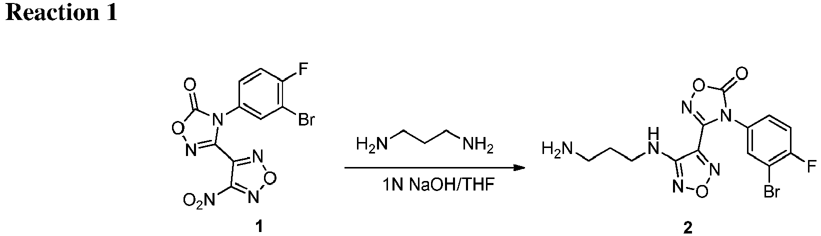

1,3-Diaminopropane (35 mg, 0.5 mmol) was added to a solution of Compound **1** (170 mg, 0.5 mmol, the synthesis method of which was referred to the synthesis of Compound **2a** in XSD3-047 Final report) in tetrahydrofuran (10 mL), the reaction solution was stirred at room temperature for 0.5 h, and the reaction solution was directly used to prepare and obtain the desired product of 130 mg.

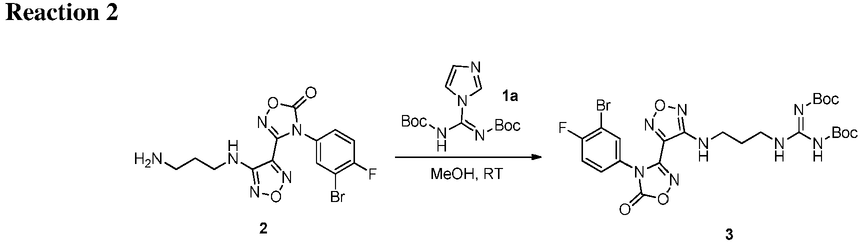

A mixture solution of Compound **2** (130 mg, 0.33 mmol) and Compound **1a** (100 mg, 0.33 mmol) in methanol (10 mL) was stirred at room temperature overnight, the reaction solution was concentrated and subjected to purification by column chromatography (petroleum ether : ethyl acetate=1:1) to obtain Compound **3** (78 mg, 45%).

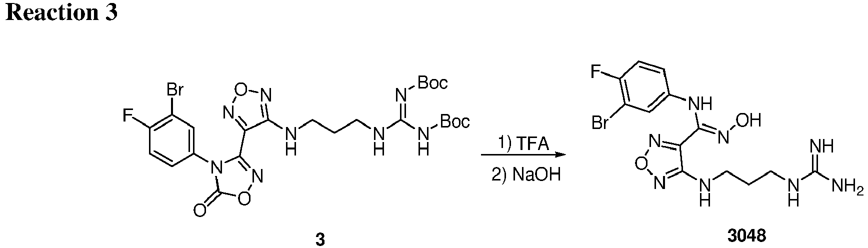

Trifluoroacetic acid (0.6 mL) was added to a solution of Compound **3** (31 mg, 0.05 mmol) in dichloromethane (3 mL), stirred at room temperature overnight, then adjusted with 1N NaOH solution to pH = 12, stirred continuously for 20 min, and the reaction was monitored by LCMS. After the reaction was completed, the reaction solution was adjusted to neutral with 0.5 N HCl, and the reaction solution was concentrated and subjected to purification by pre-HPLC to obtain the desired product (9 mg, yield 68%). For the desired product: ¹H-NMR (400MHz, DMSO-d6): δ(ppm) : 11.4 (s, 0.6 H), 8.88 (s, 1H), 7.6 (s, 1H), 7.4-6.7 (m, 6H), 6.7 (s, 1H), 6.24(s, 1H), 3.19-3.14 (m, 4H), 1.75 (m, 2H). MS: *m*/*z* 415.2 [M+1].

### Example 12

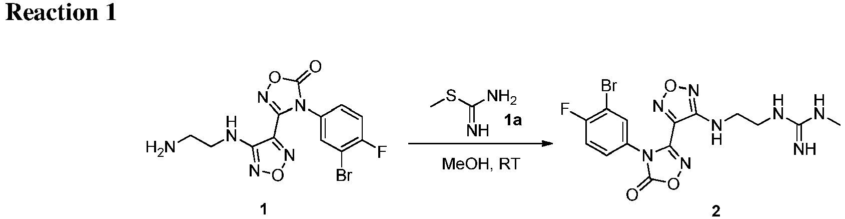

A mixture solution of Compound **1** (384 mg, 1 mmol, the preparation method of which was referred to that in the Example of 3047) and Compound **1a** (100 mg, 1.1 mmol) in tetrahydrofuran (10 mL) were stirred at room temperature for 48 h, and the reaction solution was directly used to prepare and obtain the desired product **2** (89 mg, yield 20%).

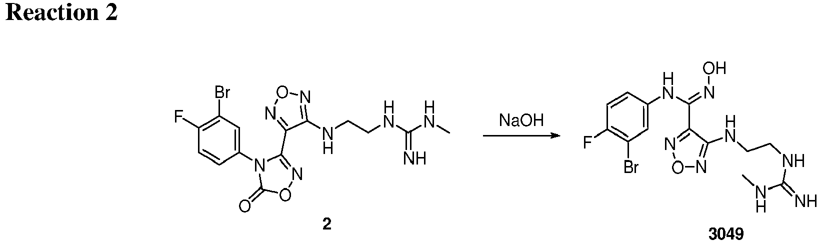

Compound 2 (89 mg, 0.2mmol) was dissolved in THF (10 mL), then adjusted with 1N NaOH solution to pH=12, and stirred continuously for 20min. The reaction was monitored by LCMS. After the reaction was completed, 0.5N HCl was used to adjust the reaction solution to neutral, and the reaction solution was concentrated and subjected to purification by pre-HPLC to obtain the desired product (13 mg, yield 15%). For the desired compound: ¹H-NMR (400MHz, DMSO-d6): δ(ppm) : 11.47(s, 0.6 H), 8.92 (s, 1H), 7.50-7.09(m, 5H), 6.70 (s, 1H), 6.28 (s, 1H), 2.72-2.50 (m, 4H), 2.50 (m, 3H). MS: *m*/*z* 416.2 [M+1].

### Example 13

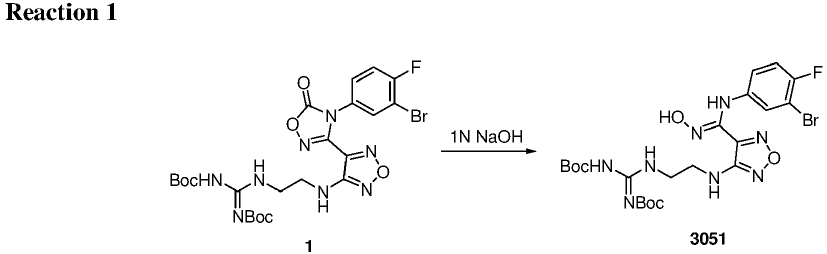

Compound **1** (102 mg, 0.016 mmol, the synthesis method of which was referred to XSD3-047 Final Report) was dissolved in THF (10 mL), then 1N NaOH solution (0.1 mL) was added at room temperature, stirred at room temperature for 0.2 h, and the reaction was monitored by LCMS. After the reaction was completed, the pH was adjusted to neutral with 0.5 N HCl solution, to obtain 52 mg of desired compound. For the desired compound: ¹H-NMR (400MHz, DMSO-d6): δ(ppm) : 11.45 (s, 1H), 8.89 (s, 1H), 8.24 (m, 1H), 7.76-7.77(m, 2H), 7.14-7.20(m, 2H), 7.18(s, 1H), 6.31 (s, 1H), 3.33-3.67 (m, 4H), 1.44-1.48(m, 18H). MS: *m*/*z* 601.2 [M+1].

### Example 14

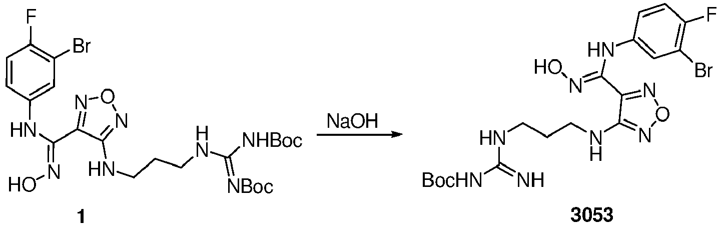

Compound **1** (52 mg, 0.1 mmol, the synthesis method of which was referred to XSD3-048 Final Report) was dissolved in THF (10 mL), then TFA (0.5 mL) was added at room temperature, stirred at room temperature for 12 h, and the reaction was monitored by LCMS. After purification by pre-HPLC, the desired compound of 15 mg was obtained. For the desired compound: ¹H-NMR (400MHz, DMSO-d6): δ(ppm) : 11.44 (s, 1H), 10.85 (s, 1H), 8.91 (s, 1H), 8.49 (s, 1H), 7.18-7.09(m, 3H), 6.78-6.75 (m, 1H), 6.28-6.25(m, 1H), 3.33-3.19 (m, 4H), 1.85-1.78(m, 2H), 1.5 (s, 3H). LC-MS characterization result: *m*/*z* 515 [M+1].

### Example 15

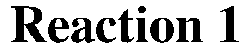

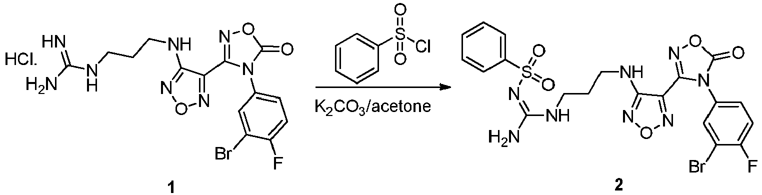

Compound **1** (100 mg, 0.23 mmol) was dissolved in acetone (10 mL), then added with potassium carbonate (0.276 g, 2.0 mmol), stirred at room temperature for 0.5 h, added dropwise with benzenesulfonyl chloride, stirred at room temperature overnight. The reaction was added with water for quenching, and the resultant was extracted with ethyl acetate, dried over anhydrous sodium sulfate, and concentrated to obtain a crude product which was directly used in the next step.

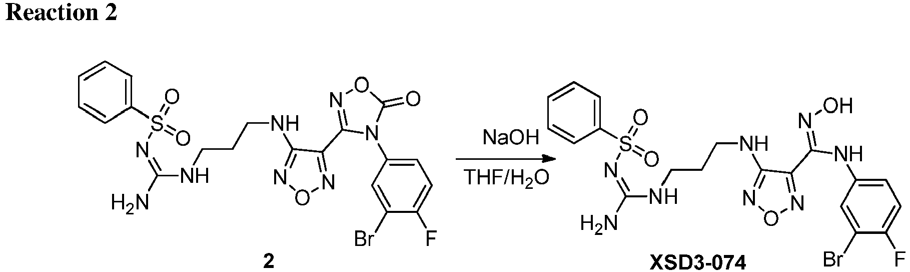

Compound **2** (120 mg, 0.5 mmol) was dissolved in tetrahydrofuran/water, added dropwise with 1N sodium hydroxide (1 mL), and stirred at room temperature for 10 min. The reaction solution was concentrated and subjected to purification by pre-HPLC to obtain 20 mg of desired product.

¹H-NMR (400MHz, DMSO-d6): δ(ppm) : 11.45 (s, 1H), 8.90 (s, 1H), 7.75-7.77(m, 2H), 7.46-7.52 (m, 3H), 7.12-7.20(m, 1H), 7.10-7.11(m, 1H), 6.75-6.78(m, 2H), 6.19 (s, 1H), 4.25(m, 2H), 3.11-3.19(m, 4H),1.67-1.70(m, 2H).

HPLC purity: @214nm 99.2 %, @254nm 99.3%.

LC-MS: *m*/*z* 555 [M+1].

### Example 16

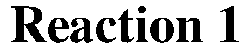

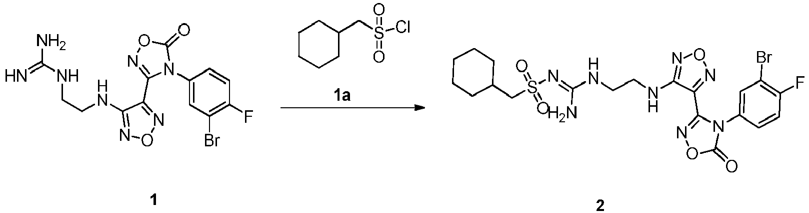

Compound **1** (106 mg, 0.25 mmol) was dissolved in acetone (10 mL), added with potassium carbonate (138 mg, 1 mmol), added dropwise with Compound **1a** (49 mg, 0.25 mmol), stirred at room temperature overnight, quenched with water, and the resultant was extracted with ethyl acetate, dried over anhydrous sodium sulfate, and concentrated to give 100 mg of a crude product.

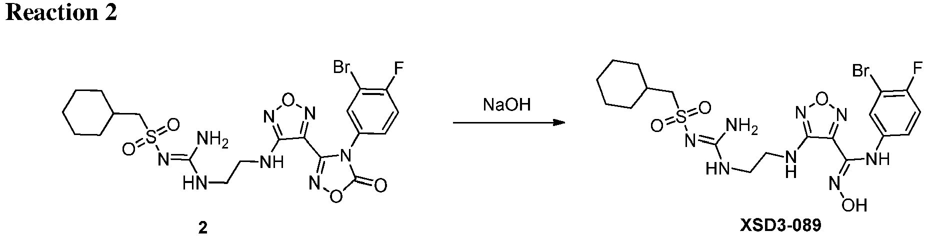

Compound **2** (100 mg) was dissolved in methanol, stirred at room temperature for 10 min, added dropwise with 1N sodium hydroxide (1 mL), stirred at room temperature for 10 min. The reaction solution was concentrated and subjected to purification by pre-HPLC to obtain 12 mg of desired product.

¹H-NMR (400MHz, DMSO-d6): δ(ppm) : 11.45 (s, 1H),8.90(s, 1H), 7.10-7.21 (m, 2H), 6.76-7.17(m, 4H), 6.28 (m, 1H),3.33-3.40 (m, 4H), 2.76 (m, 2H), 0.98-1.86 (m, 11H).

HPLC purity: @214nm93.7%, @254nm 97.6%.

LC-MS: *m*/*z* 563 [M+1].

### Example 17

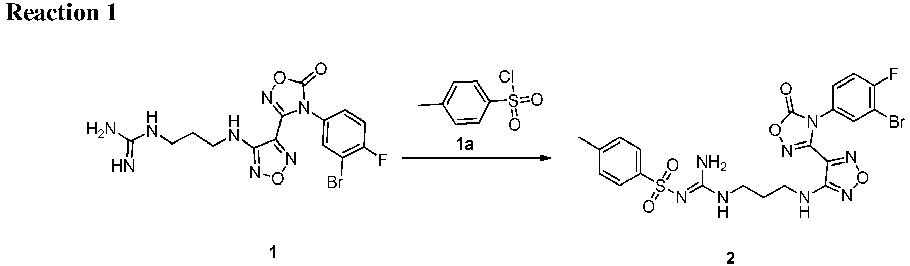

Compound **1** (110 mg, 0.25 mmol) was dissolved in acetone (10 mL), added with potassium carbonate (138 mg, 1 mmol), added dropwise with Compound **1a** (47.5 mg, 0.25 mmol), stirred at room temperature overnight, quenched with water. The resultant was extracted with ethyl acetate, dried over anhydrous sodium sulfate, concentrated to obtain 100 mg of a crude product.

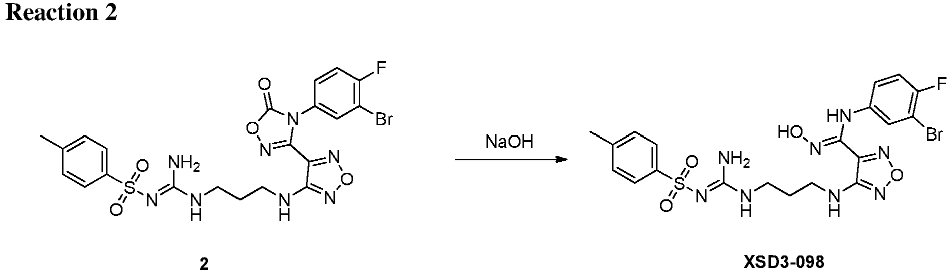

Compound **2** (100 mg) was dissolved in methanol, stirred at room temperature for 10 min, added dropwise with 1N sodium hydroxide (1 mL), stirred at room temperature for 10 min, and the reaction solution was concentrated and subjected to purification by pre-HPLC to prepare 15 mg of desired product.

¹H-NMR (400MHz, CDCl3): δ(ppm) : 9.96 (s, 1H),7.77-7.79(m, 2H), 6.18-7.32 (m, 9H), 3.31-3.42(m, 4H),2.40 (s, 3H),1.76 (m, 2H).

HPLC purity: @214nm98.6%, @254nm 98.9%.

LC-MS: *m*/*z* 571 [M+1].

### Example 18

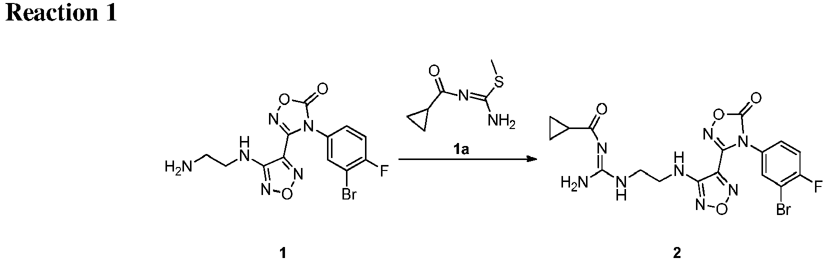

Compound **1** (96 mg, 10 mmol) and Compound **1a** (48 mg, 0.25 mmol) were dissolved in acetonitrile (10 mL), stirred at room temperature overnight, quenched with water. The resultant was extracted with ethyl acetate, dried over anhydrous sodium sulfate and concentrated to give 100 mg of a crude product.

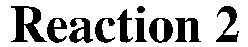

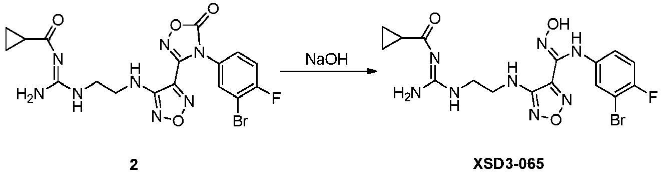

Compound **2** (100 mg) was dissolved in methanol, stirred at room temperature for 10 min, added dropwise with 1N sodium hydroxide (1 mL), stirred at room temperature for 10 min. The reaction solution was concentrated and subjected to purification by pre-HPLC to prepare 15 mg of desired product.

¹H-NMR (400MHz, DMSO-d6): δ(ppm) : 11.76 (s, 1H), 11.45 (s, 1H), 8.68-9.04(m, 3H), 7.09-7.20 (m, 2H), 6.73-6.77(m, 1H), 6.33-6.36 (m, 1H), 3.53-3.54 (m, 2H), 3.44-3.46 (m, 2H),1.18(m, 1H),

1.00-1.02 (m, 2H), 0.90-0.92 (m, 2H).

HPLC purity: @214nm 98.8 %, @254nm 99.8%.

LC-MS: *m*/*z* 471 [M+1].

### Example 19

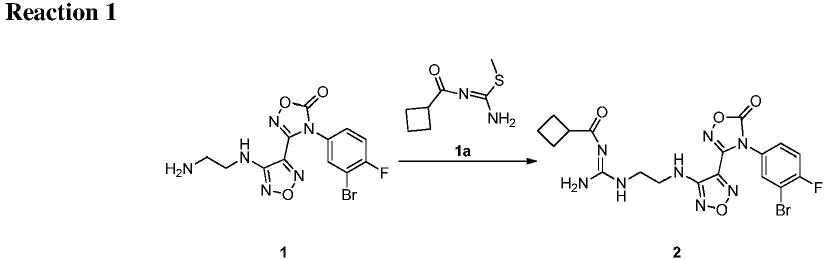

Compound **1** (96 mg, 0.25 mmol) and Compound **1a** (48 mg, 0.25 mmol) were dissolved in acetonitrile (10 mL), stirred at room temperature overnight, quenched with water. The resultant was extracted with ethyl acetate, dried over anhydrous sodium sulfate, and concentrated to obtain 100 mg of a crude product.

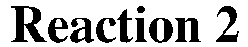

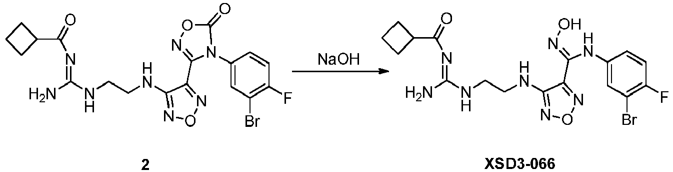

Compound **2** (100 mg) was dissolved in methanol, stirred at room temperature for 10 min, added dropwise with 1N sodium hydroxide (1 mL), stirred at room temperature for 10 min. The reaction solution was concentrated and subjected to purification by pre-HPLC to prepare 12 mg of desired product.

¹H-NMR (400MHz, DMSO-d6): δ(ppm) : 11.48 (s, 1H), 11.28 (s, 1H), 8.75-8.92(m, 3H), 7.09-7.20 (m, 2H), 6.73-6.77(m, 1H), 6.35-6.38 (m, 1H), 3.53-3.54 (m, 2H), 3.44-3.46 (m, 2H), 3.24-3.28 (m, 2H), 2.13-2.20 (m, 3H),1.77-1.98(m, 2H).

HPLC purity: @214nm 97.9 %, @254nm 98.6%.

LC-MS: *m*/*z* 485 [M+1].

### Example 20

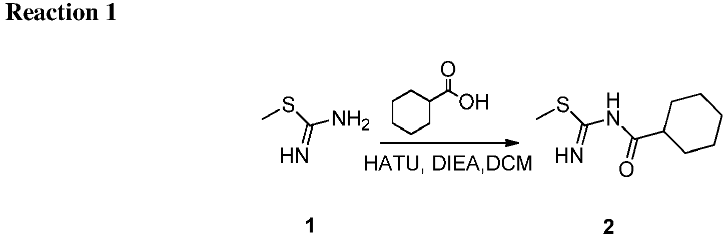

Compound **1** (500 mg, 5.5 mmol) was dissolved in 3 mL of dichloromethane, and O-(7-azabenzotriazole)-N,N,N',N'-tetramethyluronium hexafluorophosphate (128 mg, 0.336 mmol), cyclohexanecarboxylic acid (800 mg, 6.25 mmol) and diisopropylethylamine (1.16 g, 896 mmol) were added in sequence, the reaction was carried out at room temperature overnight under the protection of nitrogen gas, quenched with water, the resultant was extracted with ethyl acetate (50 mL × 5), the extract liquors were combined and washed with a saturated aqueous solution of sodium chloride and dried over anhydrous sodium sulfate, evaporated to dryness under reduced pressure to obtain 40 mg of product, which was directly used in the next step.

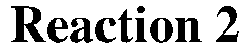

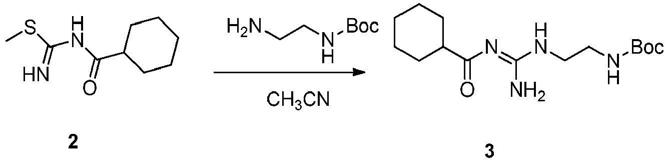

Compound **2** (530 mg, 2.65 mmol) was dissolved in acetonitrile (5 mL), added with compound **2a** (850 mg, 5.3 mmol), stirred at room temperature for 24 h, and directly evaporated to dryness under reduced pressure to give 300 mg of product that was directly used in the next step.

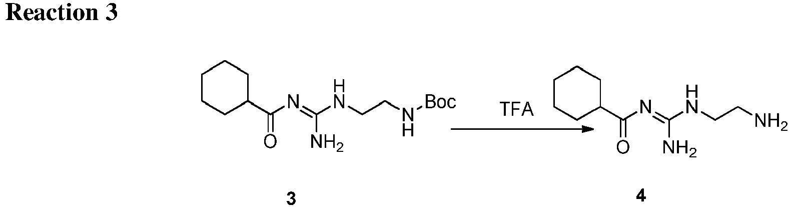

Compound **3** (300 mg, 0.99 mmol) was dissolved in dichloromethane (10 mL), stirred at room temperature for 5 min, added with trifluoroacetic acid (5 mL), reacted at room temperature for 2 h, and the resultant was directly evaporated to dryness under reduced pressure to give 200 mg of the desired product **4,** which was a crude product and directly used in the next step.

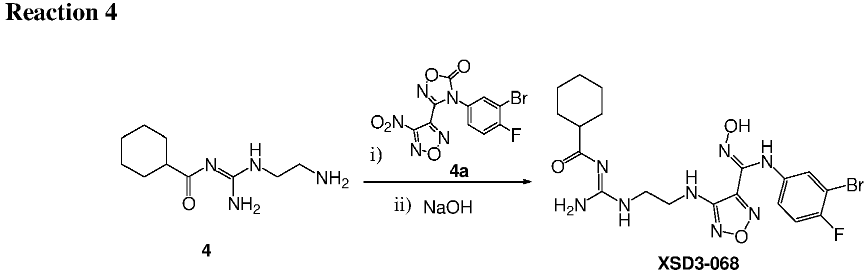

Compound **4** (200 mg, 0.99 mmol) was dissolved in tetrahydrofuran, added with Compound **4a** (250 mg, 1.0 mmol), stirred at room temperature for 10 min, added dropwise with 1N sodium hydroxide (1 mL), stirred at room temperature for 10 min. The reaction solution was concentrated and subjected to purification by pre-HPLC to prepare 3 mg of desired product.

¹H-NMR (400MHz, DMSO-d6): δ(ppm) : 11.90 (s, 1H),11.55 (s, 1H), 8.67-9.12(m, 3H), 7.10-7.20 (m, 2H),6.73-6.77(m, 1H), 6.37 (s, 1H), 3.33-3.54 (m, 4H),2.16-2.21(m, 1H), 1.56-1.81 (m, 6H), 1.15- 1.36 (m, 1H).

HPLC purity: @214nm 97.7 %, @254nm 98.0%.

LC-MS: *m*/z511 [M+1].

### Example 21

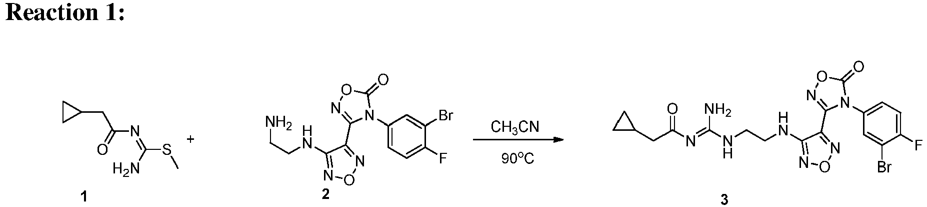

Compound **1** (35 mg, 0.2 mmol) and Compound **2** (90 mg, 0.22 mmol) were dissolved in 10 mL of acetonitrile and stirred at 90°C for 16 h. The reaction solution was directly evaporated to dryness under reduced pressure to give Compound **3,** which was a crude product and directly used in the next step (100 mg).

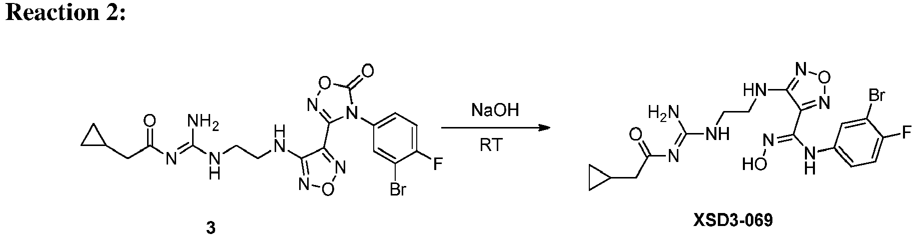

Compound **3** (100 mg, 0.1 mmol) was dissolved in a solution of 1.5 N NaOH (2 mL) in THF (5 mL), stirred at room temperature for 0.5 hour, and the resultant was subjected to purification by pre-HPLC to prepare the desired compound (30 mg, yield: 30%).

HNMR (DMSO, 400M): 11.40 (s, 1H), 11.33 (s, 1H), 9.09 (s, 1H), 8.93 (s, 1H), 8.,7 (s, 1H), 7.09-7.20 (m, 2H), 6.73-6.77 (m, 1H),6.36 (s, 1H)3.45-3.55 (m, 4H) ,2.31-2.33(m, 2H), 0.95-0.99 (m,1H), 0.47-0.51 (m, 2H),0.16-0.20(m, 2H).

HPLC purity: @214nm 98.8 %, @254nm 98.9%.

LC-MS: m/z 483.1 [M+1].

### Example 22

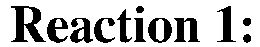

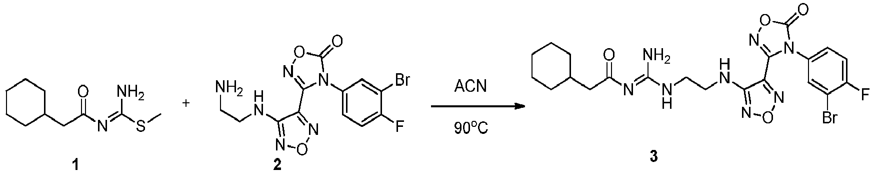

Compound **1** (44 mg, 0.2 mmol) and Compound **2** (90 mg, 0.22 mmol) were dissolved in 10 mL of acetonitrile and stirred at 90°C for 16 h. The reaction solution was directly evaporated to dryness under reduced pressure to give Compound **3,** which was a crude product and used directly in the next step (100 mg).

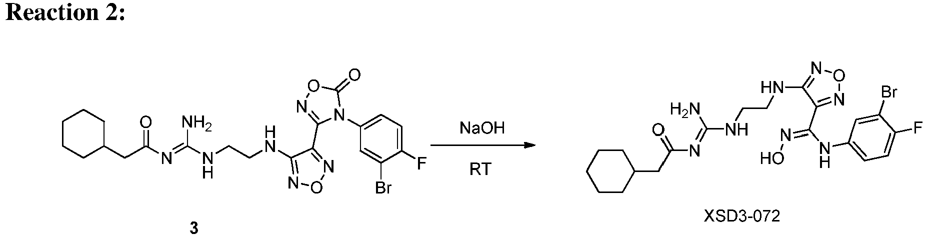

Compound **3** (100 mg, 0.1 mmol) was dissolved in a solution of 1N NaOH (4 mL) in tetrahydrofuran (5 mL), stirred at room temperature for 1 hour, and subjected to purification by pre-HPLC to prepare the desired compound (35 mg, yield: 35%).

HNMR (DMSO, 400M): 11.40 (s, 1H), 11.33 (s, 1H), 9.18 (s, 1H), 9.02 (s, 1H), 8.78 (s, 1H, 7.10-7.17 (m, 2H), 6.73-6.76 (m, 1H),6.35-6.38 (m, 1H)3.46-3.65 (m, 4H) ,2.26-2.38(m, 2H) , 1.47-1.69 (m,5H), 0.80-1.22 (m,6H),

HPLC purity: @214nm 96.5%, @254nm 96.5%.

LC-MS: m/z527.2 [M+1].

### Example 23

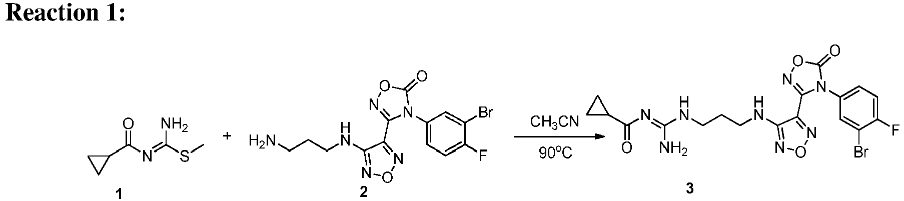

Compound **1** (35 mg, 0.26 mmol) and Compound **2** (90 mg, 0.22 mmol) were dissolved in 10 mL of acetonitrile and stirred at 90°C for 20 h. The reaction solution was directly evaporated to dryness under reduced pressure to give Compound **4,** which was a crude product and used directly in the next step (110 mg).

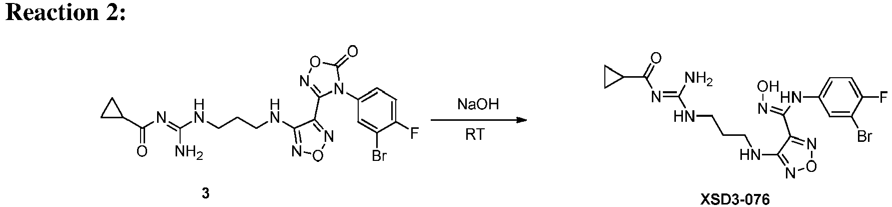

Compound **3** (100 mg, 0.2 mmol) was dissolved in a solution of 1N NaOH (1.5 mL) in tetrahydrofuran (5 mL), stirred at room temperature for 0.5 hour, and the resultant was subjected to purification by pre-HPLC to prepare the desired compound (15 mg, yield: 15%).

HNMR (DMSO, 400M): 12.50 (s, 1H), 11.50 (br, 1H), 9.19 (s, 1H), 8.65-8.91 (m, 2H)7.12-7.21 (m, 2H), 6.73-6.77 (m, 1H),6.36 (s, 1H)3.24-3.36 (m, 4H) ,1.77-1.84(m, 3H) , 0.90-1.02(m, 4H).

HPLC purity: @214nm 98.6%, @254nm 98.3%.

LC-MS: m/z481.1 [M+1].

### Example 24

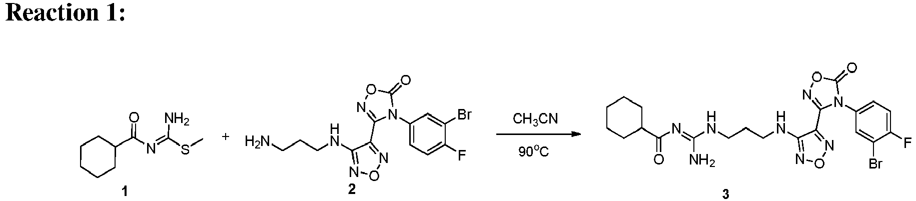

Compound **1** (42 mg, 0.2 mmol) and Compound **2** (90 mg, 0.22 mmol) were dissolved in 10 mL of acetonitrile and stirred at 90°C for 20 h. The reaction solution was directly evaporated to dryness under reduced pressure to give Compound **4,** which was a crude product and used directly in the next step (120 mg).

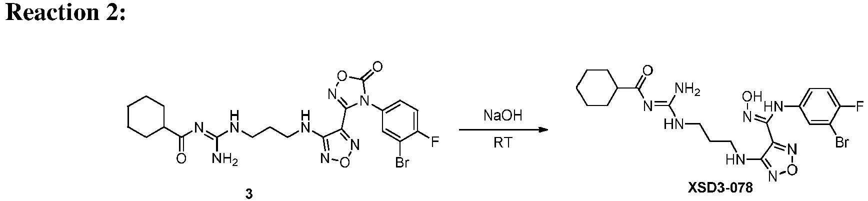

Compound **3** (110 mg, 0.2 mmol) was dissolved in a solution of 1N NaOH (5 mL) in tetrahydrofuran (5 mL), stirred at room temperature for 1 hour, and the resultant was subjected to purification by pre-HPLC to prepare the desired compound (35 mg, yield: 35%).

HNMR (DMSO, 400M): 11.44 (s, 1H), 11.35 (s, 1H), 9.06 (s, 1H), 8.92 (s, 1H), 8.64 (s, 3H), 7.16-7.21 (t, 1H),7.09-7.11 (q, 1H), 6.74-6.78 (m, 1H),6.26-6.29 (m, 1H),3.23-3.34 (m, 4H) ,2.49-2.50 (m, 1H) , 1.61-1.81(m, 7H),1.17-1.32(m, 5H).

HPLC purity: @214nm 99.4%, @254nm 99.8%.

LC-MS: m/z527.2 [M+1].

### Example 25

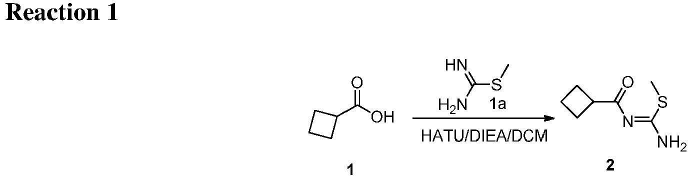

In two parallel pots, Compound 1 (500 mg, 8.77 mmol) was dissolved in DCM, added with compound **1a** (3.3 g, 21.93 mmol), HATU (4.01 g, 5.52 mmol), and DIEA (3.71 g, 56.5 mmol), stirred at room temperature overnight, quenched with water, the resultant was extracted with ethyl acetate, dried over anhydrous sodium sulfate, and subjected to purification by column chromatography to obtain 128mg of product.

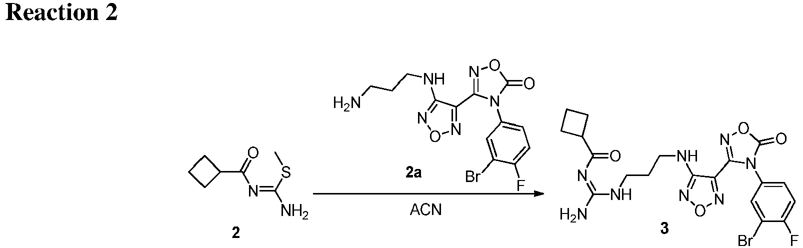

Compound **2** (128 mg, 0.743 mmol) was dissolved in ACN, added with compound **2a** (300 mg, 0.752 mmol), stirred at 90°C overnight. The resultant was extracted with ethyl acetate, dried over anhydrous sodium sulfate, and concentrated to obtain 300 mg of crude product.

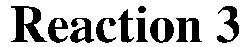

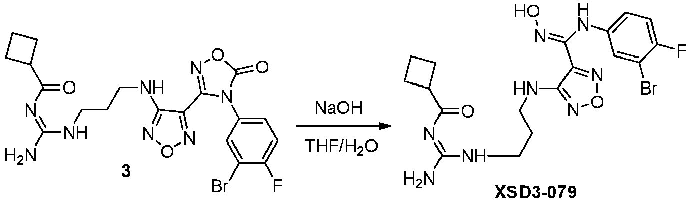

Compound **3** (300 mg) was dissolved in THF/H₂O, stirred at room temperature for 10 min, added dropwise with 1N sodium hydroxide (1 mL), and stirred at room temperature for 10 min. After the hydrolysis was completed when monitored by LCMS, 1M HCl was added dropwise to neutral, the reaction solution was extracted with EA, and the extract was concentrated and subjected to purification by pre-HPLC (acid method) to prepare 25 mg of desired product.

¹H-NMR (400MHz, DMSO-d6): δ(ppm) : 11.341 (s, 2H), 8.649-8.923(m, 4H), 6.274-7.188(m, 4H), 3.255-3.324 (m, 4H), 1.694-2.067(m,9H).

HPLC purity: @214nm99.05%, @254nm 98.1%.

LC-MS: *m*/*z*497[M+1].

### Example 26

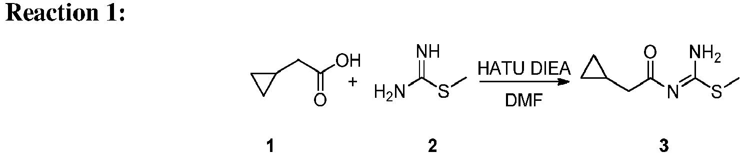

Compound **1** (1.0 g, 10 mmol) and Compound **2** (2.7 mg, 30 mmol) were dissolved in 20 mL of DMF and stirred at room temperature, then added with HATU (3.8 g, 10 mmol), DIEA (6.5 g, 50 mmol) and stirred for 10 h. The reaction solution was added to 60 mL of water, extracted with ethyl acetate, and the extract was evaporated to dryness under reduced pressure and subjected to purification by column chromatography to obtain Compound **3** (0.4 g, yield: 24%).

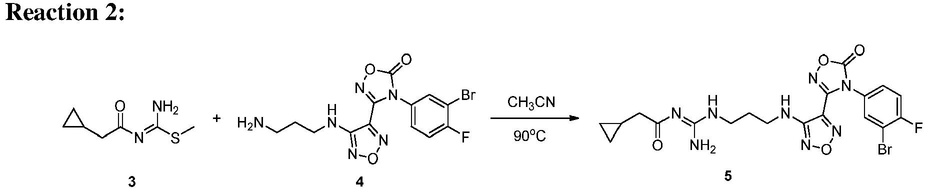

Compound **3** (35 mg, 0.2 mmol) and Compound **4** (90 mg, 0.22 mmol) were dissolved in 10 mL of acetonitrile and stirred at 90°C for 20 h, and the reaction solution was directly evaporated to dryness under reduced pressure to give Compound 5, which was a crude product and used directly in the next step (120 mg) .

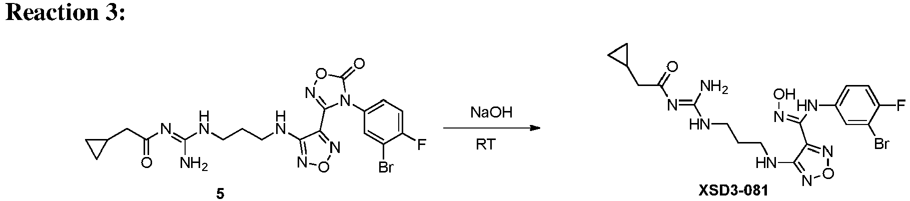

Compound **3** (110 mg, 0.2 mmol) was dissolved in a solution of 1 N NaOH (4 mL) in tetrahydrofuran (5 mL), stirred at room temperature for 1 hour, and the resultant was subjected to purification by pre-HPLC to obtain the desired compound (15 mg, yield: 15%).

HNMR (DMSO, 400M): 11.43 (s, 1H), 11.27 (s, 1H), 9.06 (s, 1H), 8.92 (s, 1H), 8.64 (s, 3H), 7.16-7.21 (t, 1H),7.09-7.11 (q, 1H), 6.74-6.78 (m, 1H),6.26-6.29 (m, 1H),3.23-3.35 (m, 4H) ,2.32-2.34 (m, 2H), 1.80-1.84(m, 2H),0.95-1.01(m, 1H),0.49-0.51(m, 5H),0.18-0.19(m, 2H).

HPLC purity: @214nm 96.5%, @254nm 97.9%.

LC-MS: m/z497.2 [M+1].

### Example 27

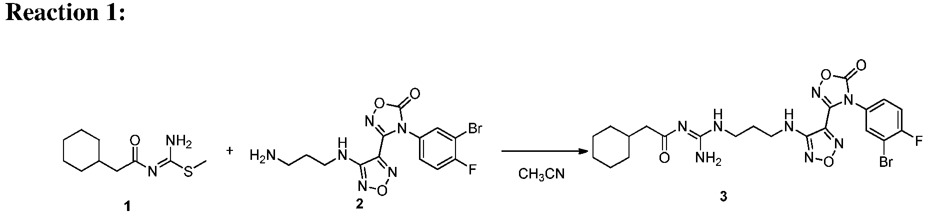

Compound **1** (45 mg, 0.2 mmol) and Compound **2** (90 mg, 0.22 mmol) were dissolved in 10 mL of acetonitrile and stirred at 90°C for 20 h, and the reaction solution was directly evaporated to dryness under reduced pressure to give Compound **4,** which was a crude product and used directly in the next step (120 mg crude, y>99%).

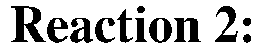

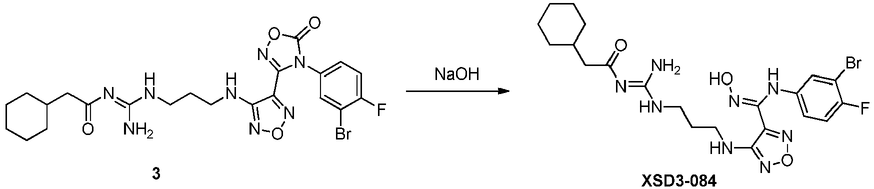

Compound **3** (110 mg, 0.2 mmol) was dissolved in a solution of 1N NaOH (4 mL) in tetrahydrofuran (5 mL) and stirred at room temperature for 1 hour, and the resultant was subjected to purification by pre-HPLC to prepare the desired compound (55 mg y=50%).

HNMR (DMSO, 400M): 11.53 (s, 1H), 11.44 (s, 1H), 9.13 (s, 1H), 8.92 (s, 1H), 8.67 (s, 1H), 7.16-7.21 (t, 1H), 7.09-7.11 (q, 1H), 6.74-6.78 (m, 1H),6.26-6.29 (m, 1H),3.23-3.34 (m, 4H) ,2.27-2.28 (m, 2H), 1.66-1.84(m, 8H) ,0.91-1.17(m, 5H).

LC-MS: *m*/*z* 583.2 [M+1].

### Example 28

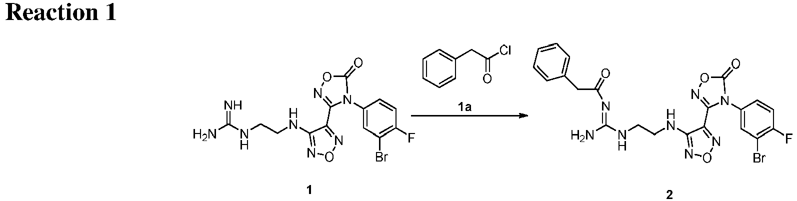

Compound **1** (106 mg, 0.25 mmol) was dissolved in acetone (10 mL), added with potassium carbonate (138 mg, 1 mmol), added dropwise with Compound **1a** (43.5 mg, 0.25 mmol), stirred at room temperature overnight, quenched with water. The resultant was extracted with ethyl acetate, dried over anhydrous sodium sulfate, and concentrated to obtain 100 mg of a crude product.

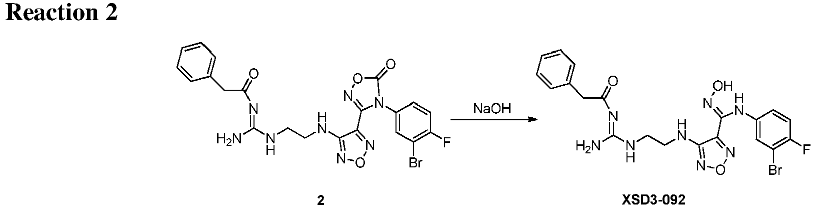

Compound **2** (100 mg) was dissolved in methanol, stirred at room temperature for 10 min, added dropwise with 1N sodium hydroxide (1 mL), stirred at room temperature for 10 min, and the reaction solution was concentrated and subjected to purification by pre-HPLC to prepare 9 mg of desired product.

¹H-NMR (400MHz, DMSO-d6): δ(ppm) : 11.48 (s, 1H), 11.44 (s, 1H), 8.92-8.95(m, 3H), 7.27-7.34 (m, 5H), 7.09-7.17(m, 2H), 6.74-6.76 (m, 1H), 6.32-6.35 (m, 1H), 3.75-3.77 (m, 2H), 3.50-3.56 (m, 2H), 3.44-3.46 (m, 2H).

HPLC purity: @214nm 97.9 %, @254nm 99.2%.

LC-MS: *m*/*z* 521 [M+1].

### Example 29

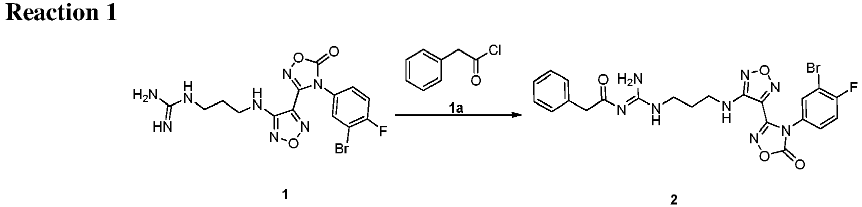

Compound **1** (110 mg, 0.25 mmol) was dissolved in acetone (10 mL), added with potassium carbonate (138 mg, 1 mmol), added dropwise with Compound **1a** (43.5 mg, 0.25 mmol), stirred at room temperature overnight, quenched with water. The resultant was extracted with ethyl acetate, dried over anhydrous sodium sulfate, and concentrated to obtain 100 mg of a crude product.

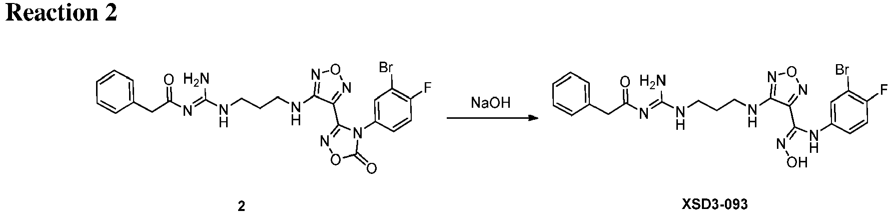

Compound **2** (100 mg) was dissolved in methanol, stirred at room temperature for 10 min, added dropwise with 1N sodium hydroxide (1 mL), stirred at room temperature for 10 min, and the reaction solution was concentrated and subjected to purification by pre-HPLC to prepare 9 mg of desired product.

¹H-NMR (400MHz, DMSO-d6): δ(ppm) : 11.64 (s, 1H), 11.42 (s, 1H), 9.03-9.07(m, 3H), 7.27-7.34 (m, 5H), 7.09-7.17(m, 2H), 6.74-6.76 (m, 1H), 6.32-6.35 (m, 1H), 3.75-3.77 (m, 2H), 3.14-3.34 (m, 4H), 1.80-1.84 (m, 2H).

HPLC purity: @214nm 96.0%, @254nm 96.0%.

LC-MS: *m*/*z* 535 [M+1].

### Example 30

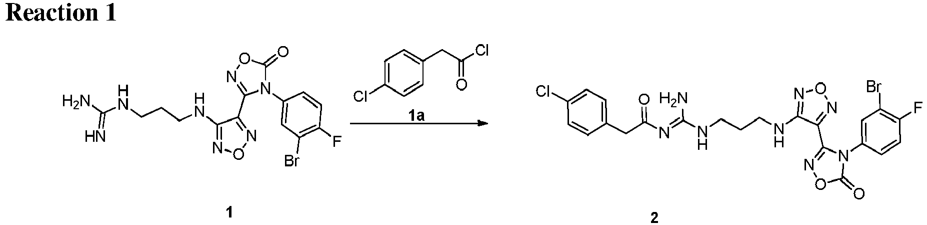

Compound **1** (330 mg, 0.75 mmol) was dissolved in acetone (30 mL), added with potassium carbonate (414 mg, 3 mmol), added dropwise with Compound **1a** (130.5 mg, 0.75 mmol), stirred at room temperature overnight, quenched with water. The resultant was extracted with ethyl acetate, dried over anhydrous sodium sulfate, and concentrated to obtain 300 mg of a crude product.

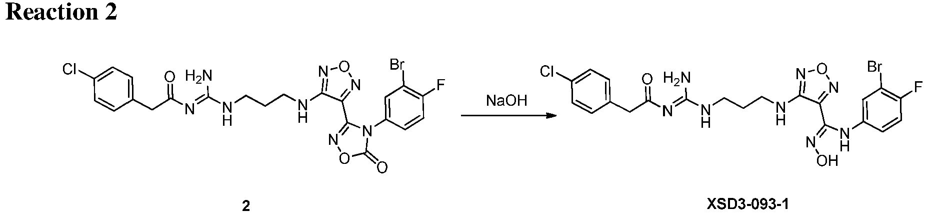

Compound **2** (300 mg) was dissolved in methanol, stirred at room temperature for 10 min, added dropwise with 1N sodium hydroxide (1 mL), stirred at room temperature for 10 min, and the reaction solution was concentrated and subjected to purification by pre-HPLC to obtain 59 mg of desired product.

¹H-NMR (400MHz, DMSO-d6): δ(ppm) : 11.64 (s, 1H), 11.42 (s, 1H), 9.03-9.07(m, 4H), 7.32-7.35 (m, 2H), 7.15-7.32(m, 3H), 7.08-7.11(m, 1H) 6.74-6.77 (m, 1H), 6.25-6.28 (m, 1H), 3.75-3.77 (m, 2H), 3.14-3.34 (m, 4H), 1.80-1.84 (m, 2H).

HPLC purity: @214nm 99.90%, @254nm 99.70%.

LC-MS: *m*/z569[M+1].

### Example 31

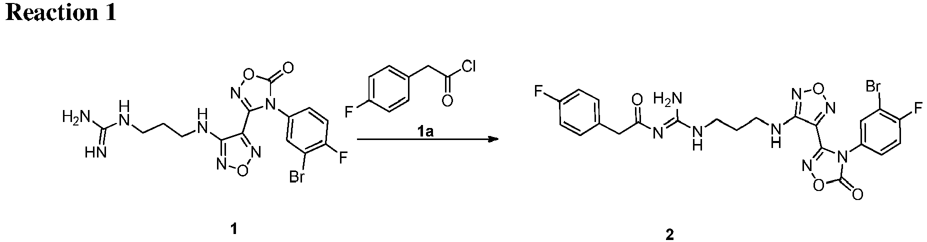

Compound **1** (330 mg, 0.75 mmol) was dissolved in acetone (30 mL), added with potassium carbonate (414 mg, 3 mmol), added dropwise with Compound **1a** (130.5 mg, 0.75 mmol), stirred at room temperature overnight, quenched with water. The resultant was extracted with ethyl acetate, dried over anhydrous sodium sulfate, and concentrated to obtain 300 mg of a crude product.

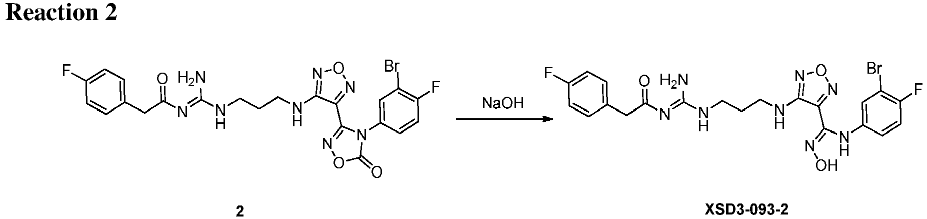

Compound **2** (300 mg) was dissolved in methanol, stirred at room temperature for 10 min, added dropwise with 1N sodium hydroxide (1 mL), stirred at room temperature for 10 min, and the reaction solution was concentrated and subjected to purification by pre-HPLC to obtain 35 mg of desired product.

¹H-NMR (400MHz, DMSO-d6): δ(ppm) : 11.64 (s, 1H),11.42 (s, 1H), 9.03-9.07(m, 4H), 7.32-7.35 (m, 2H),7.15-7.32(m, 3H), 7.08-7.11(m, 1H)6.74-6.77 (m, 1H), 6.25-6.28 (m, 1H), 3.75-3.77 (m, 2H),3.14-3.34 (m, 4H),1.80-1.84 (m, 2H).

HPLC purity: @214nm 99.40%, @254nm 99.25%.

LC-MS: *m*/*z*553[M+1].

### Example 32

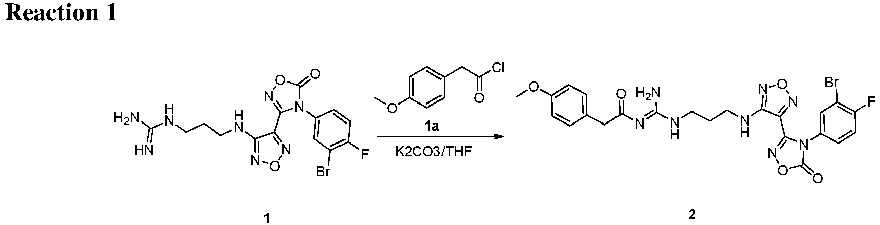

Compound **1** (330 mg, 0.75 mmol) was dissolved in tetrahydrofuran (10 mL), added with potassium carbonate (414 mg, 3 mmol), stirred at room temperature for 20 min, added dropwise with a solution of Compound **1a** (184 mg, 1 mmol) in tetrahydrofuran (1 mL), stirred at room temperature for 2 h, quenched with water. The resultant was extracted with ethyl acetate, dried over anhydrous sodium sulfate, and concentrated to obtain 500 mg of a crude product.

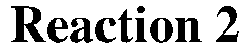

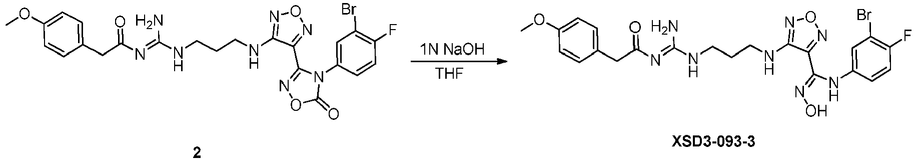

Compound **2** (500 mg) was dissolved in tetrahydrofuran (20 mL), stirred at room temperature for 5 min, added dropwise with 1N sodium hydroxide (5 mL), stirred at room temperature for 30 min. The reaction solution was extracted with water and ethyl acetate, and dried over anhydrous sodium sulfate, and concentrated to obtain a crude product. The crude product was dissolved in acetonitrile, and subjected to purification by pre-HPLC to prepare obtain 29 mg of desired product.

¹H-NMR (400MHz, DMSO-d6): δ(ppm) : 11.91(s, 1H),11.43 (s, 1H), 9.04(s, 1H), 8.90 (s, 1H),8.70(s,2H),7.25-7.23(m, 2H), 7.20-7.16 (m, 1H), 7.11-7.09 (m, 1H), 6.91-6.88(m,2H), 6.78-6.75(m,1H), 6.25(m,1H), 3.73(s,3H),3.68(s,2H), 3.28-3.23(m,4H),1.83-1.80(m,2H).

HPLC purity: @214nm 99.2%, @254nm 99.7%.

LC-MS: *m*/*z*563.2[M+1].

### Example 33

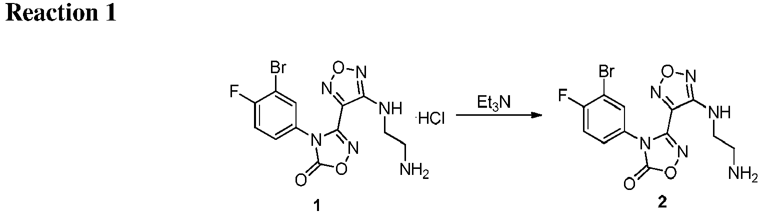

Compound **1** (150 mg, 0.47 mmol) was dissolved in DCM, added with Et₃N, stirred at room temperature for 1 h, quenched with water. The resultant was extracted with ethyl acetate, dried over anhydrous sodium sulfate, and concentrated to obtain 100 mg of crude product.

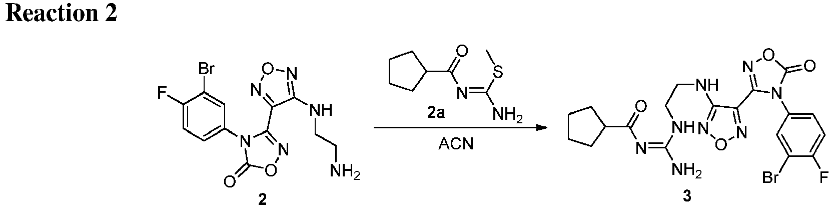

Compound **2** (100 mg, 1.08 mmol) was dissolved in ACN, added with compound **2a** (120 mg, 1.06 mmol), stirred at 90 °C overnight. The resultant was extracted with ethyl acetate, dried over anhydrous sodium sulfate, and concentrated to obtain 100 mg of crude product.

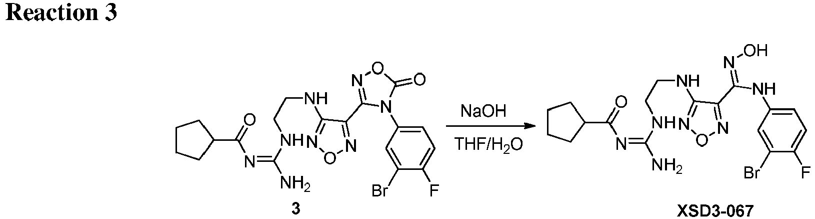

Compound **3** (100 mg) was dissolved in THF/H₂O, stirred at room temperature for 10 min, added dropwise with 1N sodium hydroxide (1 mL), stirred at room temperature for 10 min, and the reaction solution was concentrated and subjected to purification by pre-HPLC to obtain 8 mg of desired product.

¹H-NMR (400MHz, DMSO-d6): δ(ppm) : 11.579 (s, 1H),11.480 (s, 1H), 8.718-8.917(m, 3H), 6.286-7.187(m, 4H), 3.255-3.324 (m, 4H),2.499-2.507(m, 1H), 1.570-1.843(m,8H).

HPLC purity: @214nm97.3%, @254nm 98.1%.

LC-MS: *m*/*z*497[M+1].

### Example 34

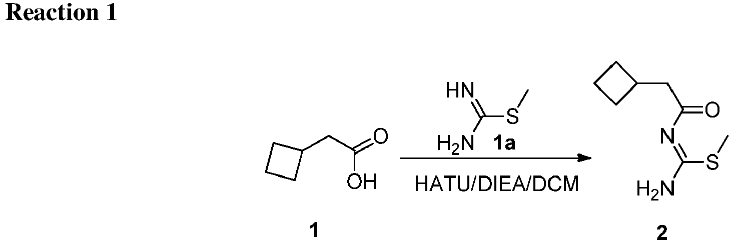

Compound **1** (500 mg, 8.77 mmol) was dissolved in DCM, added with compound **1a** (3.3 g, 21.93 mmol), HATU (4.01 g, 5.52 mmol), and DIEA (3.71 g, 56.5 mmol), stirred at room temperature overnight, quenched with water. The resultant was extracted with ethyl acetate, dried over anhydrous sodium sulfate, and subjected to purification by column chromatography to obtain 120 mg of desired product.

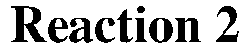

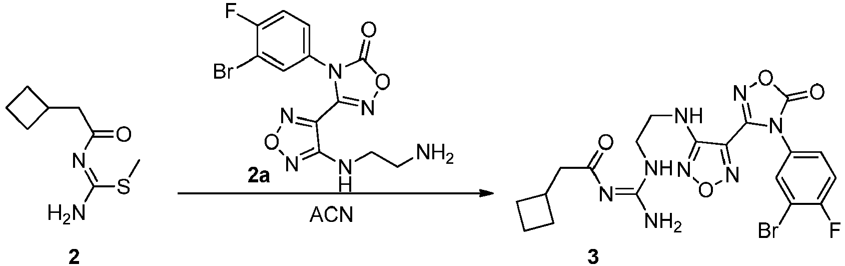

Compound **2** (120 mg, 1.08 mmol) was dissolved in ACN, added with compound **2a** (100 mg, 1.06 mmol), stirred at 90 °C overnight. The resultant was extracted with ethyl acetate, dried over anhydrous sodium sulfate, and concentrated to obtain 100 mg of crude product.

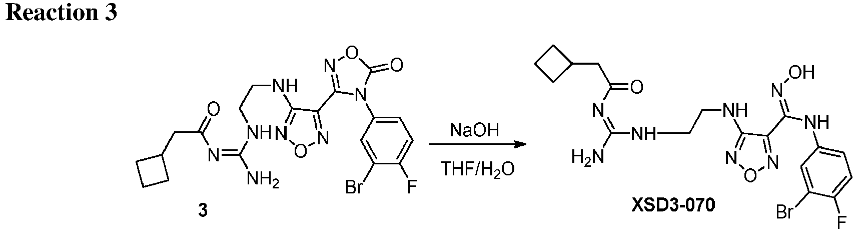

Compound **3** (100 mg) was dissolved in THF/H₂O, stirred at room temperature for 10 min, added dropwise with 1N sodium hydroxide (1 mL), stirred at room temperature for 10 min, and the reaction solution was concentrated and subjected to purification by pre-HPLC to give 12 mg of desired product.

¹H-NMR (400MHz, DMSO-d6): δ(ppm) : 12.041 (s, 1H), 8.649-8.923(m, 3H), 6.274-7.188(m, 4H), 3.255-3.324 (m, 4H),2.509-2.683(m, 2H), 1.594-2.067(m,6H).

HPLC purity: @214nm95.9%, @254nm 95.4%.

LC-MS: *m*/*z*497[M+1].

### Example 35

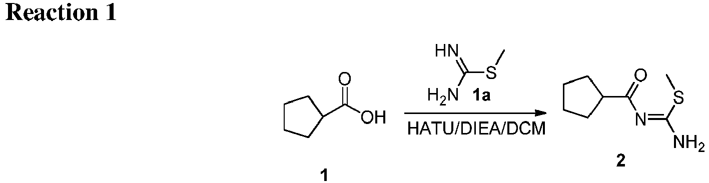

Compound **1** (1 g, 8.77 mmol) was dissolved in DCM, added with **1a** (6.3 g, 21.93 mmol), HATU (4.01 g, 10.52 mmol), and DIEA (5.71 g, 56.5 mmol), stirred at room temperature overnight, quenched with water. The resultant was extracted with ethyl acetate, dried over anhydrous sodium sulfate, and subjected to purification by column chromatography to give 200 mg of product.

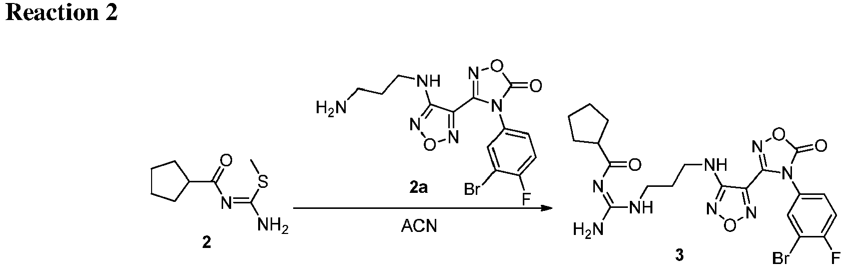

Compound **2** (200 mg, 1.08 mmol) was dissolved in ACN, added with compound **2a** (420 mg, 1.06 mmol), stirred at 90 °C overnight. The resultant was extracted with ethyl acetate, dried over anhydrous sodium sulfate, and concentrated to give 100 mg of a crude product.

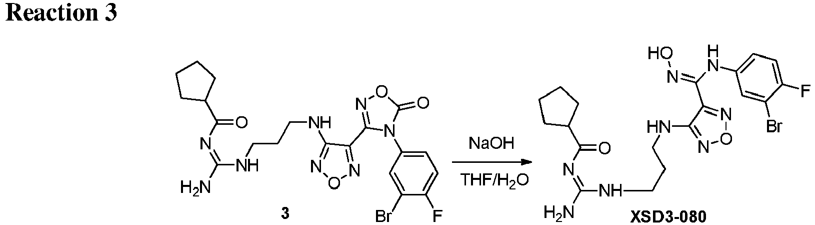

Compound **3** (100 mg) was dissolved in THF/H₂O, stirred at room temperature for 10 min, added dropwise with 1N sodium hydroxide (1 mL), and stirred at room temperature for 10 min, and the reaction solution was concentrated and subjected to purification by pre-HPLC to prepare 12 mg of desired product.

¹H-NMR (400MHz, DMSO-d6): δ(ppm) : 11.579 (s, 1H),11.480 (s, 1H), 8.718-8.917(m, 3H), 6.286-7.187(m, 4H), 3.255-3.324 (m, 4H),2.499-2.507(m, 1H), 1.570-1.843(m,10H).

HPLC purity: @214nm91.2%, @254nm 96.8%.

LC-MS: *m*/*z*511[M+1].

### Example 36

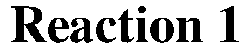

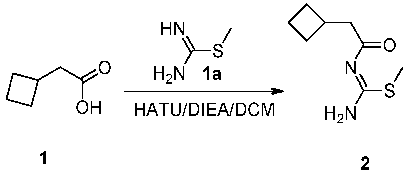

Compound **1** (500 mg, 8.77 mmol) was dissolved in DCM, added with compound **1a** (3.3 g, 21.93 mmol), HATU (4.01 g, 10.52 mmol), and DIEA (5.71 g, 56.5 mmol), stirred at room temperature overnight, quenched with water. The resultant was extracted with ethyl acetate, dried over anhydrous sodium sulfate, and subjected to purification by column chromatography to obtain 120 mg of desired product.

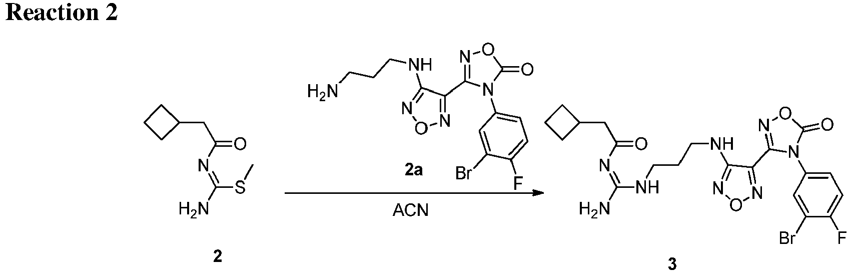

Compound **2** (120 mg, 1.08 mmol) was dissolved in ACN, added with compound **2a** (100 mg, 1.06 mmol), stirred at 90 °C overnight. The resultant was extracted with ethyl acetate, dried over anhydrous sodium sulfate, and concentrated to obtain 100 mg of a crude product.

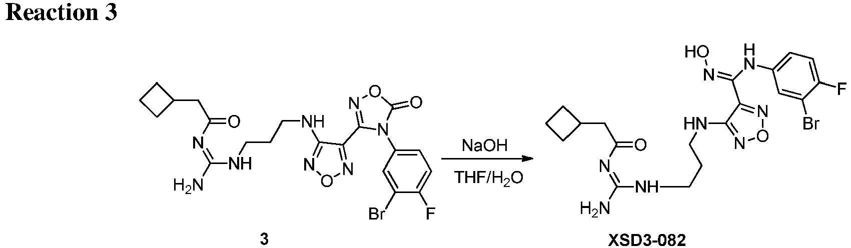

Compound **3** (100 mg) was dissolved in THF/H₂O, stirred at room temperature for 10 min, added dropwise with 1N sodium hydroxide (1 mL), stirred at room temperature for 10 min, and the reaction solution was concentrated and subjected to purification by pre-HPLC to give 12 mg of desired product.

¹H-NMR (400MHz, DMSO-d6): δ(ppm) : 11.441 (s, 2H), 8.649-8.923(m, 3H), 6.274-7.188(m, 4H), 3.255-3.324 (m, 4H), 1.694-2.067(m,11H).

HPLC purity: @214nm99.1%, @254nm 99.3%.

LC-MS: *m*/*z*511[M+1].

### Example 37

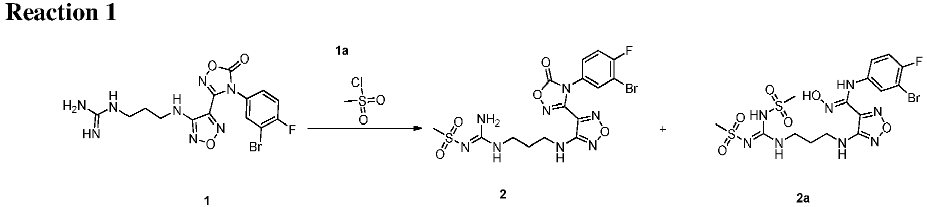

Compound **1** (110 mg, 0.25 mmol) was dissolved in acetone (10 mL), added with potassium carbonate (138 mg, 1 mmol), added dropwise with Compound **1a** (113 mg, 1 mmol), stirred at room temperature overnight, quenched with water. The resultant was extracted with ethyl acetate, dried over anhydrous sodium sulfate, and concentrated to obtain 100 mg of a crude product.

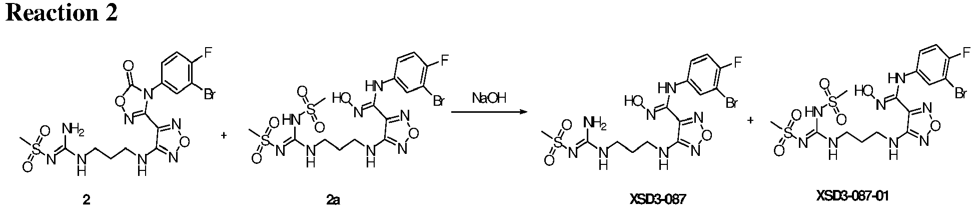

Compounds **2** and **2a** (100 mg) were dissolved in methanol, stirred at room temperature for 10 min, added dropwise with 1N sodium hydroxide (1 mL), stirred at room temperature for 10 min. The reaction solution was concentrated and subjected to purification by pre-HPLC to give the desired products XSD3-087 (15 mg) and XSD3-087-01 (17 mg).

XSD3-087 ¹H-NMR (400MHz,DMSO): δ(ppm) : 11.41 (s, 1H), 8.90(m, 1H), 6.18-7.32 (s, 1H),7.10-7.21(m, 2H),7.10-7.12 (m, 2H),6.76-6.79 (m, 1H), 6.23 (m, 1H), 3.12-3.23 (m, 4H), 6.76-6.79 (m, 1H), 2.67 (s, 3H),1.72-1.75 (m, 2H).

HPLC purity: @214nm93.5%, @254nm 94.6%.

LC-MS: *m*/*z*492 [M+1].

XSD3-087-01 ¹H-NMR (400MHz,DMSO): δ(ppm) : 11.43 (s, 1H),8.90(m, 1H), 8.01 (m, 2H), 7.10-7.20 (s, 2H),6.85 (m, 1H),6.23(m, 1H),3.72-3.76 (m, 2H), 3.40 (s, 3H), 3.24-3.26 (m, 2H), 2.93 (s, 3H), 1.87-1.91 (m, 2H).

HPLC purity: @214nm98%, @254nm 98.6%.

LC-MS: *m*/*z*570 [M+3].

### Example 38

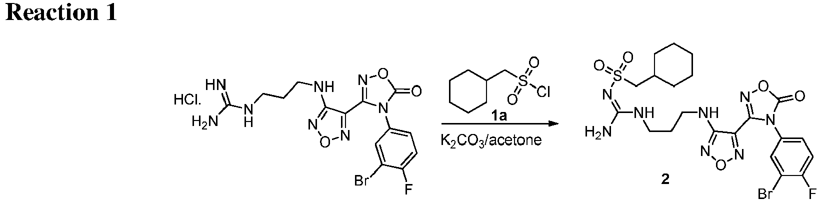

Compound **1** (100 mg, 0.21 mmol) was dissolved in acetone (10 mL), added with potassium carbonate (78 mg, 0.55 mmol), added dropwise with Compound **1a** (41 mg, 0.23 mmol), stirred at room temperature overnight, quenched with water. The resultant was extracted with ethyl acetate, dried over anhydrous sodium sulfate, and concentrated to obtain 100 mg of a crude product.

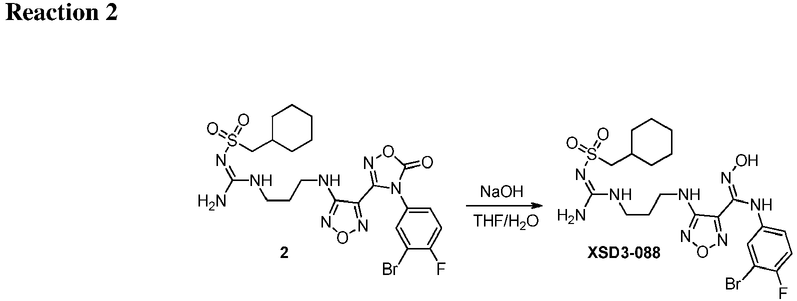

Compound **2** (100 mg) was dissolved in THF/H₂O, stirred at room temperature for 10 min, added dropwise with 1N sodium hydroxide (1 mL), stirred at room temperature for 10 min. The reaction solution was concentrated and subjected to pre-HPLC to prepare 5 mg of desired product of 5 mg.

¹H-NMR (400MHz, DMSO-d6): δ(ppm) : 11.421 (s, 1H),8.897(s, 1H), 7.153-7.197 (m, 2H),7.105-7.120 (m, 1H),6.746-7.098(m, 1H), 6.220 (s, 1H),4.078(s, 2H), 3.104-3.129 (m, 4H), 2.735-2.761(m, 2H), 1.535-1.853(m, 7H), 0.976-1.202(m, 6H).

HPLC purity: @214nm99.8%, @254nm 99.3%.

LC-MS: *m*/*z*575[M+1].

### Example 39

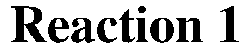

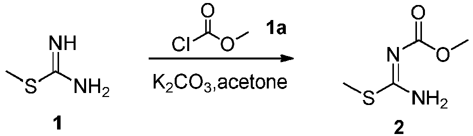

Compound **1** (1.0 g, 3.6 mmol) was dissolved in acetone (16 mL), added with potassium carbonate (993 mg, 7.2 mmol), added dropwise with Compound **1a** (254 mg, 2.7 mmol), stirred at room temperature overnight, quenched with water. The resultant was extracted with ethyl acetate, dried over anhydrous sodium sulfate, and concentrated to obtain 300 mg of a crude product.

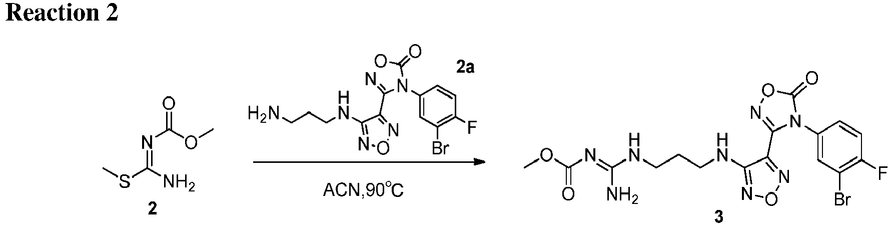

Compound **2a** (100 mg) was dissolved in acetonitrile, added with compound **2** (40 mg, 0.27 mmol), stirred at 90 °C for 12 h, quenched with water. The resultant was extracted with ethyl acetate, dried over anhydrous sodium sulfate, and concentrated to obtain 80 mg of a crude product.

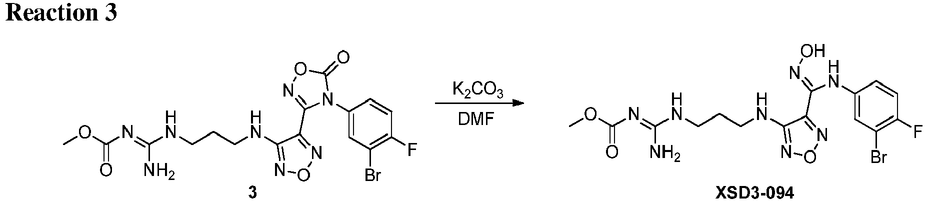

Compound **3** (80 mg) was dissolved in DMF, stirred at room temperature for 10 min, added with potassium carbonate (132 mg, 0.96 mmol), stirred at room temperature for 12 h, quenched with water. The resultant was extracted with ethyl acetate, dried over anhydrous sodium sulfate, concentrated to obtain 60 mg of a crude product, which was subjected to purification by pre-HPLC to obtain 14.2 mg of desired product.

¹H-NMR (400MHz, DMSO-d6): δ(ppm) : 11.53 (s, 1H), 8.91 (s, 1H), 8.58-8.27(m, 3H), 7.38-7.12 (m, 2H), 6.78(s, 1H), 6.28 (s, 1H), 3.65 (s, 3H), 3.28-3.24 (m, 4H), 1.79 (s, 2H).

HPLC purity: @214nm 94.5 %, @254nm 95.4%.

LC-MS: *m*/*z* 473 [M+1].

### Example 40

### Reaction 1

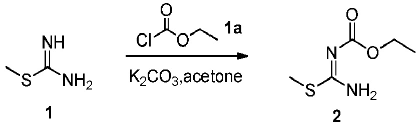

Compound **1** (1.0 g, 3.6 mmol) was dissolved in acetone (16 mL), added with potassium carbonate (993 mg, 7.2 mmol), added dropwise with Compound **1a** (294 mg, 2.7 mmol), stirred at room temperature overnight, quenched with water. The resultant was extracted with ethyl acetate, dried over anhydrous sodium sulfate, and concentrated to obtain 400 mg of a crude product.

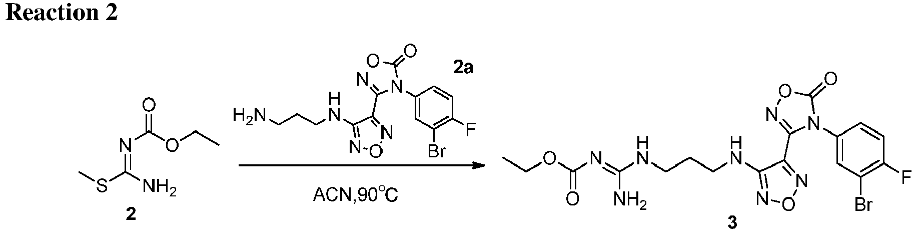

Compound **2a** (100 mg) was dissolved in acetonitrile, added with Compound **2** (42 mg, 0.27 mmol), stirred at 90 °C for 12 h, quenched with water. The resultant was extracted with ethyl acetate, dried over anhydrous sodium sulfate, and concentrated to obtain 66 mg of a crude product.

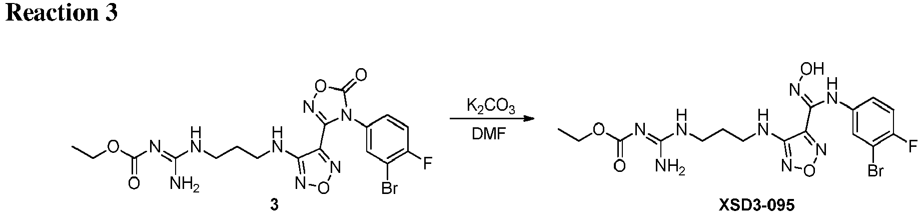

Compound **3** (66 mg) was dissolved in DMF, stirred at room temperature for 10 min, added with potassium carbonate (108 mg, 0.78 mmol), stirred at room temperature for 12 h, quenched with water. The resultant was extracted with ethyl acetate, dried over anhydrous sodium sulfate, and concentrated to obtain 50 mg of a crude product, which was subjected to purification by pre-HPLC to prepare 26.5 mg of desired product.

¹H-NMR (400MHz, DMSO-d6): δ(ppm) : 11.47 (s, 1H), 11.24 (s, 1H), 8.92(s, 1H), 8.62(s, 2H), 7.21-7.09 (m, 2H), 6.79-6.75 (m, 1H), 6.29-6.25 (m, 1H), 4.25-4.20 (m, 2H), 3.32-3.24 (m, 4H), 1.85-1.80 (m, 2H), 1.28-1.24 (m, 3H).

HPLC purity: @214nm 97.8 %, @254nm 98.6%.

LC-MS: *m*/*z* 487 [M+1].

### Example 41

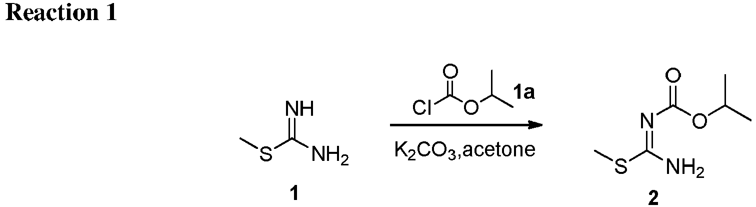

Compound **1** (1.0 g, 3.6 mmol) was dissolved in acetone (16 mL), added with potassium carbonate (993 mg, 7.2 mmol), added dropwise with Compound **1a** (732 mg, 6.0 mmol), stirred at room temperature overnight, quenched with water. The resultant was extracted with ethyl acetate, dried over anhydrous sodium sulfate, and concentrated to obtain 360 mg of a crude product.

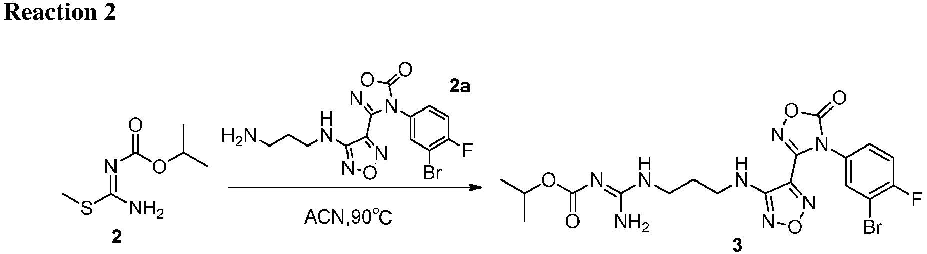

Compound **2a** (100 mg) was dissolved in acetonitrile, added with Compound **2** (45 mg, 0.27 mmol), stirred at 90 °C for 12 h, quenched with water. The resultant was extracted with ethyl acetate, dried over anhydrous sodium sulfate, and concentrated to obtain 51 mg of a crude product.

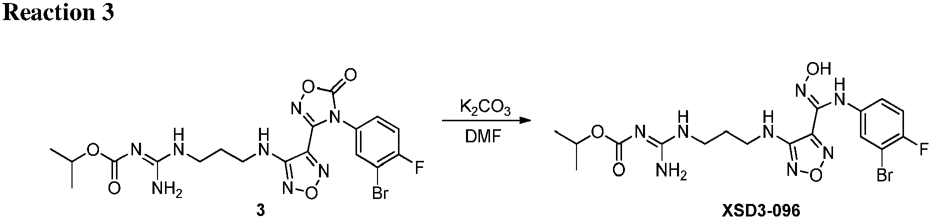

Compound **3** (51 mg) was dissolved in DMF, stirred at room temperature for 10 min, added with potassium carbonate (108 mg, 0.78 mmol), stirred at room temperature for 12 h, quenched with water. The resultant was extracted with ethyl acetate, dried over anhydrous sodium sulfate, and concentrated to obtain 50 mg of a crude product, which was subjected to purification by pre-HPLC to obtain 10.6 mg of desired product.

¹H-NMR (400MHz, DMSO-d6): δ(ppm) : 11.48 (s, 1H), 11.18 (s, 1H), 8.92(s, 1H), 8.60(s, 2H), 7.21-7.09 (m, 2H), 6.79-6.75 (m, 1H), 6.30-6.25 (m, 1H), 4.98-4.91 (m, 1H), 3.32-3.24 (m, 4H), 1.83-1.78 (m, 2H), 1.28-1.27 (m, 6H).

HPLC purity: @214nm 95.2 %, @254nm 98.0%.

LC-MS: *m*/*z* 501 [M+1].

### Example 42

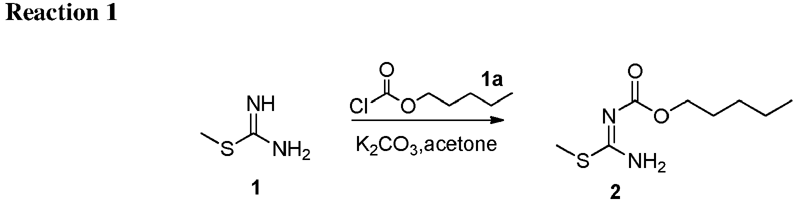

Compound **1** (1.0 g, 3.6 mmol) was dissolved in acetone (16 mL), added with potassium carbonate (993 mg, 7.2 mmol), added dropwise with Compound **1a** (540 mg, 3.6 mmol), stirred at room temperature overnight, quenched with water. The resultant was extracted with ethyl acetate, dried over anhydrous sodium sulfate, and concentrated to obtain 330 mg of a crude product.

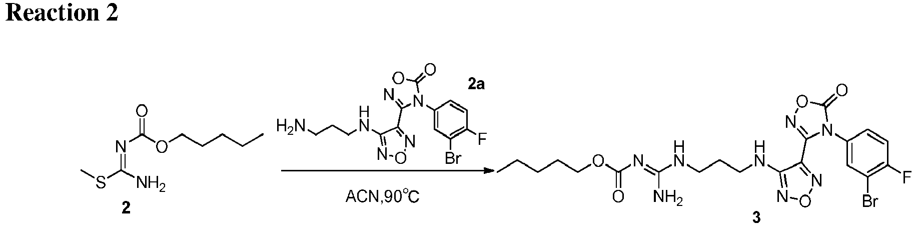

Compound **2a** (100 mg) was dissolved in acetonitrile, added with Compound **2** (50 mg, 0.27 mmol), stirred at 90 °C for 12 h, quenched with water. The resultant was extracted with ethyl acetate, dried over anhydrous sodium sulfate, and concentrated to obtain 90 mg of a crude product.

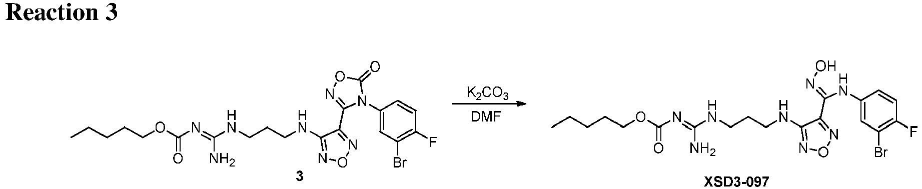

Compound **3** (90 mg) was dissolved in DMF, stirred at room temperature for 10 min, added with potassium carbonate (132 mg, 0.96 mmol), stirred at room temperature for 12 h, quenched with water. The resultant was extracted with ethyl acetate, dried over anhydrous sodium sulfate, and concentrated to obtain 70 mg of a crude product, which was subjected to purification by pre-HPLC to obtain 32.5 mg of desired product.

¹H-NMR (400MHz, DMSO-d6): δ(ppm) : 11.51 (s, 1H), 11.24 (s, 1H), 8.91(s, 1H), 8.55-8.27 (m, 3H), 7.21-7.09 (m, 2H), 6.79-6.75 (m, 1H), 6.29-6.25 (m, 1H), 4.25-4.20 (m, 2H), 3.32-3.24 (m, 4H), 1.80 (s, 2H), 1.57 (s, 2H), 1.28 (s, 4H), 0.88-0.85 (m, 3H).

¹H-NMR (400MHz, CDCl3): δ(ppm) : 7.23-7.20 (m, 1H), 7.10 (s, 1H), 7.04-7.00 (m, 1H), 6.92-6.88 (m, 1H), 5.85 (s, 1H), 4.09-4.06 (m, 2H), 3.58-3.53 (m, 2H), 3.38-3.35 (m, 2H), 1.98-1.97 (m, 2H), 1.70-1.63 (m, 2H), 1.38-1.35 (m, 4H), 0.91-0.88 (m, 3H).

HPLC purity: @214nm 98.1 %, @254nm 97.8%.

LC-MS: *m*/*z* 529[M+1]

Referring to Synthetic Method I and the above examples, the following compounds were synthesized.

| Example No. | Substituent | | | Substance No. | MS |
|---|---|---|---|---|---|
| Example 43 | R₂ is | 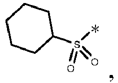 | n is 2, R₁, R₃, R₄ are H. | XSD3-075 | 560.3 |
| Example 44 | R₂ is ethylcarbonyl, n is 1, R₁, R₃, R₄ are H. | | | XSD3-063 | 456.3 |
| Example 45 | R₂ is isopropylcarbonyl, n is 1, R₁, R₃, R₄ are H. | | | XSD3-064 | 470.2 |
| Example 46 | R₂ is methylcarbonyl, n is 2, R₁, R₃, R₄ are H. | | | XSD3-086 | 456.1 |
| Example 47 | R₂ is cyano, n is 1, R₁, R₃, R₄ are H. | | | XSD3-073 | 425.1 |
| Example 48 | R₂ is cyano, n is 2, R₁, R₃, R₄ are H. | | | XSD3-085 | 439.1 |
| Example 49 | R₂ is | 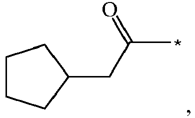 | n is 1, R₁, R₃, R₄ are H. | XSD3-071 | 510.2 |
| Example 50 | R₂ is | 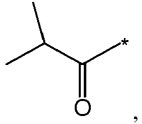 | n is 2, R₁, R₃, R₄ are H. | XSD3-077 | 469.1 |
| Example 51 | R₂ is | 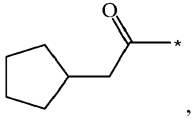 | n is 2, R₁, R₃, R₄ are H. | XSD3-083 | 524.2 |
| Example 52 | R₃ is 1,2-ethanedily n is 1, R₁, R₂, R₄ are H. | | | XSD3-090 | 426.1 |
| Example 53 | R₃ is 1,2-ethanedily n is 2, R₁, R₂, R₄ are H. | | | XSD3-091 | 440.1 |
| Example 54 | R₂ is | 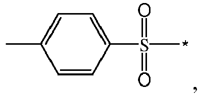 | n is 1, R₁, R₃, R₄ are H. | XSD3-099 | 554.1 |

### Example 55: Determination of the activity for inhibiting indoleamine 2,3-dioxygenase and IC₅₀

The construction of plasmid containing the human indoleamine 2,3-dioxygenase gene, the expression in E. coli, the extraction and the purification were performed according to the method reported by Littlejohn et al. (Takikawa O, Kuroiwa T, Yamazaki F, et al. J. Biol. Chem. 1988, 263, 2041-2048). In a 96-well plate, 50 mM potassium phosphate buffer (pH 6.5), 20 mM ascorbate, 20 µM methylene blue and purified human indoleamine 2,3-dioxygenase protein were mixed, and 200 µM L-tryptophan and inhibitor were added to the mixture. The reaction was carried out at 37 °C for 60 minutes, then the reaction was terminated by adding 30% trichloroacetic acid, and the mixture was incubated at 65 °C for 15 minutes to hydrolyze N-formyl-kynurenine into kynurenine, and centrifuged at 3400g for 5 min to remove the precipitated protein. The supernatant was transferred to a new 96-well plate, a solution of 2% (w/v) p-dimethylaminobenzaldehyde in acetic acid was added, the reaction was performed by incubation at 25 °C for 10 minutes, and the reading was carried out on a spectrophotometer at 480 nm. The control wells were those without indoleamine 2,3-dioxygenase inhibitor or without indoleamine 2,3-dioxygenase, and used as the necessary nonlinear regression parameters to determine IC₅₀ of each compound. The nonlinear regression and the determination of IC₅₀ values were carried out by using GraphPad PRism 4 software. Those compounds with an IC₅₀ of less than 10µM were considered as effective inhibitors in the assay. The compounds of examples of the present invention had excellent activity for inhibiting indoleamine 2,3-dioxygenase.

**Table 1: IC₅₀ values of compounds**

| Structural formula No. | IC₅₀ (nM) |
|---|---|
| 3047 | 37 |
| 3048 | 20 |
| 3049 | 22 |
| 3051 | 23 |
| 3053 | 16 |
| INCB024360 | 102 |

| | |
|---|---|
| Note: INCB024360 was an indoleamine 2,3-dioxygenase inhibitor with a structure of | |

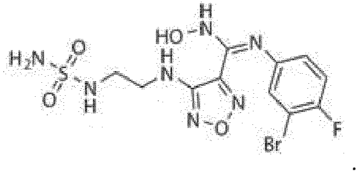

The IC₅₀ values of the following compounds were determined by the method described in Example 55, and the specific results are shown in Table 2:

**Table 2: IC₅₀ values of compounds**

| Structural formula No. | IC₅₀ (nM) |
|---|---|
| XSD3-058 | 57 |
| XSD3-079 | 95 |
| XSD3-093 | 44 |
| XSD3-093-1 | 56 |
| XSD3-093-2 | 61 |
| XSD3-093-3 | 76 |
| XSD3-074 | 63 |
| XSD3-088 | 72 |
| XSD3-089 | 67 |
| XSD3-076 | 102 |
| XSD3-081 | 89 |
| XSD3-069 | 345 |
| XSD3-052 | 343 |
| XSD3-065 | 117.9 |
| XSD3-066 | 131.5 |
| XSD3-067 | 142 |
| XSD3-068 | 539 |
| XSD3-070 | 345 |
| XSD3-072 | 502 |
| XSD3-078 | 145 |
| XSD3-080 | 87 |
| XSD3-082 | 101 |
| XSD3-084 | 160 |
| XSD3-092 | 399 |

### Example 56: In vivo antitumor activity test of indoleamine 2,3-dioxygenase inhibitors

### 1. Animal grouping and test method

LLC cells in logarithmic growth phase were taken, the cell viability thereof was detected by trypan blue staining method, the viable cell concentration was adjusted to 1×10⁷ cells/ml, and subcutaneously injected at a dose of 0.2 ml/mouse into homologous C57BL6 mice. Once tumors were established, the mice were randomly divided into model group, cyclophosphamide (CTX) group, compound INCB024360 group, compound 3047 group according to their tumor weights and body weights, there were 10 mice in each group, wherein the CTX group was intraperitoneally injected with a dose of 150 mg·kg⁻¹, the compound INCB024360 group and the compound 3047 group were subjected to intragastric administration, the model group was given the same volume of normal saline at the same time, and the administration frequency for each group was once per day. The test was terminated after 21 days of administration.

24 Hours after the last administration, the animals were weighed and sacrificed, the tumors were taken and weighted, and average tumor inhibition rate (I) was calculated according to the following formula: I = (1 - average tumor weight of the drug-administered group / average tumor weight of the model group) × 100%

### 2. Data statistics and processing method

The experimental data were analyzed by spss16.0, and one-way ANOVA, and difference with p<0.05 was statistically significant.

### 3. Test results and discussion

**Table 3: Inhibition results of compounds on LLC tumors in mice**

| Group | Number of animals (mouse) | Tumor weight (g) | Tumor inhibition rate (%) |
|---|---|---|---|
| Model group | 10 | 1.514±0.312 | / |
| CTX group | 10 | 0.701±0.314^{##} | 53.7 |
| INCB024360 group | 10 | 0.653±0.178^{##} | 56.9 |
| Compound 3047 group | 10 | 0.370±0.209^{##} | 75.6 |

In comparison with the model group, ^{##}P<0.01;
In comparison with the CTX group and the INCB024360 group, 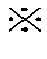 P<0.05;

As can be seen from Table 3, the tumor weight of each drug-administered group was significantly different from that of the model group (P<0.01); the compound 3047 group was significantly different from the cyclophosphamide group and the INCB024360 group (P<0.05). This result indicates that the compound of the present invention is superior to the existing chemotherapeutic drug cyclophosphamide and the compound INCB024360 in therapeutic effect of tumors.

**Table 4: Effects of compounds on body weight of mice**

| Group | Average body weight before test (g) | Average body weight after test (g) |
|---|---|---|
| Model group | 21±4.2 | 28±3.2 |
| CTX group | 22±3.1 | 19±3.3 |
| Compound 3047 group | 22±3.4 | 29±5.2^{##} |

| | | |
|---|---|---|
| In comparison with the cyclophosphamide group, p<0.01 | | |

As can be seen from Table 4, the compound 3047 group showed no significant difference in body weight in comparison with the model group, but showed a significant difference in comparison with the CTX group. This result indicates that the compound of the present invention increased the body weight in mice while controlling tumor growth, that is, the side effect was reduced, and the life quality of mice was significantly improved. Clinically, it means that the life quality of patients can be improved and the patient compliance with medications and medicament effectiveness can be significantly enhanced.

In addition, we also tested mouse colon cancer cell Colon26, mouse liver cancer cell Hepa1-6, mouse breast cancer cell 4T1 and other cell lines, and the results showed that the compounds of the present invention had significant inhibitory effects on these tumors.

The *in vivo* antitumor activity of the following compounds was determined by the method of Example 56, and the specific results are shown in the following table:

**Table 5: Inhibition results of compound on LLC tumors in mice**

| Group | Animal number (mouse) | Tumor weight (g) | Tumor inhibition rate (%) |
|---|---|---|---|
| Model group | 10 | 1.445±0.217 | / |
| Compound XSD3-058 group | 10 | 0.400±0.112^{##} | 72.3 |
| Compound XSD3-079 group | 10 | 0.384±0.092^{##} | 73.4 |

In comparison with the model group, ^{##}P<0.01.

As can be seen from Table 5, the tumor weight of each drug-administered group was significantly different from that of the model group (P<0.01). This result indicates that the compound of the present invention has significant therapeutic effect on tumor.

The effect of each compound on the body weight of mice was examined. It was found that there was no significant difference in body weight between the compound XSD3-058 group and the compound XSD3-079 group compared with the model group. This result indicates that the compounds of the present invention can increase the body weight in mice while controlling tumor growth, reduce the side effects of the drug and significantly improve the quality of life of the mouse. Clinically, the compounds of the present invention can improve the quality of life of patients and significantly enhance the patient compliance with medications and medicament effectiveness.

### Example 57: Morris water maze detection of behavioral changes in mice with Alzheimer

### 1. Animal grouping and test methods

In the present invention, 9-month-old mice were selected to establish AD models according to the method of Richardson et al. by single injection of aggregated Aβ1-42 in the bilateral hippocampal CA3 region of rats, and then divided into a model group, a compound INCB024360 group, a compound 3047 group, 10 mice per group, five male and five female. Mouse behavioral analysis was performed using a Morris water maze (Ethovision XT monitoring and analysis software, Morris water maze system, from Noldus, Netherlands). The water maze test process was divided into two parts, hidden platform acquisition test for consecutive 5 days, and spatial probe test on the sixth day, the mice were administered according to the test groups and the design doses before each test. The mice were trained 4 times a day, each time the mice were dived in different areas, the water maze was divided into areas 1, 2, 3 and 4 according to the south, east, north and west, and the platform was the 5th area located in the 4th area. Each swimming time was 60s, the interval for each training was about 1h, and when a mouse did not find the platform, the latent period was calculated as 60s. The hidden platform acquisition test was used to detect the learning ability of mice, while the spatial probe test was used to detect the spatial memory ability of mice.

### 2. Data statistics and processing method

Using the statistical analysis software SPSS16.0, the escape latent period of the hidden platform acquisition test was analyzed by variance of multiple measures; and the swimming time in each quadrant and the number of crossing target in the spatial probe test were analyzed by oneway analysis of variance. The data were expressed as mean ± standard deviation, and the significant level of difference was set as bilateral P = 0.05.

### 3. Test results and discussion

**Table 6: Latent period (s) of each group of animals for searching platform in hidden platform test**

| Group | 1^{st} Day | 2^{nd} Day | 3^{th} Day | 4^{th} Day | 5^{th} Day |
|---|---|---|---|---|---|
| Model group | 54.0±5.9 | 52.8±1.9 | 43.5±6.7 | 39.8±6.9 | 38.4±5.6 |
| INCB024360 group | 53.2±2.3 | 51.4±4.8 | 38.6±5.5 | 28.7±3.9^{#} | 19.9±6.7^{##} |
| Compound 3047 group | 52.3±5.2 | 44.0±4.4^{#} | 22.1±4.5^{##} 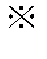 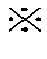 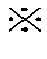 | 10.5±5.7^{##} 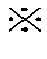 | 5.4±6.0^{##} |

In comparison with the model group, ^{#}P<0.05, ^{# #}P<0.01,

In comparison with the INCB024360 group, 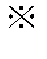 P<0.05, 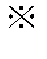 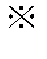 P<0.01.

**Table 7: Time and number of stays of each group of animals in platform areas**

| Group | Time of stay (s) 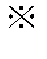 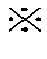 | | Number of stays (times) 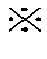 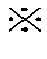 | |
|---|---|---|---|---|
| | 4^{th} Area | 5^{th} Area | 4^{th} Area | 5^{th} Area |
| Model group | 14.9±3.8 | 1.9±0.8 | 4.8±0.7 | 1.0±0.3 |
| INCB024360 group | 16.2±2.4 | 3.9±0.7^{#} | 8.4±1.0^{#} | 2.1±0.4^{# #} |
| Compound 3047 group | 25.9±5.2 ^{# #} | 5.9±0.7 ^{# #} | 13.0±1.5^{# #} | 3.8±0.6^{# #} |

In comparison with the model group, ^{#}P<0.05, ^{# #}P<0.01,
In comparison with the INCB024360 group, 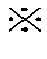 P<0.05.

As can be seen from Tables 6 and 7, Compound 3047 can significantly improve learning and memory impairment in animals, significantly improve learning ability and spatial memory ability, and is superior to compound INCB024360, indicating that the compound of the present invention has great development value in the treatment of Alzheimer's syndrome.

The effect of the following compounds on behavior of mice with Alzheimer were tested by the method of Example 57. The specific results are shown in the following table:

**Table 8: Latent period (s) of each group of animals for searching platform in hidden platform test**

| Group | 1^{st} Day | 2^{nd} Day | 3^{th} Day | 4^{th} Day | 5^{th} Day |
|---|---|---|---|---|---|
| Model group | 59.7±4.6 | 59.8±1.8 | 50. 6±5.5 | 42.8±4.9 | 38.7±5.2 |
| Compound XSD3-058 group | 45.3±3.7^{#} | 41.3±3.2^{#} | 25.4±4.6^{# #} | 15.5±4.9^{# #} | 7.3±6.1^{# #} |
| Compound XSD3-079 group | 40.5±5.5^{#} | 39.3±44^{#} | 20.3±4.2^{# #} | 17.7±5.2^{# #} | 9.9±5.2^{# #} |

In comparison with the model group, ^{#}P<0.05, ^{# #}P<0.01.

**Table 9: Time and number of stays of each group of animals in platform areas**

| Group | Time of stay (s) | | Number of stays (times) | |
|---|---|---|---|---|
| | 4^{th} Area | 5^{th} Area | 4^{th} Area | 5^{th} Area |
| Model group | 15.8±3.1 | 2.0±0.9 | 4.1±0.9 | 1.2±0.4 |
| Compound XSD3-058 group | 25.2±4.2^{# #} | 5.9±0.6^{# #} | 13.3±1.8^{# #} | 3.5±0.9^{# #} |
| Compound XSD3-079 group | 26.7±3.3^{# #} | 5.8±0.74^{# #} | 14.4±1.3^{# #} | 3.9±0.5^{# #} |

In comparison with the model group, ^{# #}P<0.01.

As can be seen from Tables 8 and 9, the compounds of the present invention can significantly improve learning and memory impairment in animals, and significantly improve learning ability and spatial memory ability, and the results indicate that the compounds of the present invention have great development value in the treatment of Alzheimer's syndrome.

### Example 58: DC-stimulated T cell proliferative response after drug treatment

Dendritic cell (DC) is the most powerful antigen-presenting cell (APC), which can effectively activate naive T cells for proliferation, which is the most important difference between DC and other APCs. DC is the initiator of the immune response. DC has become one of the hotspots in immunology research today due to its key role in CD4⁺, CD8⁺ T cell immune response. Currently, the research of DC is mainly focused on prevention and treatment effect on tumor diseases, autoimmune diseases, transplant rejection, and anti-infection.

### 1. Isolation and culture of human peripheral blood dendritic cells

The human peripheral blood leukocyte layer was taken and diluted with same volume of 0.01 mol/L PBS. PBMC was routinely isolated with a lymphocyte separation medium, and the cell concentration was adjusted to 3×10⁶ ml⁻¹ in a complete RPMI1640 medium; the cells were added to a 6-well plate, 3 ml per well, and cultured in a 5% CO₂, 37°C incubator for 2 hours, washed with PBS for 3 times to remove non-adherent cells, then added with a culture medium containing IL-4 (100 U/ml), GM-CSF (150 ng/ml) and TNF-α (500 U/ml), and then routinely cultured, the medium was changed for half every other day. After 8 days of culture, the cells were used for identification and experiment.

### 2. Preparation of T cells

The human PBMC layer was separated by the method in the step 1. The macrophages were removed by the adherence method, the B cells were removed by the nylon wool fiber syringe method, and the obtained T cells were adjusted to a cell concentration of 1×10⁶ cells/ml.

### 3. Preparation of DC

The mature DCs with a purity of 99% were centrifuged, added with RPMI1640 to adjust the cell concentration to 1×10⁵, 4×10⁴, 2×10⁴/ml, and added to a 96-well plate, two wells for each concentration, 100 µl/well. Compound INCB024360 and Compound 3047 were separately added, and cultured for 2 days.

### 4. T cell proliferation assay (MLR)

T cells were added to DCs of each of the above drug-added groups, 100 µl/well, cultured in a 5% CO₂, 37 °C incubator for 72 hours; for each well, 100 µl of the culture solution was gently sucked out 6 hours before the end of the culture, and 10 µl of MTT (5 mg/ml) was added, further cultured in incubator for 6 hours, then 100µl of 0.01mol/L HCl-10% SDS was added, stood at 37 °C overnight, and the absorbance value (A570nm) was determined by a microplate to show the T cell proliferation level.

### 5. Experimental results and analysis

**Table 10: Effect of Compound 3047 on DC-stimulated T cell proliferation**

| Group | T:DC (absorbance value) | | |
|---|---|---|---|
| | 10:1 | 25:1 | 50:1 |
| Control group | 0.801±0.003 | 0.782±0.002 | 0.755±0.005 |
| INCB024360 group | 0.830±0.002^{#} | 0.804±0.004^{#} | 0.799±0.003^{#} |
| Compound 3047 group | 0.864±0.006^{# #} 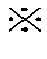 | 0.834±0.004^{# #} 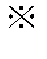 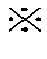 | 0.825±0.007^{# #} |

In comparison with the control group, P<0.05, ^{# #}P<0.01,
In comparison with the INCB024360 group, 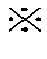 P<0.05.

As can be seen from Table 10, compared with the control group, the numbers of T cells in the compound 3047 group and the INCB024360 group were significantly increased, showing significant differences (^{#}P<0.05, ^{##}P < 0.01); and compared with the compound INCB024360 group, the compound 3047 group had a more significant effect on the proliferation of T cells, showing significant difference ( 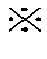 P<0.05). This indicates that the compound of the present invention has a significant effect on promoting DC-stimulated T cell proliferation, and the effect is significantly better than that of the compound INCB024360, and thereby can be used for the treatment of tumor diseases, autoimmune diseases, transplant rejection and infectious diseases.

The DC-stimulated T cell proliferative responses after treatment with the following compounds were determined by the method of Example 58, and the specific results are shown in the following table:

**Table 11: Effect of each compound on DC-stimulated T cell proliferation**

| Group | T:DC (absorbance value) | | |
|---|---|---|---|
| | 10:1 | 25:1 | 50:1 |
| Control group | 0.810±0.004 | 0.793±0.004 | 0.760±0.002 |
| Compound XSD3-058 group | 0.880±0.003^{# #} | 0.849±0.002^{# #} | 0.831±0.002^{# #} |
| Compound XSD3-079 group | 0.876±0.004^{# #} | 0.850±0.003^{# #} | 0.837±0.004^{# #} |

In comparison with the control group, ^{# #}P<0.01.

As can be seen from Table 11, compared with the control group, the number of T cells in each compound group was significantly increased, showing a significant difference (^{##}P < 0.01), indicating that the compound of the present invention can significantly promote the DC-stimulated T cell proliferation, and thereby can be used for the treatment of IDO-related diseases such as tumor diseases, autoimmune diseases, transplant rejection and infectious diseases.

## Claims

1. A compound represented by Formula I or a pharmaceutically acceptable salt thereof, 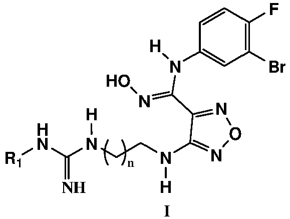
wherein, n is an integer selected from 0 to 6,
R₁ is selected from the group consisting of H, NH₂, CN, methyl, ethyl, propyl, isopropyl, butyl, cyclopropyl, cyclobutyl, cyclopentyl, cyclohexyl, methoxycarbonyl, tert-butoxycarbonyl, fluorenylmethoxycarbonyl, allyloxycarbonyl, benzyl, and 5-azaindene methylcarbonyl.

2. The compound or a pharmaceutically acceptable salt thereof according to claim 1, **characterized in that** n is selected from 0, 1, 2, 3 and 4.

3. The compound or a pharmaceutically acceptable salt thereof according to claim 1, **characterized in that** when R₁ in Formula I is H, the compound is represented by Formula

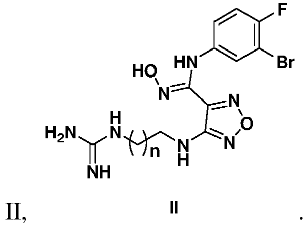

4. The compound or a pharmaceutically acceptable salt thereof according to claim 3, **characterized in that** n is 0, 1, 2 or 3.

5. The compound or a pharmaceutically acceptable salt thereof according to claim 1, **characterized in that** the compound is selected from the group consisting of:

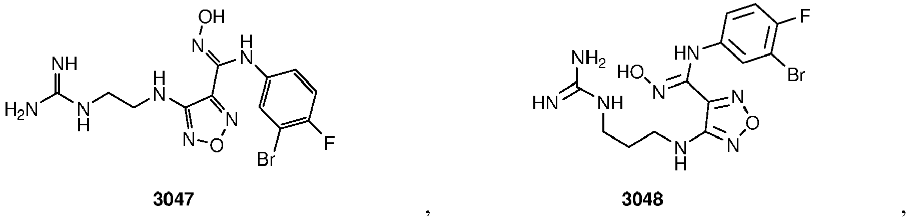

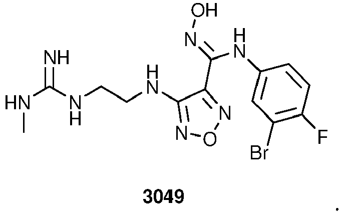

6. A compound or a pharmaceutically acceptable salt thereof, **characterized in that** the compound is selected from the group consisting of:

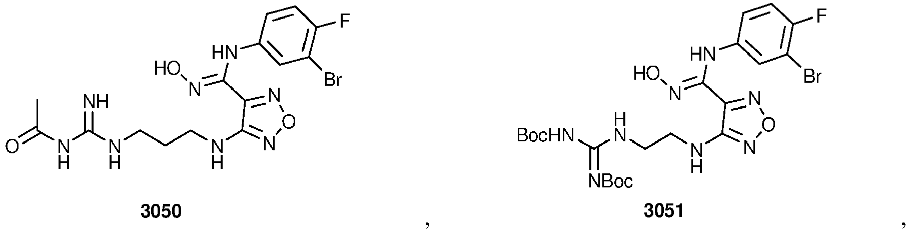

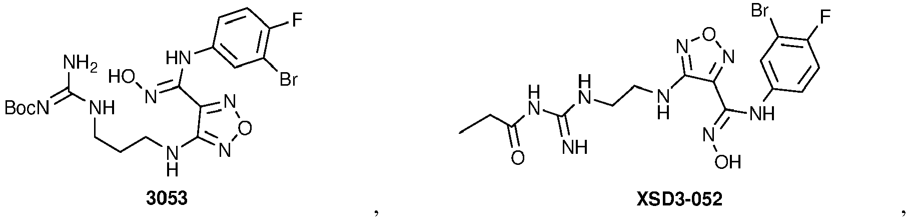

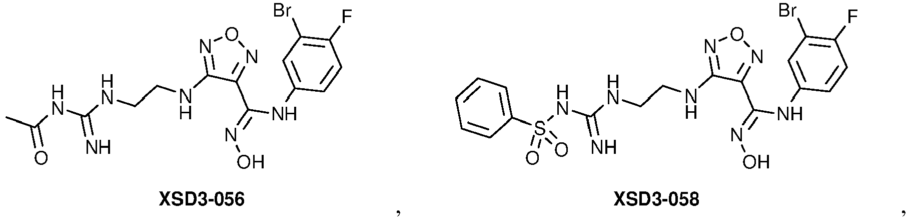

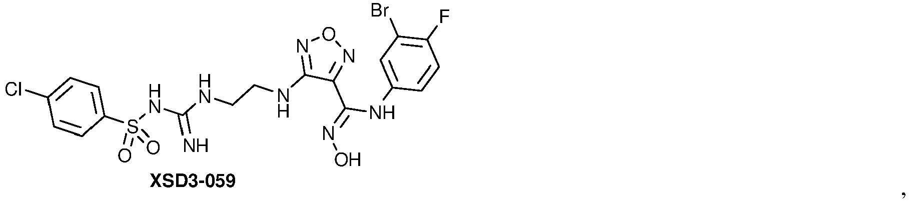

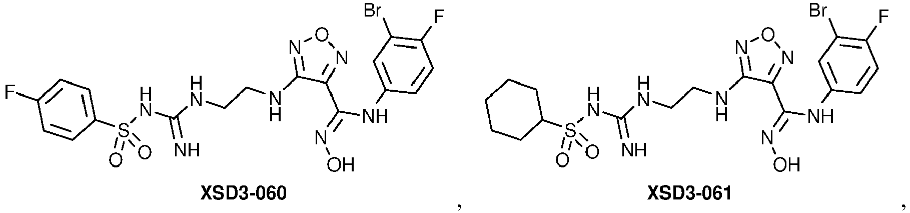

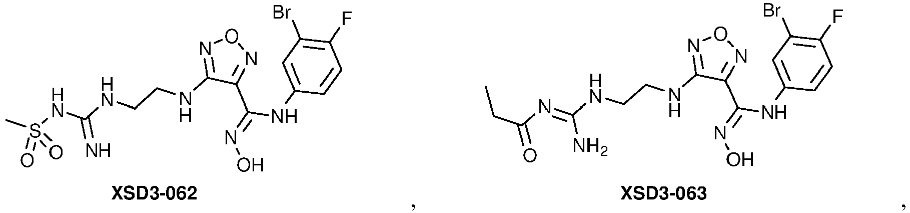

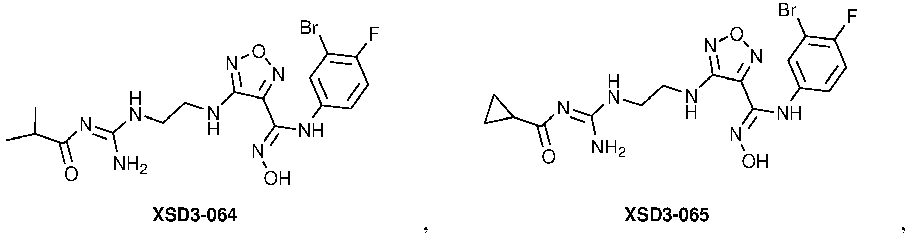

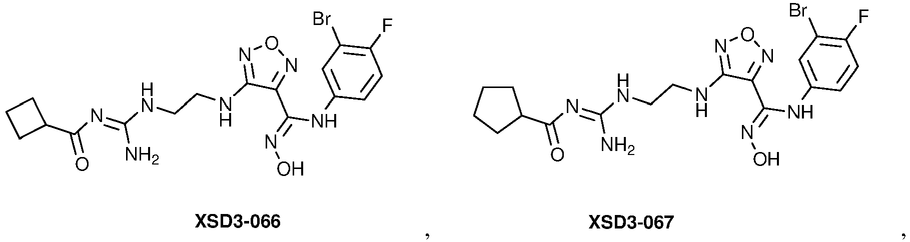

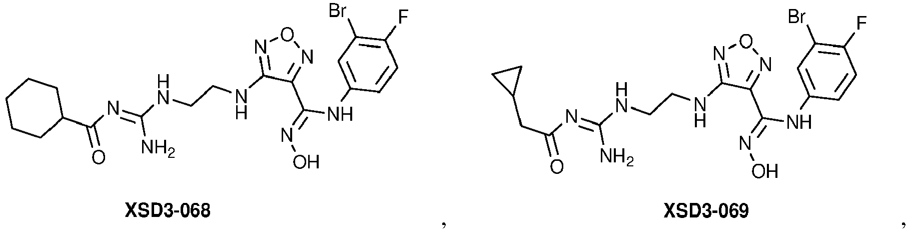

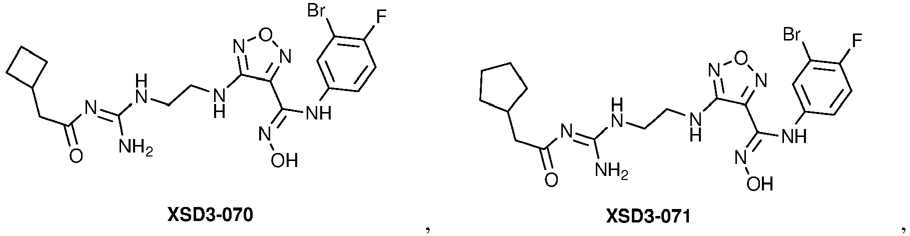

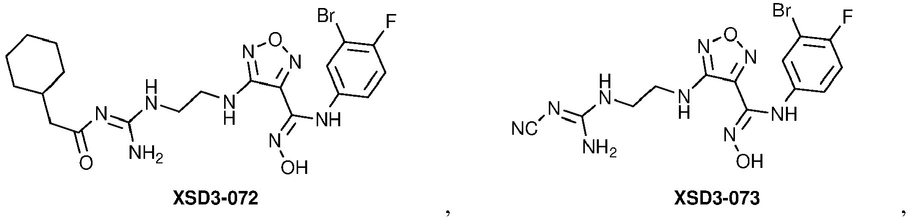

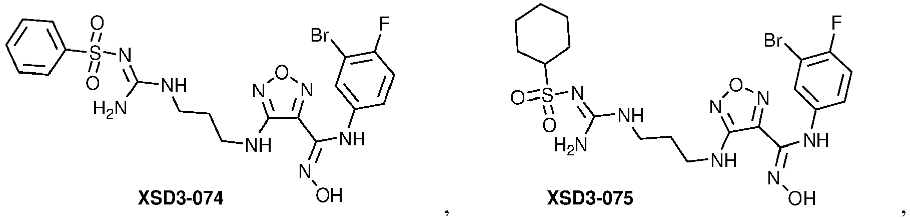

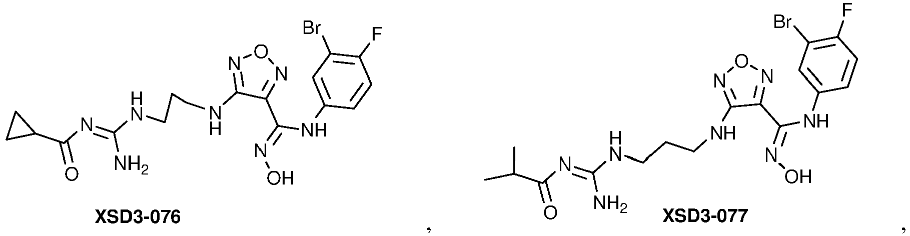

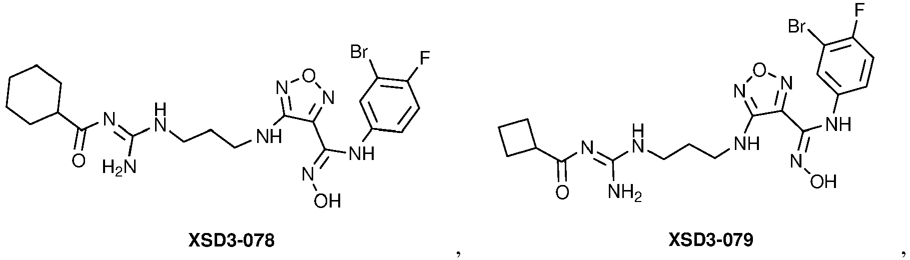

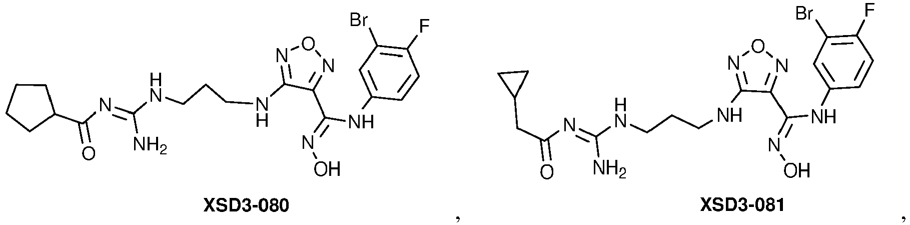

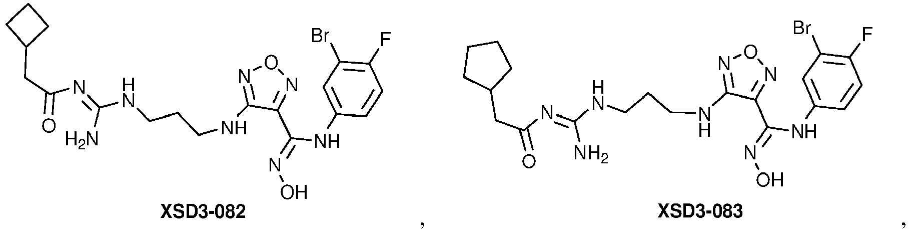

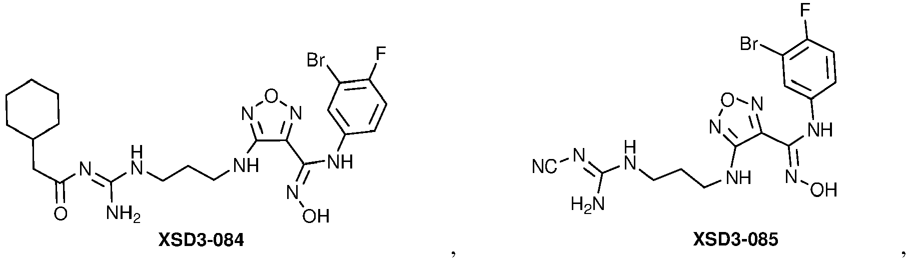

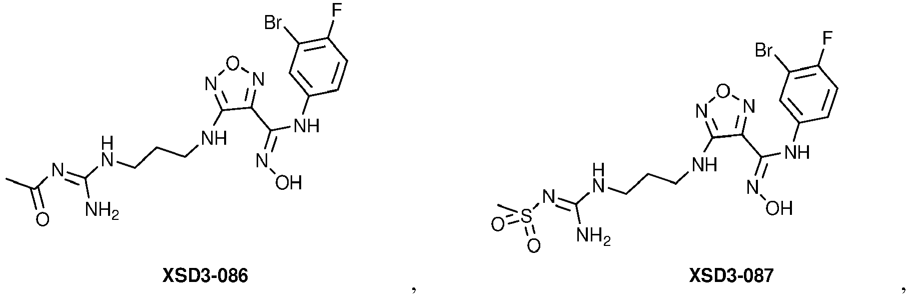

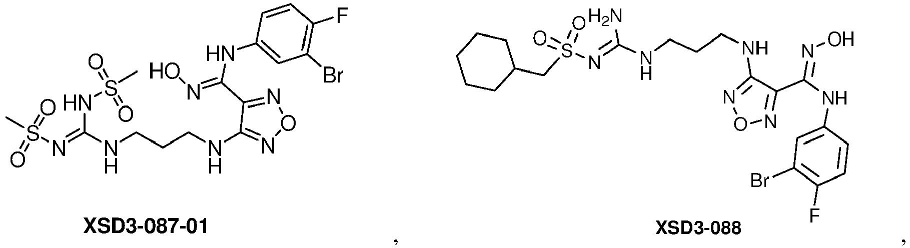

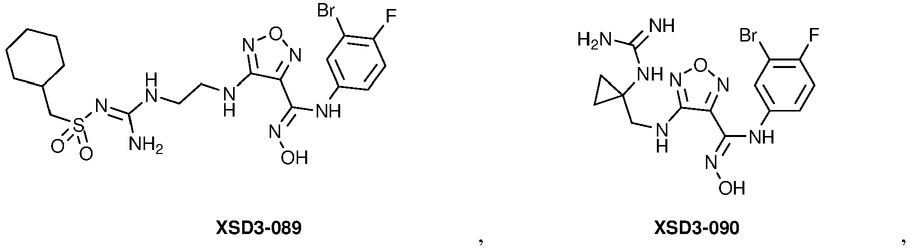

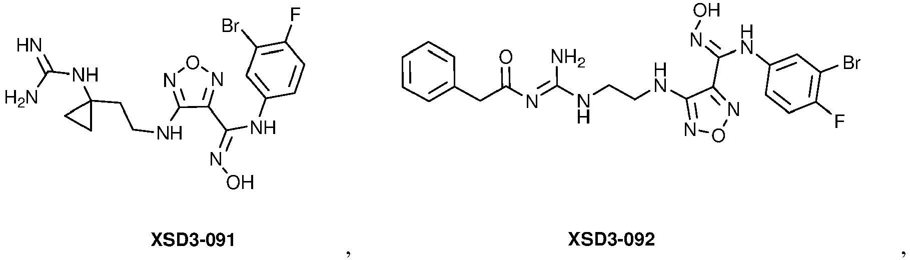

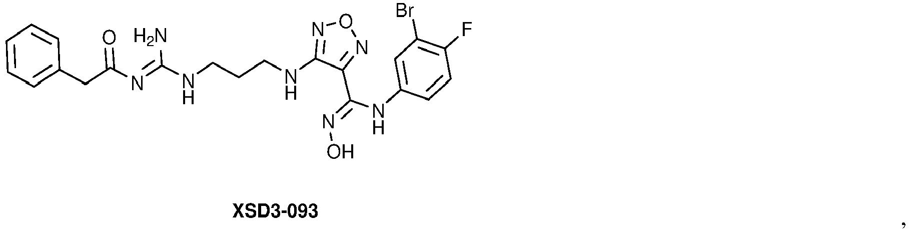

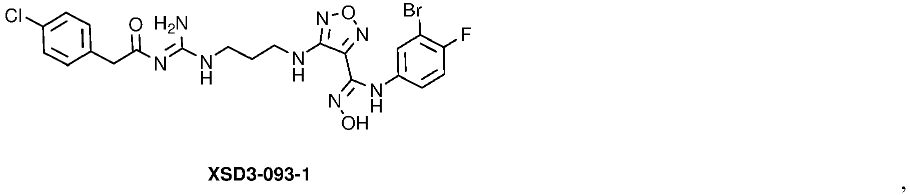

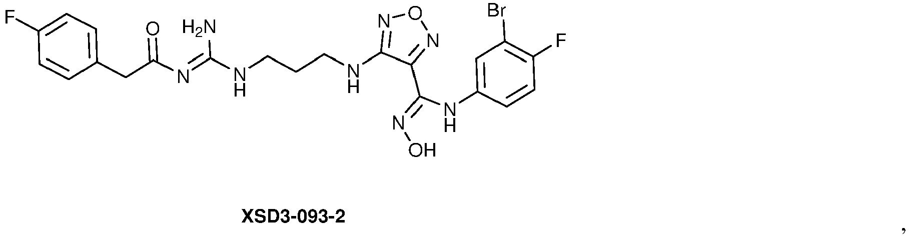

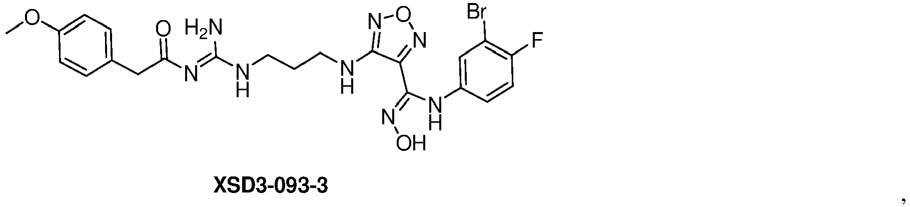

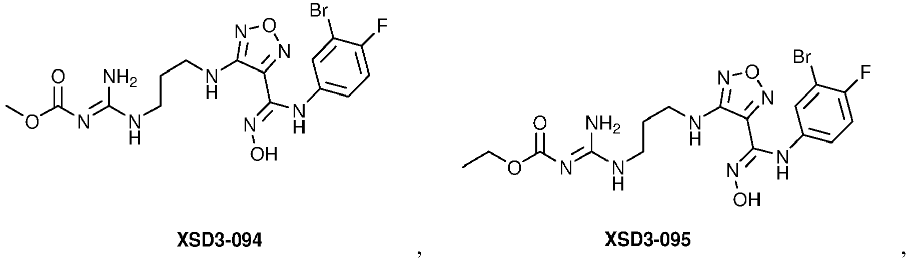

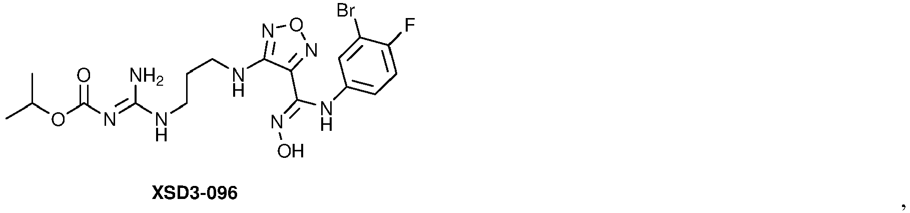

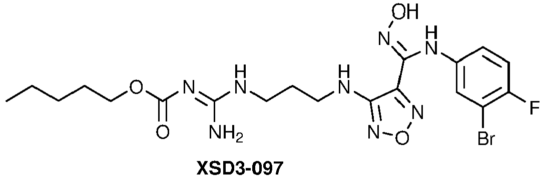

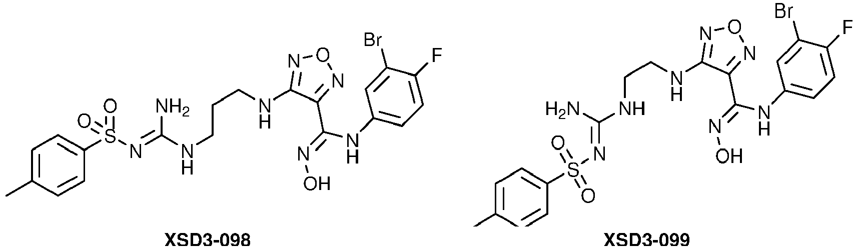

7. A pharmaceutical composition, comprising the compound or a pharmaceutically acceptable salt thereof according to any one of claims 1 to 6, and one or more pharmaceutically acceptable pharmaceutic adjuvants.

8. The compound or a pharmaceutically acceptable salt thereof according to any one of claims 1 to 6 or a pharmaceutical composition thereof, for use in treating of a cancer, an infectious disease, a neurodegenerative disease, a depression, an anxiety or an age-related cataract.

9. The compound for use according to claim 8, wherein the cancer is selected from the group consisting of lung cancer, liver cancer, colon cancer, pancreatic cancer, breast cancer, prostate cancer, brain cancer, ovarian cancer, cervical cancer, testicular cancer, renal cancer, head and neck cancer, lymphoma, melanoma or leukemia.

10. The compound for use according to claim 8, wherein the neurodegenerative disease is Alzheimer's disease.

11. The compound for use according to claim 8, wherein the infectious disease is an infection caused by a bacterium, a fungus, a virus or a parasite.

12. A method for preparing a compound represented by Formula II or a pharmaceutically acceptable salt thereof according to claim 3, 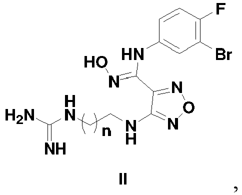 wherein n is 0, 1, 2, or 3, **characterized in that** it comprises the following steps:
1) reacting a compound represented by formula 2 with compound 3a' to obtain a compound represented by formula IIa: 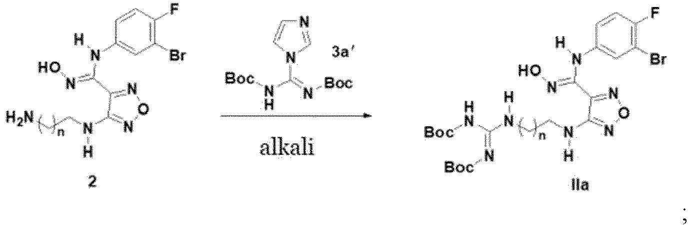
2) performing deprotection of the compound represented by formula Ila under an acidic condition, then performing reaction under an alkaline condition to obtain the compound represented by formula II,

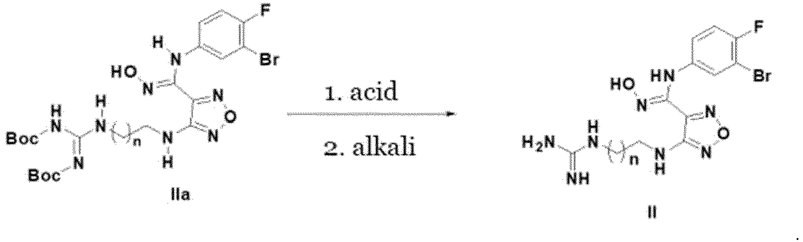

## Patentansprüche

1. Verbindung, dargestellt durch eine Formel I, oder pharmazeutisch annehmbares Salz davon, 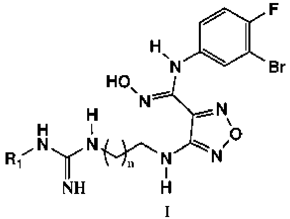
wobei n eine ganze Zahl ausgewählt aus 0 bis 6 ist,
R₁ ausgewählt ist aus der Gruppe bestehend aus H, NH₂, CN, Methyl, Ethyl, Propyl, Isopropyl, Butyl, Cyclopropyl, Cyclobutyl, Cyclopentyl, Cyclohexyl, Methoxycarbonyl, tert-Butoxycarbonyl, Fluorenylmethoxycarbonyl, Allyloxycarbonyl, Benzyl und 5-Azaindenmethylcarbonyl.

2. Verbindung oder pharmazeutisch annehmbares Salz davon nach Anspruch 1, **gekennzeichnet dadurch, dass** n aus 0, 1, 2, 3 und 4 ausgewählt ist.

3. Verbindung oder pharmazeutisch annehmbares Salz davon nach Anspruch 1, **dadurch gekennzeichnet, dass**, wenn R₁ in Formel I H ist, die Verbindung durch die Formel II dargestellt wird.

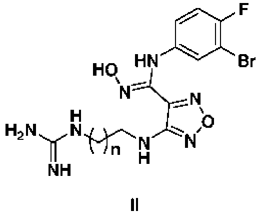

4. Verbindung oder pharmazeutisch akzeptables Salz davon nach Anspruch 3, **dadurch gekennzeichnet, dass** n 0, 1, 2 oder 3 ist.

5. Verbindung oder pharmazeutisch annehmbares Salz davon nach Anspruch 1, **gekennzeichnet dadurch, dass** die Verbindung ausgewählt ist aus der Gruppe bestehend aus:

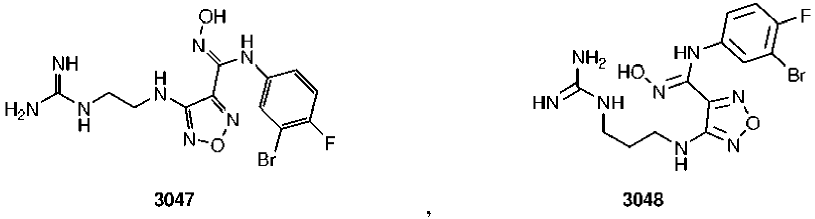

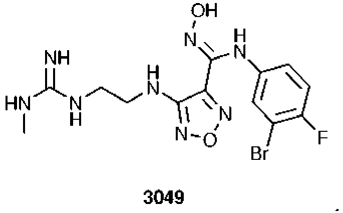

6. Verbindung oder pharmazeutisch annehmbares Salz davon, **dadurch gekennzeichnet, dass** die Verbindung ausgewählt ist aus der Gruppe bestehend aus:

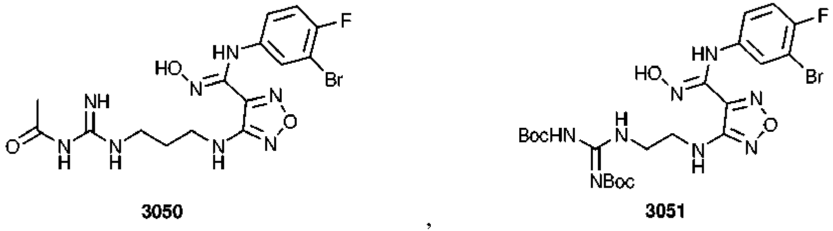

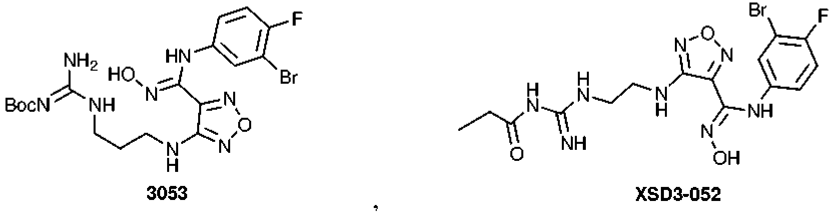

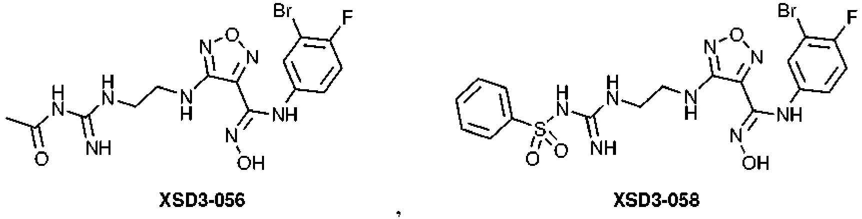

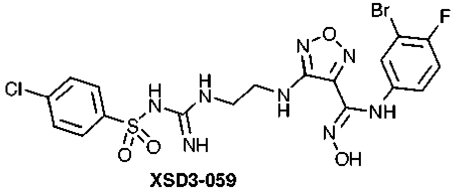

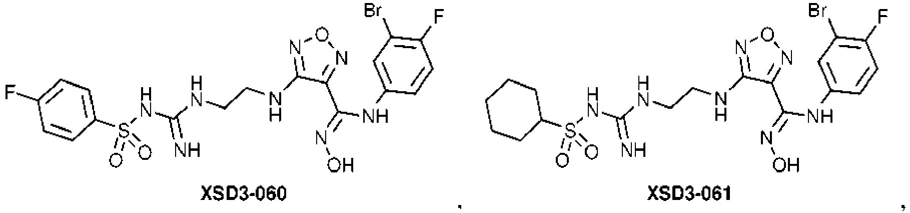

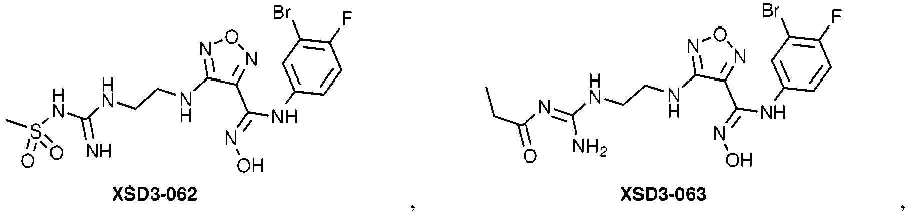

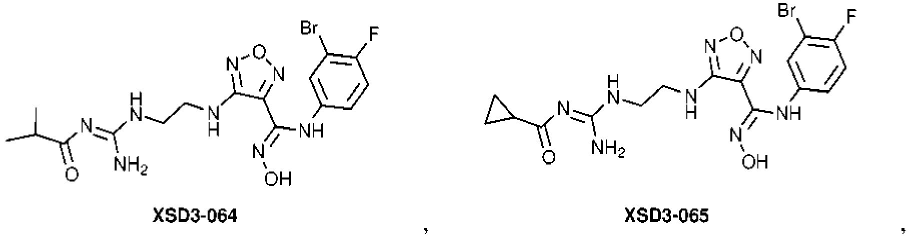

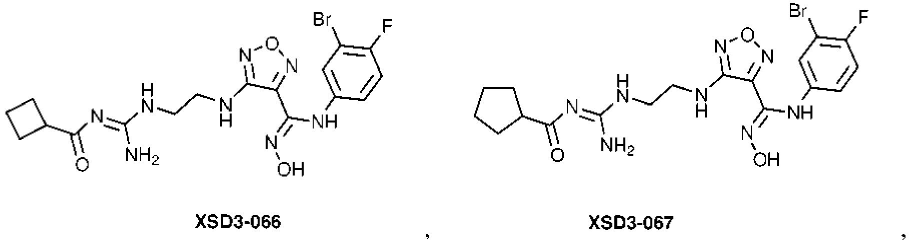

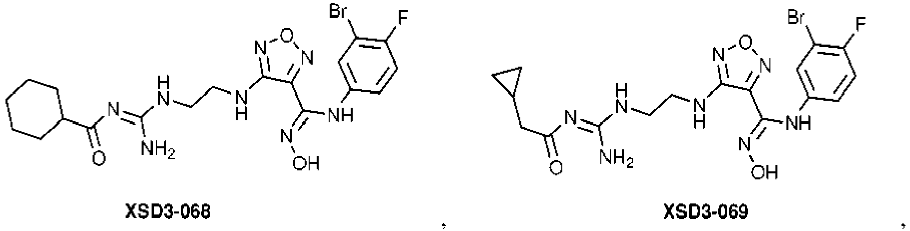

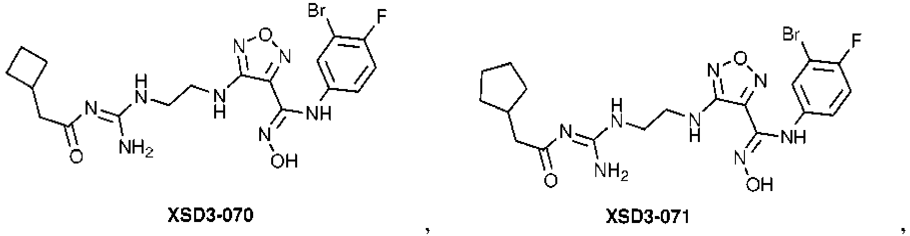

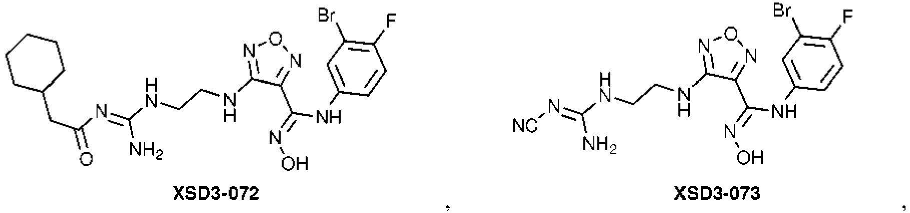

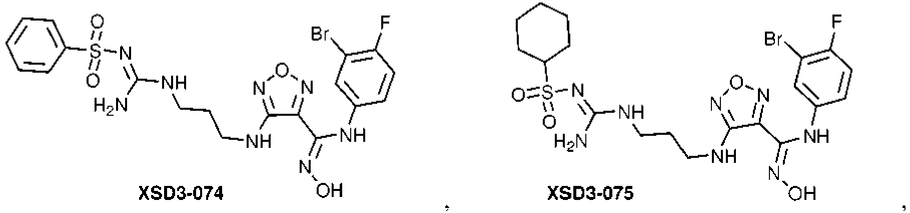

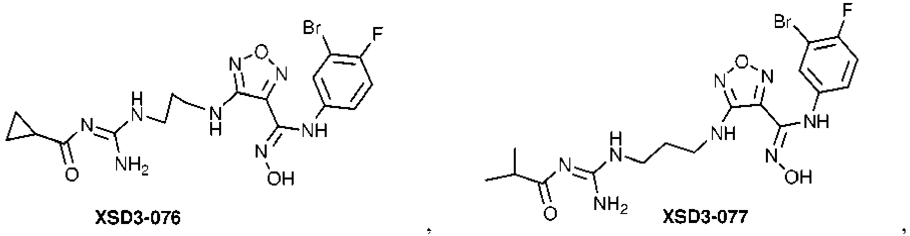

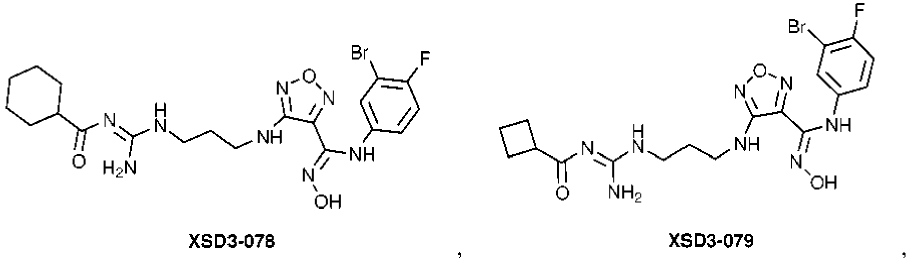

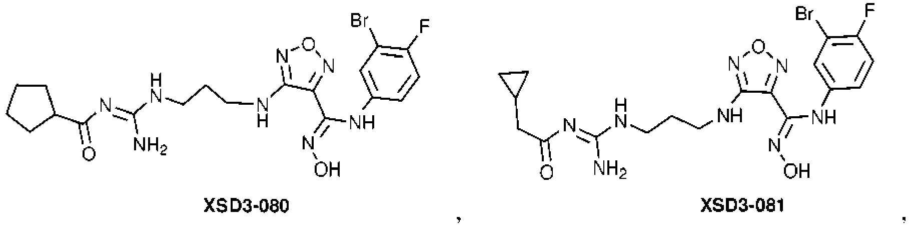

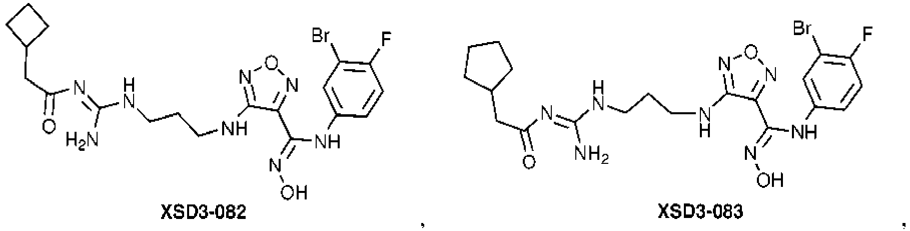

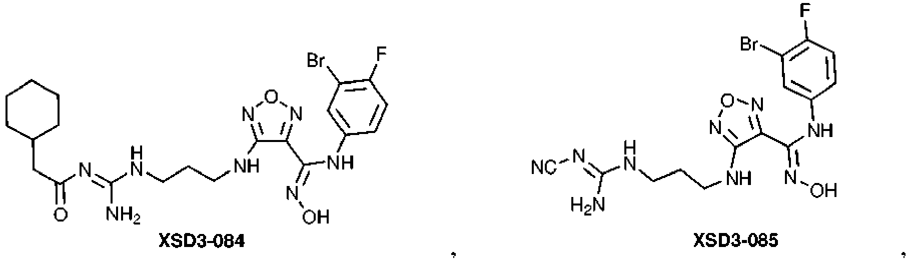

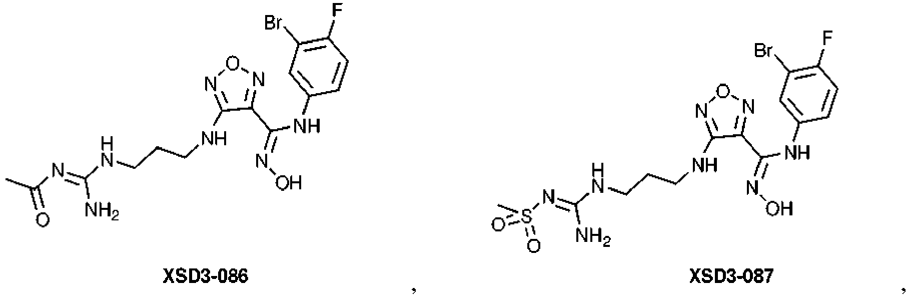

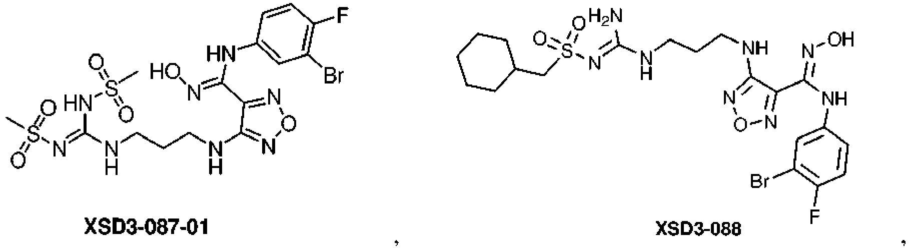

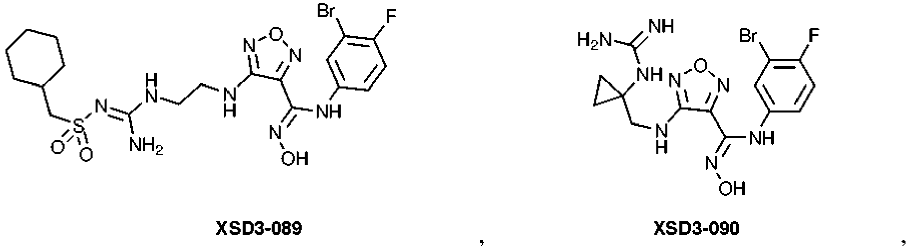

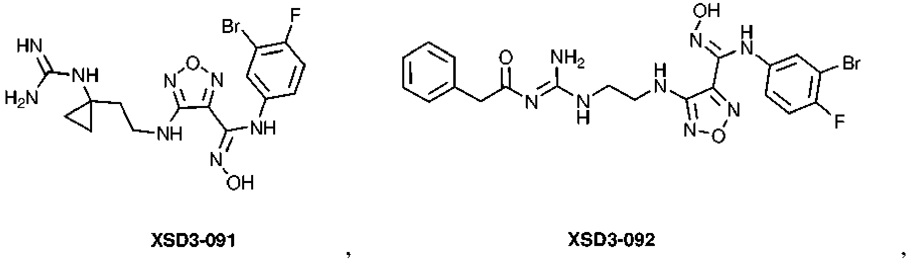

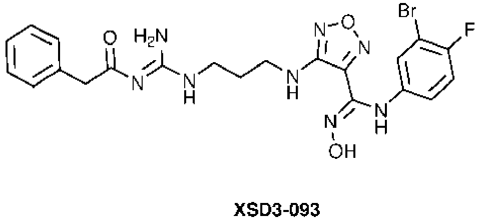

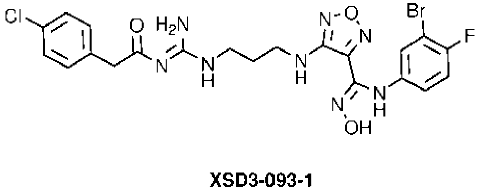

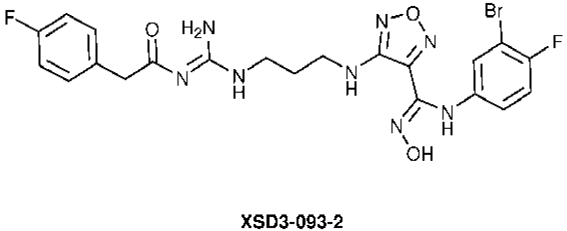

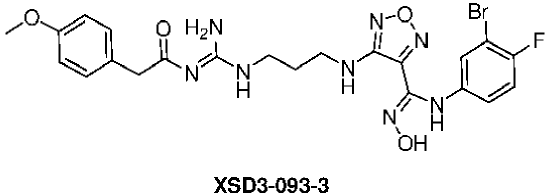

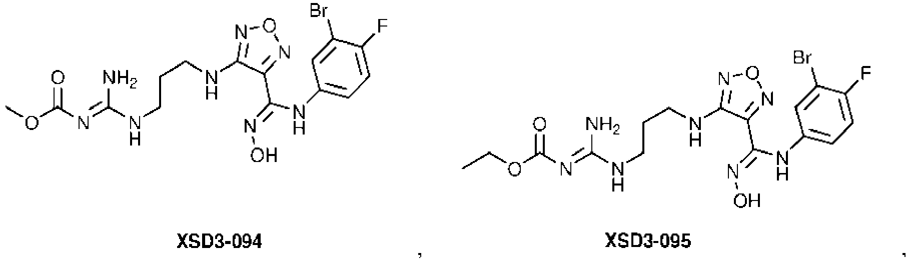

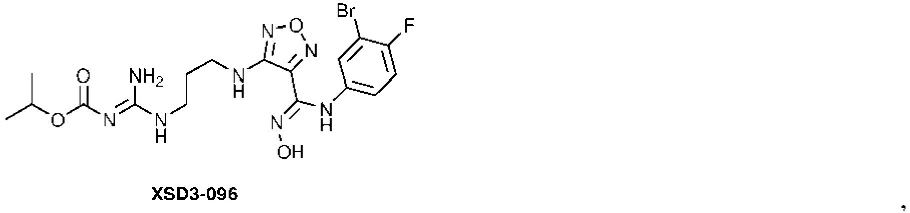

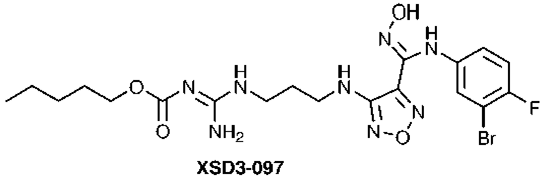

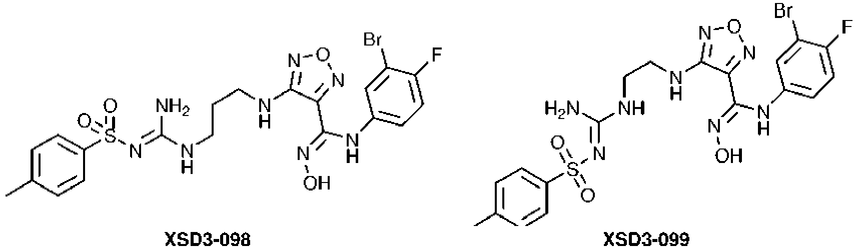

7. Pharmazeutische Zusammensetzung, umfassend die Verbindung oder ein pharmazeutisch annehmbares Salz davon nach einem der Anspruüche 1 bis 6 und mindestens einem pharmazeutisch annehmbaren Hilfsstoff.

8. Verbindung oder pharmazeutisch annehmbares Salz davon nach einem der Anspruche 1 bis 6 oder pharmazeutische Zusammensetzung davon zum Verqenden bei der Behandlung von Krebs, einer Infektionskrankhelt, einer neurodegenerativen Karankheit, einer Depression, einer Angststoörung oder eines alternsbedingten Katarakts.

9. Verbindung zum Verwenden nach Anspruch 8, wobei der Krebs ausgewählt ist aus des Gruppe bestehend aus Lungenkrebs, Dickdarmkrebs, Bauchspeicheldrünsenkrebs, Brustkrebs, Prostatakrebs, Hirnkrebs, Eierstockkrebs, Gebamutterhalskrebs, Hodenkrebs, Nierenkrebs, Kopf- und Halskrebs, Lymphom, Melanom oder Leukaämie.

10. Verbindung zum Verwenden nach Anspruch 8, wobei die neurodegenerative Erkrankung die Alzeimer-Krankheit ist.

11. Verbindung zum Verwenden nach Anspruch 8, wobei die Infektionskrankheit eine durch ein Bakterium, einen Pilz, ein Virus oder einen Parasiten verusachte infektion ist.

12. Verbindung zum Herstellen einer Verbindung, dargestellt durch Formel II, oder eines pharmazeutisch annehmbaren Salzes davon nach Anspruch 3, 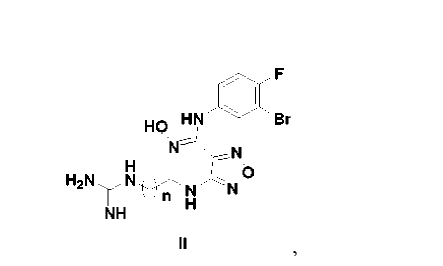 wobei n 0, 1, 2 oder 3 ist, **dadurch gekennzeichnet, dass** es die folgenden Schritte umfasst:
1) Reagieren einer Verbindung, dargestellt durch Formel 2, mit der Verbindung **3a'**, um eine Verbindung, dargetellt durch Formel **IIa**, zu erhalten: 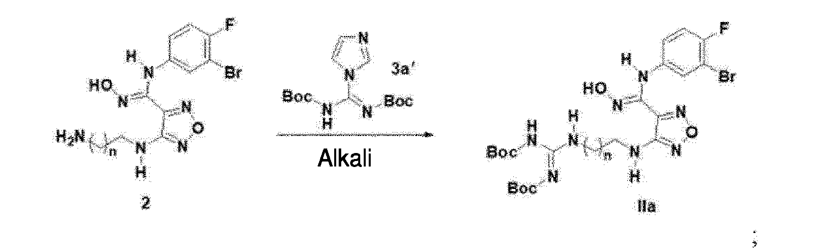
2) Durchführen einer Entschützung der Verbindung, dargetellt durch Formel **IIa**, unter sauren Bedinungen, und dann Durchführen einer Reaktion unter alkalischen Bedingungen, um die Verbindung zu erhalten, die durch Formel II dargestellt ist,

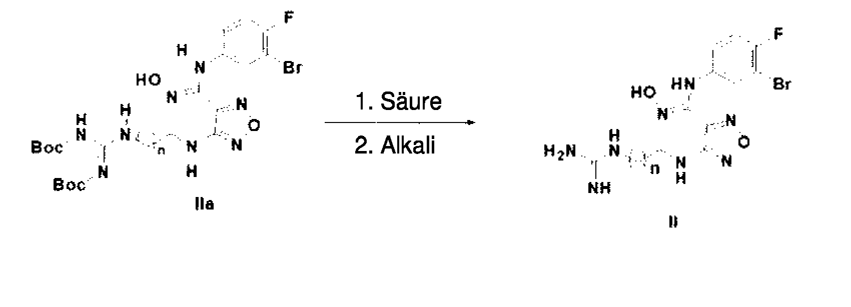

## Revendications

1. Composé représenté par la Formule I ou son sel pharmaceutiquement acceptable, 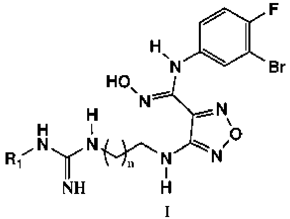
dans lequel n est un entier sélectionné parmi 0 et 6,
R₁ est choisi dans le groupe constitué par H, NH₂, CN, méthyle, éthyle, propyle, isopropyle, butyle, cyclopropyle, cyclobutyle, cyclopentyle, cyclohexyle, méthoxycarbonyle, tertbutoxycarbonyle, fluorénylméthoxycarbonyle, allyloxycarbonyle, benzyle et 5-azaindène méthylcarbonyle.

2. Composé ou sel pharmaceutiquement acceptable de celui-ci selon la revendication 1, **caractérisé en ce que** n est choisi parmi 0, 1, 2, 3 et 4.

3. Composé ou sel pharmaceutiquement acceptable de celui-ci selon la revendication 1, **caractérisé en ce que** lorsque R₁ dans la formule I est H, le composé est représenté par la Formule II,

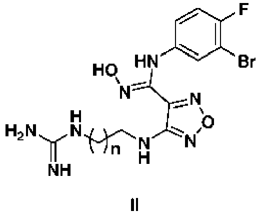

4. Composé ou sel pharmaceutiquement acceptable de celui-ci selon la revendication 3, **caractérisé en ce que** n est 0, 1, 2 ou 3.

5. Composé ou sel pharmaceutiquement acceptable de celui-ci selon la revendication 1, **caractérisé en ce que** le composé est sélectionné dans le groupe constitué par :

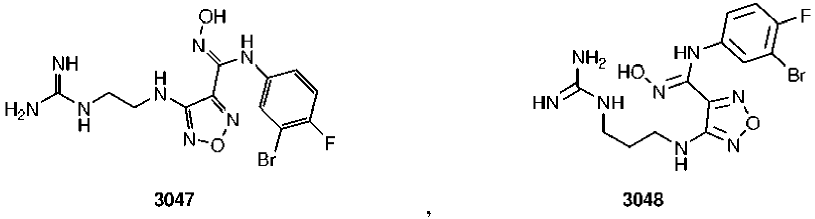

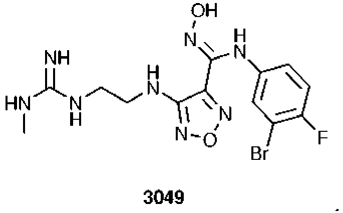

6. Composé ou sel pharmaceutiquement acceptable de celui-ci, **caractérisé en ce que** le composé est choisi dans le groupe constitué par :

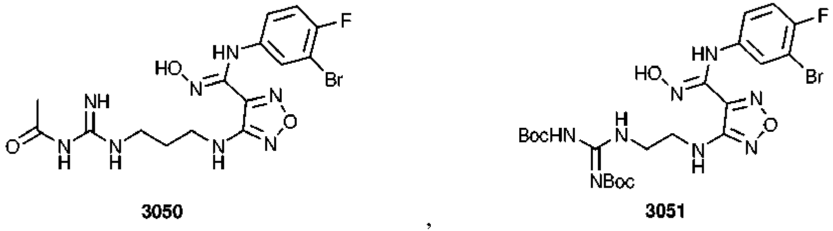

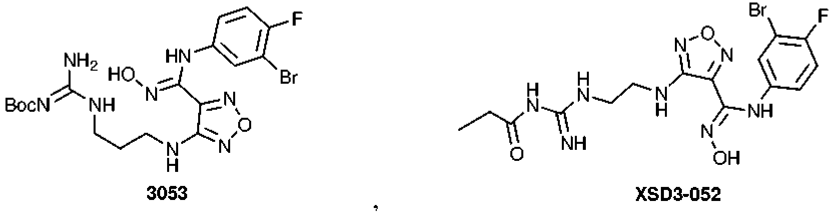

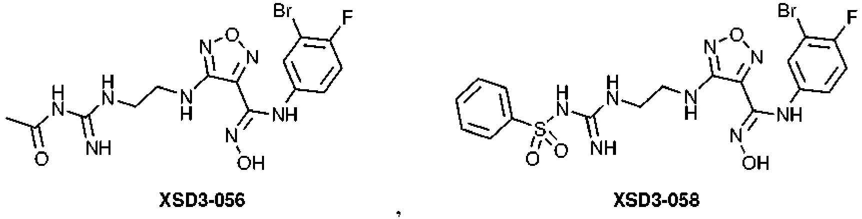

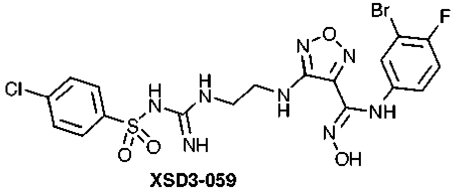

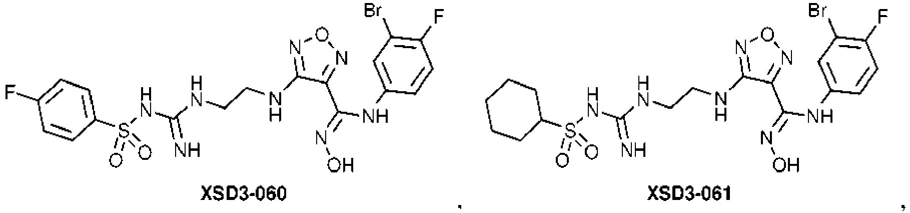

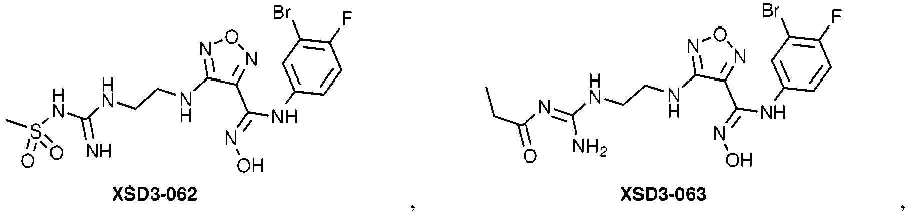

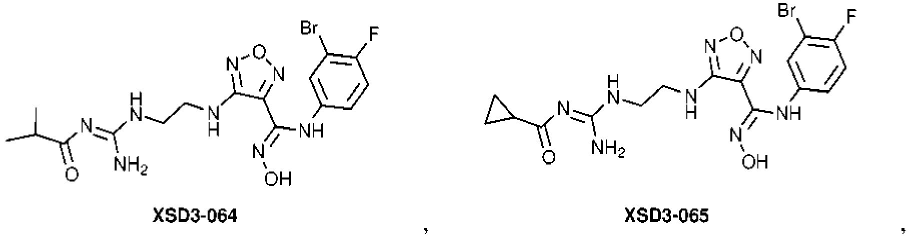

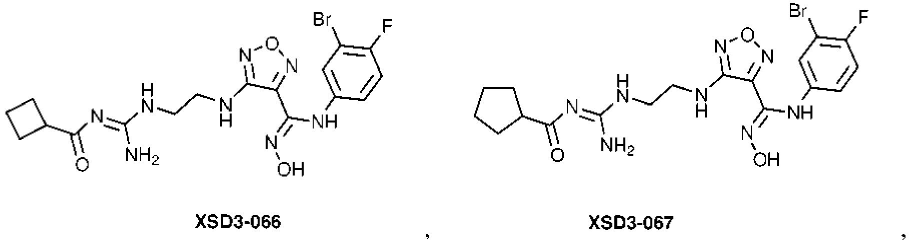

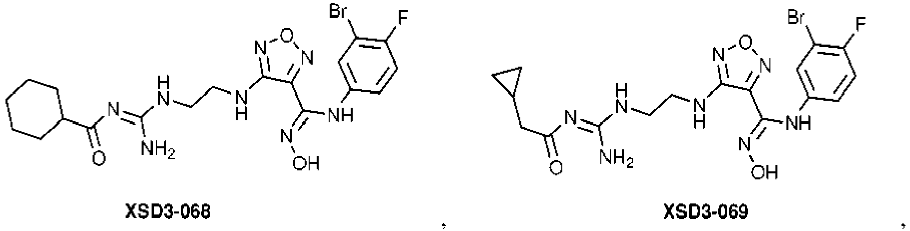

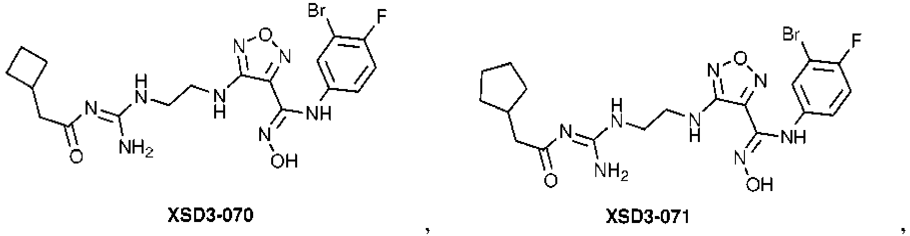

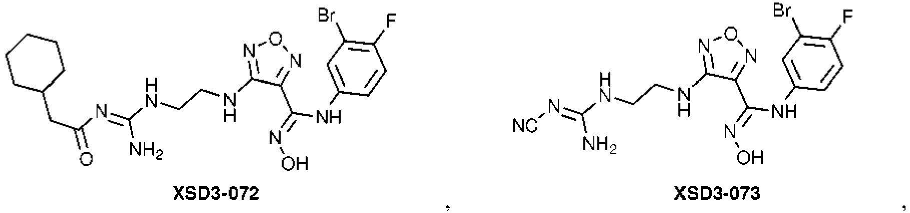

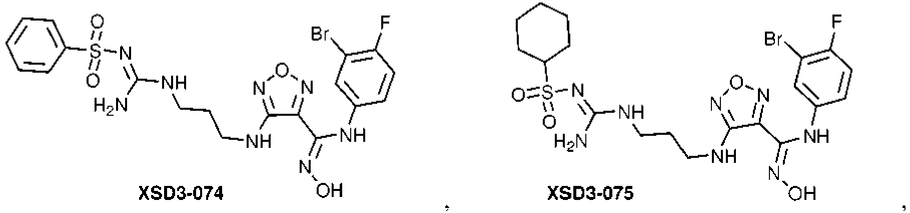

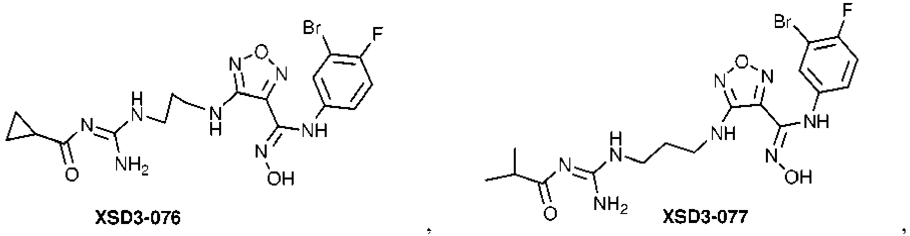

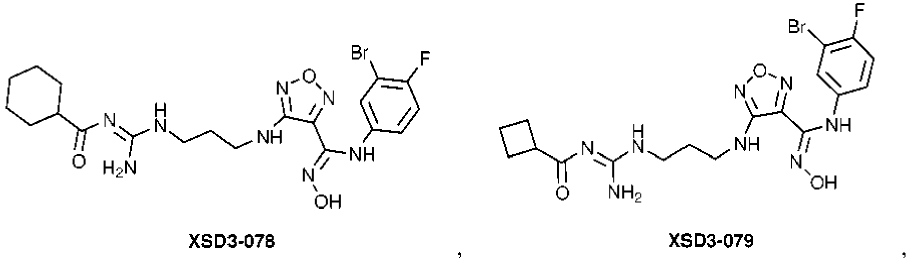

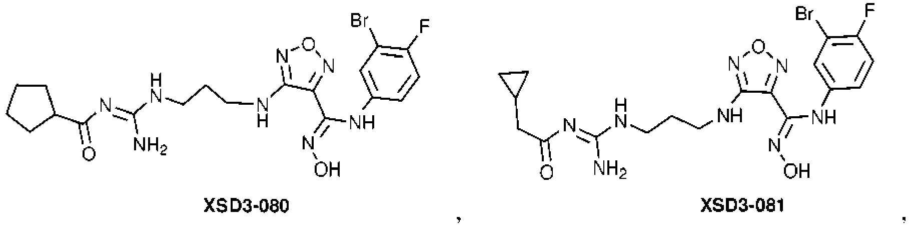

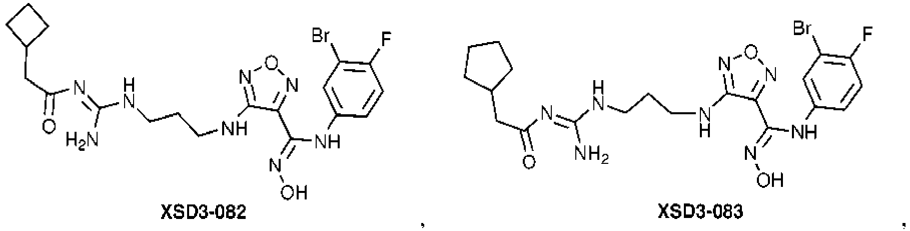

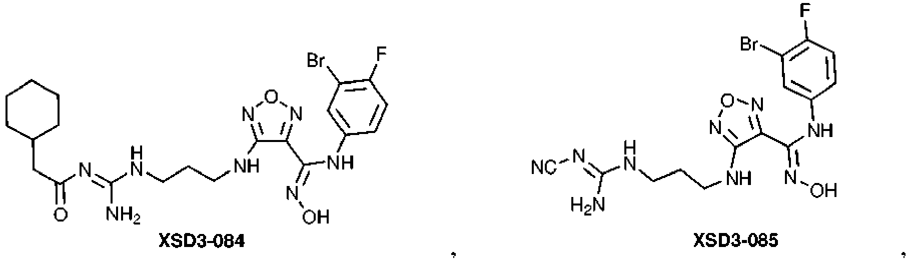

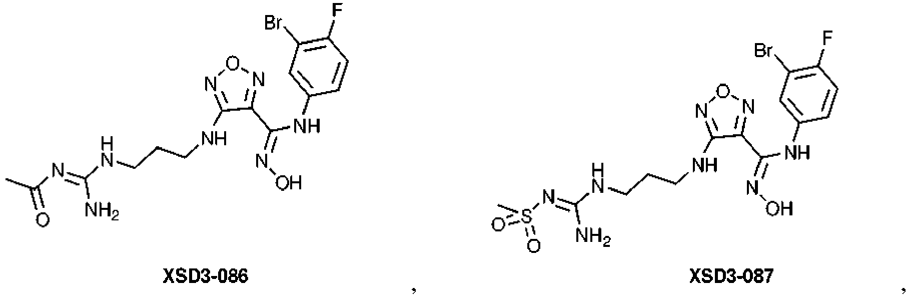

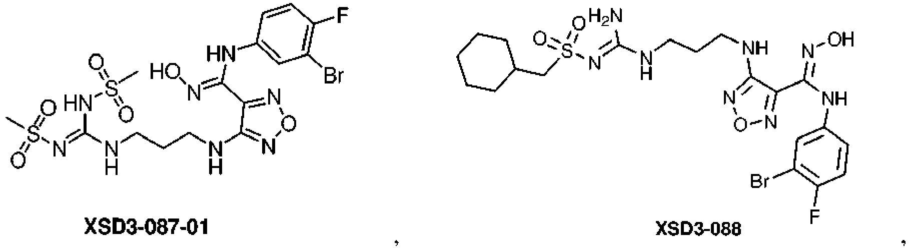

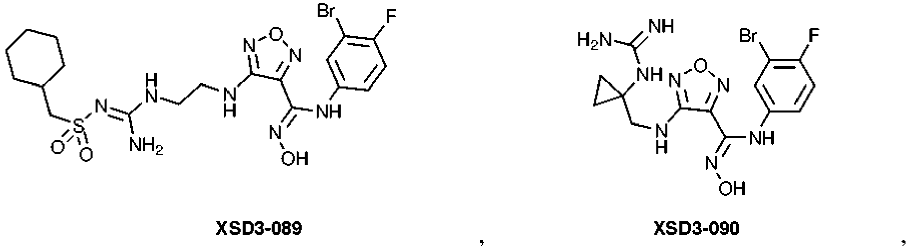

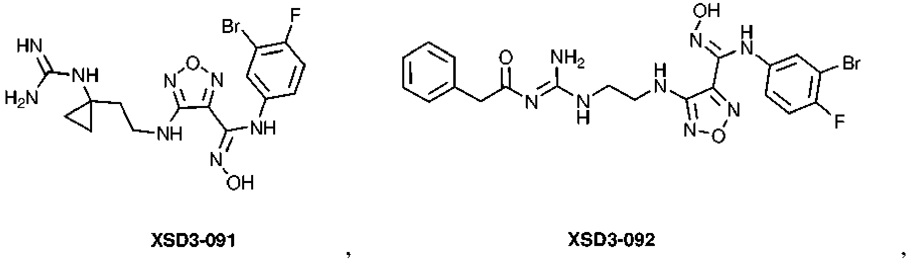

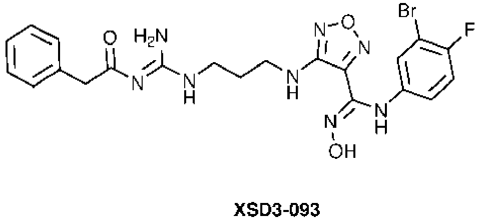

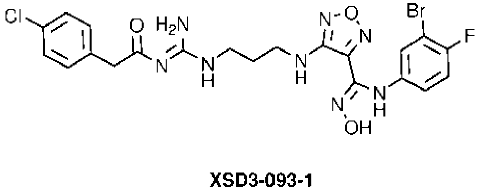

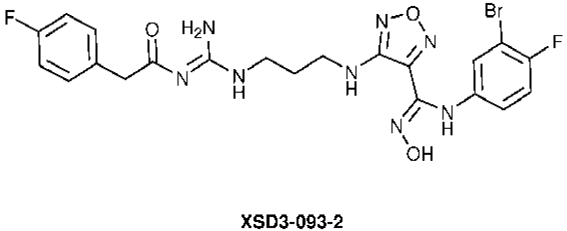

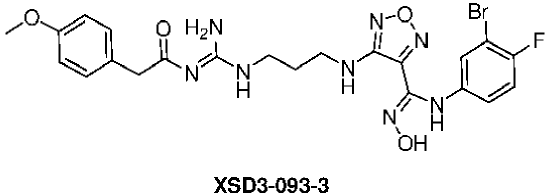

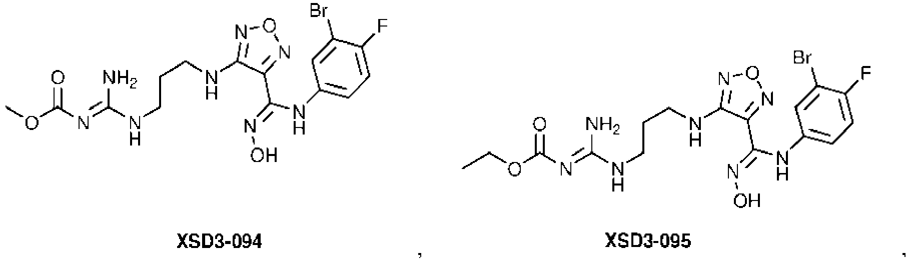

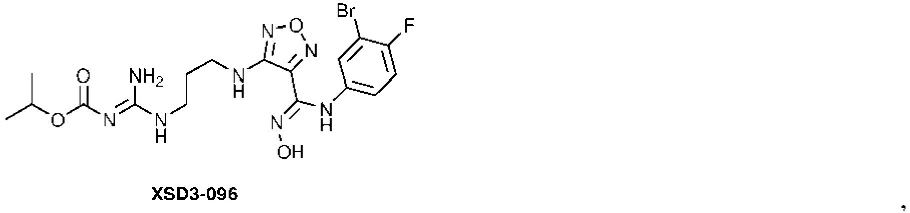

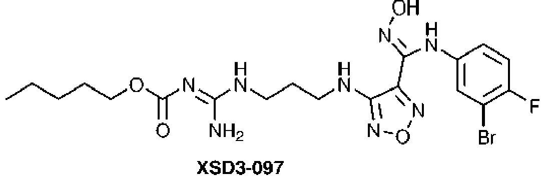

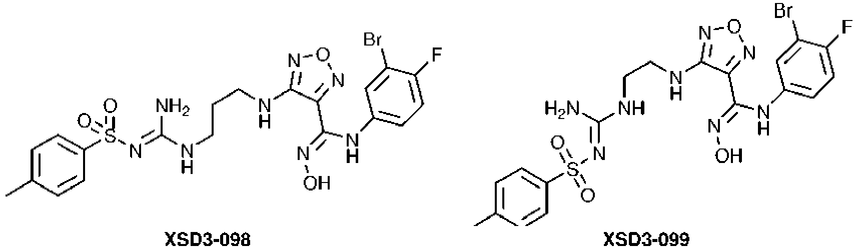

7. Composition pharmaceutique comprenant au moins un composé ou un sel pharmaceutiquement acceptable de celui-ci selon l'une quelconque des revendications 1 à 6 et un ou plusieurs adjuvants pharmaceutiques pharmaceutiquement acceptables.

8. Composé ou un sel pharmaceutiquement acceptable de celui-ci selon l'une quelconque des revendications 1 à 6 ou une composition pharmaceutique de celui-ci, pour une utilisation dans le traitement d'un cancer, d'une maladie infectieuse, d'une maladie neurodégénérative, d'une dépression, d'une anxiété ou d'une cataracte liée à l'âge.

9. Composé à utiliser selon la revendication 8, dans lequel le cancer est choisi dans le groupe constitué par le cancer du poumon, le cancer du foie, le cancer du côlon, le cancer du pancréas, le cancer du sein, le cancer de la prostate, le cancer du cerveau, le cancer des ovaires, le cancer du col de l'utérus, le cancer des testicules, le cancer du rein, le cancer de la tête et du cou, le lymphome, le mélanome ou la leucémie

10. Composé pour une utilisation selon la revendication 8, dans lequel la maladie neurodégénérative est la maladie d'Alzheimer.

11. Composé à utiliser selon la revendication 8, dans lequel la maladie infectieuse est une infection causée par une bactérie, un champignon, un virus ou un parasite.

12. Procédé pour préparer un composé représenté par la Formule II ou un sel pharmaceutiquement acceptable de celui-ci, selon la revendication 3, 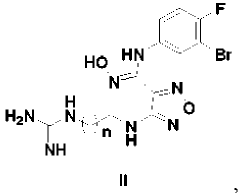 dans lequel n est 0, 1, 2 ou 3, **caractérisé en ce qu'**il comprend les étapes suivantes :
1) réaction d'un composé représenté par la formule **2** avec le composé **3a'** pour obtenir un composé représenté par la formule **IIa** : 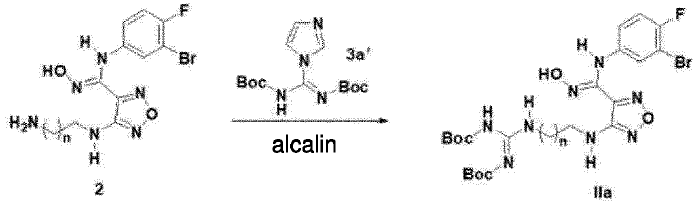
2) réalisation d'une déprotection du composé représenté par la formule **IIa** dans des conditions acides, puis réaction dans des conditions alcalines pour obtenir le composé représenté par la formule **II**,
